(19) Europäisches Patentamt
European Patent Office
Office européen des brevets

(11) **EP 4 423 301 B1**

(12) **EUROPEAN PATENT SPECIFICATION**

(45) Date of publication and mention
of the grant of the patent:
**10.12.2025 Bulletin 2025/50**

(21) Application number: **22818527.8**

(22) Date of filing: **28.10.2022**

(51) International Patent Classification (IPC):
***C12Q 1/6886*** *(2018.01)*

(52) Cooperative Patent Classification (CPC):
**C12Q 1/6886;** C12Q 2600/118; C12Q 2600/158

(86) International application number:
**PCT/US2022/048191**

(87) International publication number:
**WO 2023/076574 (04.05.2023 Gazette 2023/18)**

(54) **TUMOR MICROENVIRONMENT TYPES IN BREAST CANCER**

TUMORMIKROUMGEBUNGSTYPEN BEI BRUSTKREBS

TYPES DE MICROENVIRONNEMENT TUMORAL DANS LE CANCER DU SEIN

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.10.2021 US 202163273171 P**

(43) Date of publication of application:
**04.09.2024 Bulletin 2024/36**

(60) Divisional application:
**25212124.9**

(73) Proprietor: **BostonGene Corporation**
**Waltham, MA 02453 (US)**

(72) Inventors:
• **GURYLEVA, Mariia**
**St. Petersburg, 199106 (RU)**
• **KHORKOVA, Svetlana**
**Dzerzhinsky, 140090 (RU)**
• **KOTLOV, Nikita**
**4041 Limassol (CY)**
• **ZOTOVA, Anastasia**
**Moscow, 109145 (RU)**
• **VALIEV, Ivan**
**Petersburg, 198035 (RU)**
• **BAGAEV, Alexander**
**Waltham, MA 02451 (US)**
• **SHAMSUTDINOVA, Diana**
**Naberezhnye Chelny, 423832 (RU)**

• **ELIAS-NOMIE, Krystle**
**Houston, TX 77008 (US)**
• **BUTUSOVA, Anna**
**Yerevan, 0012 (AM)**
• **ANTYSHEVA, Zoia**
**Moscow, 124482 (RU)**

(74) Representative: **Mewburn Ellis LLP**
**Aurora Building**
**Counterslip**
**Bristol BS1 6BX (GB)**

(56) References cited:
**US-A1- 2021 208 161**

• **WANG YANYAN ET AL: "Identification of Tumor Microenvironment-Related Prognostic Biomarkers in Luminal Breast Cancer", FRONTIERS IN GENETICS, vol. 11, 10 November 2020 (2020-11-10), XP93022446, DOI: 10.3389/ fgene.2020.555865**
• **ROJAS KATERIN ET AL: "In silico transcriptomic mapping of integrins and immune activation in Basal-like and HER2+ breast cancer", CELLULAR ONCOLOGY, SPRINGER, DORDRECHT, vol. 44, no. 3, 19 January 2021 (2021-01-19), pages 569 - 580, XP037484789, ISSN: 2211-3428, [retrieved on 20210119], DOI: 10.1007/S13402-020-00583-9**

**(Cont. next page)**

- **LUIS FELIPE CAMPESATO ET AL: "High IL-1R8 expression in breast tumors promotes tumor growth and contributes to impaired antitumor immunity", ONCOTARGET, vol. 8, no. 30, 25 July 2017 (2017-07-25), pages 49470 - 49483, XP055529971, DOI: 10.18632/oncotarget.17713**
- **BAGAEV ALEXANDER ET AL: "Conserved pan-cancer microenvironment subtypes predict response to immunotherapy", CANCER CELL, CELL PRESS, US, vol. 39, no. 6, 20 May 2021 (2021-05-20), pages 845, XP086620334, ISSN: 1535-6108, [retrieved on 20210520], DOI: 10.1016/J.CCELL.2021.04.014**
- **DATABASE EMBASE [online] ELSEVIER SCIENCE PUBLISHERS, AMSTERDAM, NL; 1 March 2023 (2023-03-01), KHORKOVA S ET AL: "A molecular classification system for basal-like breast cancer based on the tumor microenvironment is prognostic for survival", Database accession no. EMB-640586601**
- **SCHUBERT MICHAEL ET AL: "Perturbation-response genes reveal signaling footprints in cancer gene expression", NATURE COMMUNICATIONS, vol. 9, no. 1, 2 January 2018 (2018-01-02), UK, XP093184943, ISSN: 2041-1723, Retrieved from the Internet <URL:https://www.nature.com/articles/s41467-017-02391-6.pdf> DOI: 10.1038/s41467-017-02391-6**
- **WANG YANYAN ET AL: "Identification of Tumor Microenvironment-Related Prognostic Biomarkers in Luminal Breast Cancer", FRONTIERS IN GENETICS, vol. 11, 10 November 2020 (2020-11-10), XP093022446, DOI: 10.3389/fgene.2020.555865**
- **JAMES R. BRADFORD ET AL: "Consensus Analysis of Whole Transcriptome Profiles from Two Breast Cancer Patient Cohorts Reveals Long Non-Coding RNAs Associated with Intrinsic Subtype and the Tumour Microenvironment", PLOS ONE, vol. 11, no. 9, 29 September 2016 (2016-09-29), pages e0163238, XP055540557, DOI: 10.1371/journal.pone.0163238**
- **SADEGHALVAD MONA ET AL: "Immune microenvironment in different molecular subtypes of ductal breast carcinoma", BREAST CANCER RESEARCH AND TREATMENT, vol. 185, no. 2, 3 October 2020 (2020-10-03), pages 261 - 279, XP037359090, ISSN: 0167-6806, DOI: 10.1007/S10549-020-05954-2**

**Description**

BACKGROUND

Breast cancer is a highly heterogeneous disease. Correctly characterizing the type or types of cancer a patient or subject has and, potentially, selecting one or more effective therapies for the patient can be crucial for the survival and overall wellbeing of that patient. Advances in characterizing cancers, predicting prognoses, identifying effective therapies, and otherwise aiding in personalized care of patients with cancer are needed.

Wang Yanyan et al. "Identification of Tumor Microenvironment-Related Prognostic Biomarkers in Luminal Breast Cancer", FRONTIERS IN GENETICS, (20201110), vol. 11 describes a study where immune scores (ISs) and stromal scores (SSs) for The Cancer Genome Atlas (TCGA) luminal BC cohort were obtained from the online ESTIMATE (Estimation of STromal and Immune cells in MAlignant Tumor tissues using Expression data) portal, and the relationships between ISs and SSs and the overall survival of luminal BC patients were assessed by the Kaplan-Meier method.

James R. Bradford et al. "Consensus Analysis of Whole Transcriptome Profiles from Two Breast Cancer Patient Cohorts Reveals Long Non-Coding RNAs Associated with Intrinsic Subtype and the Tumour Microenvironment", PLOS ONE, (20160929), vol. 11, no. 9 describes a study where consensus lncRNA signatures indicative of breast cancer subtype were derived based on two clinical RNA-Seq datasets.

Sadeghalvad Mona et al. "Immune microenvironment in different molecular subtypes of ductal breast carcinoma", BREAST CANCER RESEARCH AND TREATMENT, (20201003), vol. 185, no. 2 describes important immune cells and immune system-associated molecules in Ductal Carcinoma In situ (DCIS) and Invasive Ductal Carcinoma (IDC) with special emphasis on their associations with different molecular subtypes and patients' prognosis.

US 2021/208161 A1 describes the use of the CC chemokine ligand (CCL24) molecule as a diagnostic and therapeutic target for breast cancer.

SUMMARY

The claimed invention is defined in the appended claims.

Aspects of the disclosure relate to methods for identifying molecular types of breast cancer (BC), such as the four breast cancer types: basal-like breast cancer (BLBC), luminal breast cancer, normal-like breast cancer, and HER2-enriched breast cancer (H2EBC). Each of the molecular subtypes can be further independently subdivided into tumor microenvironment (TME) subtypes. The breast cancer TME type of a subject may be indicative of one or more characteristics of the subject (or the subject's cancer), for example the likelihood a subject will have a good prognosis or respond to a therapeutic agent such as an immunotherapy (also referred to as an IO agent or immuno-oncology agent), an anti-VEGF therapy, or a tyrosine kinase inhibitor (TKI).

The claimed invention provides a method for determining a basal-like breast cancer (BLBC) tumor microenvironment (TME) type of a subject having basal-like breast cancer, the method comprising using at least one computer hardware processor to perform obtaining RNA expression data for the subject, the RNA expression data indicating RNA expression levels for at least some genes in each group of at least some of a plurality of gene groups listed in Table 1 in a biological sample comprising tumor tissue of the subject, wherein the RNA expression data indicates RNA expression levels for at least three genes from each of at least: the granulocytes gene group, the angiogenesis gene group, the B-cells gene group, the M1 signatures gene group, and the endothelium gene group; generating a BLBC TME signature for the subject using the RNA expression data, the BLBC TME signature comprising gene group scores for respective gene groups in the at least some of the plurality of gene groups, the generating comprising: determining gene groups scores using the RNA expression levels and a gene set enrichment analysis technique; and identifying, using the BLBC TME signature and from among a plurality of BLBC TME types, a BLBC TME type for the subject. The plurality of BLBC TME types comprises: Immune Enriched (IE) type, TLS (TLS) type, Desert (D) type, Fibrotic (F) type, and Granulocyte enriched (G) type. BLBC Type G comprises the highest Granulocytes and M1 signatures gene group scores of the plurality of BLBC TME types, types G and F have higher Angiogenesis gene group scores than the other BLBC TME types of the plurality of BLBC TME types, the TLS type has the highest B cells gene group score of the plurality of BLBC TME types, and types TLS and F have the highest Endothelium expression level gene group scores of the plurality of BLBC TME types.

In some embodiments, the method further comprises generating one or more PROGENy signatures using the RNA expression levels.

In some embodiments, the one or more PROGENy signatures comprise TGFb, NFkB, and/or VEGF signaling PROGENy signatures. In some embodiments, the identifying the BLBC TME type of the subject comprises using the one or more PROGENy signatures.

In some embodiments, obtaining the RNA expression data for the subject comprises obtaining sequencing data previously obtained by sequencing a biological sample obtained from the subject, wherein the biological sample comprises tumor tissue of the subject.

In some embodiments, the RNA expression data comprises at least 1 million reads, at least 5 million reads, at least 10 million reads, at least 20 million reads, at least 50 million reads, or at least 100 million reads.

In some embodiments, the RNA expression data comprises whole exome sequencing (WES) data, bulk RNA sequencing (RNA-seq) data, single cell RNA sequencing (scRNA-seq) data, or next generation sequencing (NGS) data. In some embodiments, the sequencing data comprises microarray data.

In some embodiments, the method further comprises normalizing the RNA expression data to transcripts per million (TPM) units prior to generating the BLBC TME signature.

In some embodiments, obtaining the RNA expression data for the subject comprises sequencing a biological sample obtained from the subject.

In some embodiments, the biological sample comprises breast tissue of the subject. The biological sample comprises tumor tissue of the subject.

In some embodiments, the RNA expression levels comprise RNA expression levels for at least three genes from each of at least two of the following gene groups: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

In some embodiments, the RNA expression levels comprise RNA expression levels for each of the genes from each of the following gene groups: MHC I group: HLA-C, TAPBP, HLA-B, B2M, TAP2, HLA-A, TAP1, and NLRC5; MHC II group: HLA-DQB 1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

In some embodiments, determining the gene group scores comprises determining a respective gene group score for each of at least two of the following gene groups, using, for a particular gene group, RNA expression levels for at least three genes in the particular gene group to determine the gene group score for the particular group, the gene groups including: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

In some embodiments, determining the gene group scores comprises determining a respective gene group score for each of the following gene groups, using, for a particular gene group, RNA expression levels for each of the genes in each gene group to determine the gene group score for each particular group, the gene groups including: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

In some embodiments, determining the gene group scores comprises determining a first score of a first gene group using a single-sample Gene Set Enrichment Analysis (ssGSEA) technique from RNA expression levels for at least some of the genes in one or the following gene groups: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

In some embodiments, determining the gene group scores comprises using a single-sample GSEA (ssGSEA) technique to determine the gene group scores from RNA expression levels for each of the genes in each of the following gene groups: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group:

ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

In some embodiments, generating the BLBC TME signature further comprises normalizing the gene group scores, wherein the normalizing comprises applying median scaling to the gene group scores.

In some embodiments, the plurality of BLBC TME types is associated with a respective plurality of BLBC TME signature clusters, wherein identifying, using the BLBC TME signature and from among a plurality of BLBC TME types, the BLBC TME type for the subject comprises associating the BLBC TME signature of the subject with a particular one of the plurality of BLBC TME signature clusters; and identifying the BLBC TME type for the subject as the BLBC TME type corresponding to the particular one of the plurality of BLBC TME signature clusters to which the BLBC TME signature of the subject is associated.

In some embodiments, the method further comprises generating the plurality of BLBC TME signature clusters, the generating comprising obtaining multiple sets of RNA expression data by sequencing biological samples from multiple respective subjects, each of the multiple sets of RNA expression data indicating RNA expression levels for at least some genes in each of the at least some of the plurality of gene groups listed in Table 1; generating multiple BLBC TME signatures from the multiple sets of RNA expression data, each of the multiple BLBC TME signatures comprising gene group expression scores for respective gene groups in the plurality of gene groups, the generating comprising, for each particular one of the multiple BLBC TME signatures: determining the BLBC TME signature by determining the gene group expression scores using the RNA expression levels in the particular set of RNA expression data for which the particular one BLBC TME signature is being generated; and clustering the multiple BLBC signatures to obtain the plurality of BLBC TME signature clusters.

In some embodiments, the method further comprises updating the plurality of BLBC TME signature clusters using the BLBC TME signature of the subject, wherein the BLBC TME signature of the subject is one of a threshold number BLBC TME signatures for a threshold number of subjects, wherein when the threshold number of BLBC TME signatures is generated the BLBC TME signature clusters are updated, wherein the threshold number of BLBC TME signatures is at least 50, at least 75, at least 100, at least 200, at least 500, at least 1000, or at least 5000 BLBC TME signatures.

In some embodiments, the updating is performed using a clustering algorithm selected from the group consisting of a dense clustering algorithm, spectral clustering algorithm, k-means clustering algorithm, hierarchical clustering algorithm, and an agglomerative clustering algorithm.

In some embodiments, the method further comprises determining an BLBC TME type of a second subject, wherein the BLBC TME type of the second subject is identified using the updated BLBC TME signature clusters, wherein the identifying comprises determining an BLBC TME signature of the second subject from RNA expression data obtained by sequencing a biological sample obtained from the second subject; associating the BLBC TME signature of the second subject with a particular one of the plurality of the updated BLBC TME signature clusters; and identifying the BLBC TME type for the second subject as the BLBC TME type corresponding to the particular one of the plurality of updated BLBC TME signature clusters to which the BLBC TME signature of the second subject is associated.

The plurality of BLBC TME types comprises: Immune Enriched (IE) type, TLS (TLS) type (also referred to as B-cell enriched), Desert (D) type, Fibrotic (F) type, and Granulocyte enriched (G) type.

In some embodiments, the method further comprises identifying at least one therapeutic agent for administration to the subject using the BLBC TME type of the subject.

In some embodiments, the at least one therapeutic agent comprises an immuno-oncology (IO) agent. In some embodiments, the IO agent comprises an immune checkpoint inhibitor. In some embodiments, the immune checkpoint inhibitor comprises an anti-PD-1 antibody, anti-PD-L1 antibody, or anti-CTLA4 antibody.

In some embodiments, the at least one therapeutic agent comprises an anti-VEGF therapy. In some embodiments, the anti-VEGF therapy comprises an anti-VEGF antibody.

In some embodiments, the at least one therapeutic agent comprises a tyrosine kinase inhibitor (TKI).

In some embodiments, identifying the at least one therapeutic agent based upon the BLBC TME type of the subject comprises identifying an immune checkpoint inhibitor as the at least one therapeutic agent when the subject is identified as having G type, IE type or TLS type BLBC TME. In some embodiments not part of the claims, the method further comprises administering the identified immune checkpoint inhibitor to the subject.

In some embodiments, identifying the at least one therapeutic agent based on the BLBC TME type of the subject comprises identifying an anti-VEGF therapy as the at least one therapeutic agent when the subject is identified as having F type BLBC TME. In some embodiments not part of the claims, the method further comprises administering the identified anti-VEGF therapy to the subject.

In some embodiments, identifying the at least one therapeutic agent based on the BLBC TME type of the subject comprises identifying a TKI therapy when the subject is identified as having D type BLBC TME. In some embodiments not part of the claims, the method further comprises administering the identified TKI to the subject.

In some aspects, the disclosure provides a system, comprising: at least one computer hardware processor; and at least one non-transitory computer readable medium storing processor-executable instructions that, when executed by the at least one computer hardware processor, cause the at least one computer hardware processor to perform a method as claimed.

In some aspects, the disclosure provides at least one non-transitory computer readable medium storing processor-executable instructions that, when executed by at least one computer hardware processor, cause the at least one computer hardware processor to perform a method as claimed.

BRIEF DESCRIPTION OF DRAWINGS

FIG. 1 is a diagram depicting a flowchart of an illustrative process 100 for determining a basal-like breast cancer (BLBC) tumor microenvironment (TME) type for a subject, according to some embodiments of the technology as described herein.

FIG. 2 is a diagram depicting a flowchart of an illustrative process 200 for determining a luminal and normal-like breast cancer (LNLBC) tumor microenvironment (TME) type for a subject, according to some embodiments of the technology as described herein not part of the claims.

FIG. 3 is a diagram depicting a flowchart of an illustrative process 300 for determining a HER2-enriched breast cancer (H2EBC) tumor microenvironment (TME) type for a subject, according to some embodiments of the technology as described herein not part of the claims.

FIG. 4 is a diagram depicting a flowchart of an illustrative process for processing sequencing data to obtain RNA expression data, according to some embodiments of the technology as described herein.

FIG. 5 is a diagram depicting an illustrative technique for determining gene group scores, according to some embodiments of the technology as described herein.

FIG. 6 is a diagram depicting an illustrative technique for identifying a basal-like breast cancer (BLBC) tumor microenvironment (TME) type using a BLBC TME signature, according to some embodiments of the technology as described herein.

FIG. 7 shows a representative heatmap of basal-like breast cancer (BLBC) samples classified into five distinct TME subtypes (G, IE, TLS, F, D) based on unsupervised dense clustering of BLBC TME signatures. Each column represents one sample. Heatmap represents the z-score of each of the BG signatures.

FIG. 8 shows representative data showing selected gene group scores compared across BLBC TME types.

FIG. 9 shows representative data for cell deconvolution analysis across the samples. The columns represent single samples and are ordered by BLBC TME types as follows: G, IE, TLS, F, and D. The bar plots represent the cell composition fraction. Each legend marker corresponds to a particular cell type.

FIGs. 10A-10C show representative data indicating BLBC TME typing is supported by TCGA histological data. FIG. 10A shows representative TCGA histological images of TME types; FIG. 10B shows representative box plots depicting percentage of stromal tumor-infiltrating lymphocytes (sTILs); FIG. 10C shows representative data for levels of fibrosis across TME types.

FIG. 11 shows representative data for overall survival (OS) analysis of primary basal-like breast cancer samples, treated with chemotherapy, across different TME subtypes. Kaplan-Meier (top) and log hazard ratios (bottom) are shown.

FIG. 12 shows a representative heatmap of single molecule expressions across BLBC TME types (G, IE, TLS, F, D). Each column represents one sample. Panel on the top corresponds to the sample annotation. Heatmap represents the z-score of each molecule.

FIG. 13 shows a representative heatmap of luminal and normal-like breast cancer (LNLBC) samples classified into five distinct TME types (D, IE, IE/F, E, F) based on unsupervised dense clustering of LNLBC TME signatures.

FIG. 14 shows representative data showing selected gene group scores compared across BLBC TME types.

FIG. 15 shows representative data for cell deconvolution analysis across the samples. The columns represent single samples and are ordered by LNLBC TME types as follows: D, IE, IE-med, E, and F. The bar plots represent the cell composition fraction. Each legend marker corresponds to a particular cell type.

FIG. 16 shows representative data for overall survival (OS) analysis across LNLBC TME types in hormone treated samples in a Metabric dataset.

FIG. 17 shows a representative heatmap of single molecule expressions across LNLBC TME types (D, IE, IE/F, E, and F). Panel on the top corresponds to the sample annotation. Heatmap represents the median scaled expression of each molecule.

FIG. 18 shows a representative heatmap of HER2-enriched luminal and normal-like breast cancer (H2EBC) samples classified into five distinct TME types (D, IE-med, IE, F, and End-Ar-H) based on unsupervised dense clustering of H2EBC TME signatures. Each column represents one sample. Heatmap represents the signal of each of the signatures.

FIG. 19 shows representative data showing selected gene group scores compared across BLBC TME types.

FIG. 20 shows representative data for cell deconvolution analysis across the samples. The columns represent single samples and are ordered by the TME subtypes as follows: D, IE, IE-med, E, and F. Panel on the top corresponds to the sample annotation. Each legend marker corresponds to a particular cell type.

FIG. 21 shows representative data for overall survival (OS) analysis across H2EBC TME types in chemotherapy-treated samples in a SCAN-B dataset.

FIG. 22 shows a representative heatmap of single molecule expressions across H2EBC TME types (D, IE-med, IE, F, and End-Ar-H). Panel on the top corresponds to the sample annotation. Heatmap represents the median scaled expression of each molecule.

FIG. 23 depicts an illustrative implementation of a computer system that may be used in connection with some embodiments of the technology described herein.

## DETAILED DESCRIPTION

Aspects of the disclosure relate to methods for characterizing subjects having breast cancer, and for determining the tumor microenvironment (TME) type of a subject's breast cancer. The disclosure is based, in part, on classification of intrinsic subtypes of breast cancer (basal-like breast cancer according to the claims, luminal breast cancer, normal-like breast cancer, and HER2-enriched breast cancer, according to embodiments not part of the claims) into phenotypically distinct TME types within each intrinsic subtype. In some embodiments, the methods comprise identifying a subject as having a particular breast cancer TME type based upon a TME signature computed for the subject from their RNA expression data. The breast cancer TME type identified for the subject may have various prognostic, diagnostic, and/or therapeutic applications. For example, in some embodiments, methods developed by the inventors and described herein are useful for identifying a subject's prognosis, such as a therapeutic response prognosis, based upon the TME type identified for the subject.

Breast cancer is a highly heterogeneous group of solid tumor cancers that originate in breast tissue. Worldwide, breast cancer is the most common invasive cancer in women, with over 2 million diagnoses per year. Classification of a subject's breast cancer type is an important process that may provide insight into tumor biology and the subject's prognosis. Tumor classification may also guide a physician's decisions on therapeutic and surgical interventions for a patient. Historically, breast cancers have been classified by histopathological methods. However, due to the high heterogeneity of breast cancer tumor phenotypes, histopathological classification lacks the resolution to provide informative data at the molecular level.

Molecular characterization of breast cancers has also been described, for example by Perou et al. (Nature. 2000;406(6796):747-752). Four intrinsic subtypes of breast cancer have been identified: basal-like breast cancer (BLBC), luminal breast cancer (also referred to as luminal A breast cancer), normal-like breast cancer (also referred to as luminal B breast cancer), and HER2-enriched breast cancer (H2EBC). In some embodiments, luminal breast cancers (e.g., luminal A breast cancer and normal-like breast cancer, may be combined and referred to as luminal and normal-like breast cancer (LNLBC). Luminal carcinomas express estrogen receptor (ER) and comprise variable cell proliferation markers. HER2 overexpression is the defining characteristic of H2EBC tumors. H2EBC tumors also lack ER and progesterone receptor (PR) expression. Basal-like breast cancer (BLBC) lacks expression of ER, PR, and HER2 (and thus may be referred to as "triple-negative" breast cancer). BLBC tumors do express basal cell markers, for example cytokeratin (CK) 5/6 and/or epidermal growth factor receptor (EGFR). Molecular classification of breast cancers into four intrinsic molecular types suffers many of the same challenges as histopathological classification of breast cancer, for example as described by Zhang (Arch Pathol Lab Med. 2022 Sep 22. doi: 10.5858/arpa.2022-0070-RA). In particular, there remains a high level of tumor heterogeneity within each intrinsic cancer type, and thus the existing classifications do not reflect the complexity of the underlying tumor biology within each type. Additionally, a high level of variability has been observed within each intrinsic breast cancer type in the context of prognosis and diversified treatment responses.

The disclosure describes statistical techniques for analyzing expression data (e.g., RNA expression data), which was obtained from a biological sample obtained from a subject that has breast cancer, is suspected of having breast cancer, or is at risk of developing breast cancer, in order to generate a gene expression signature for the subject (termed a "TME signature" herein) and use this signature to identify a particular TME type that the subject may have.

The inventors have recognized that certain intrinsic molecular subtypes of breast cancer (e.g., BLBC, LNLBC, and H2EBC) may be further separated into phenotypically distinct TME types within each intrinsic subtype. For example, the inventors have recognized that each intrinsic breast cancer molecular subtype may be characterized as having five phenotypically distinct types. In some embodiments, BLBC may be characterized as having five phenotypically distinct types (Immune Enriched (IE) type, TLS (TLS) type (also referred to as B-cell enriched), Desert (D) type, Fibrotic (F) type, and Granulocyte enriched (G) type). In some embodiments, LNLBC may be characterized as having five phenotypically distinct types (Immune Desert (D) type, Fibrotic (F) type, Immune-Enriched, Non-fibrotic (IE) type, Immune-Enriched, Fibrotic (IE/F) type, and Angiogenic (E) type). In some embodiments, H2EBC may be characterized as having five phenotypically distinct types (Immune Desert (D) type, Fibrotic (F) type, Immune-Enriched, Non-fibrotic (IE) type, Moderately Immune-Enriched (IE-med) type, and Endothelium enriched (End-Ar-H) type). As described further in the Examples, each breast cancer TME type was identified using a combination of gene group expression scores, and, optionally, one or more PROGENy signatures, to produce breast cancer TME signature that characterize patients having breast cancers more accurately than previously developed methods. In some embodiments, such TME types are useful for identifying the prognosis and/or likelihood that a subject will respond to particular therapeutic interventions (e.g., immunotherapy agents, anti-VEGF agents, tyrosine kinase inhibitors, etc.).

The use of TME signatures comprising the combinations of gene group scores described by the disclosure represents an improvement over previously described molecular characterization of breast cancer because the specific groups of genes used to produce the TME signatures described herein better reflect the molecular tumor microenvironments of breast cancer because these gene groups are associated with the underlying biological pathways controlling tumor behavior and the host tumor microenvironment. These focused combinations of gene groups (e.g., gene groups consisting of some or all of the gene group genes listed in Table 1, Table 2, and/or Table 3) are unconventional, and differ from previously described molecular signatures, which do not account for the high levels of genotypic and phenotypic heterogeneity within each broad molecular subtype of breast cancer.

The TME typing methods described herein have several utilities. For example, identifying a subject's TME type using methods described herein may allow for the subject to be diagnosed as having (or being at a high risk of developing) an aggressive form of breast cancer (e.g., BLBC TME type D or G) at a timepoint that is not possible with previously described breast cancer characterization methods. Earlier detection of aggressive breast cancer types, enabled by the TME signatures described herein, improve the patient diagnostic technology by enabling earlier chemotherapeutic intervention for patients than currently possible for patients tested for breast cancer using other methods (e.g., histological analysis).

As described herein, the inventors have also determined that subjects identified by methods described herein as having certain TME types (e.g., BLBC TME type G, IE or TLS; LNLBC TME type IE or IE/F; H2EBC TME type IE or IE-med) are characterized has having an increased likelihood of responding to immunotherapeutic agents, for example immune checkpoint inhibitors. Conversely, the inventors have determined that subjects having other TME types (e.g., TME type F of BLBC, LNLBC, or H2EBC) are characterized has having an increased likelihood of responding to anti-VEGF agents, while subjects having BLBC TME type D are characterized has having an increased likelihood of responding to a TKI. Thus, the techniques developed by the inventors and described herein improve patient treatment and associated outcomes by increasing patient comfort, and avoiding toxic side effects of chemotherapy that is not expected to be effective for the subject.

*Breast Cancer*

Aspects of the disclosure relate to methods of determining the breast cancer TME type of a subject having, suspected of having, or at risk of having breast cancer. As used herein, a subject may be a mammal, for example a human, non-human primate, rodent (e.g., rat, mouse, guinea pig, etc.), dog, cat, horse etc. In some embodiments, the subject is a human. The terms "individual" or "subject" may be used interchangeably with "patient." As used herein, "breast cancer" or "BC" refers to any breast cancer, for example, ductal carcinoma in situ, invasive ductal carcinoma, inflammatory breast cancer, and metastatic breast cancer, or any other type of malignancy caused by one or more various genetic mutations in the body that affect cells (originally present in or metastasized to) the breast and/or tissue surrounding the breast of a subject. As used herein, "cancer" refers to any malignant and/or invasive growth or tumor caused by abnormal cell growth in a subject, including solid tumors, blood cancer, bone marrow or lymphoid cancer, etc. According to the claims, a breast cancer is a basal-like breast cancer (BLBC). In some examples not part of the claims, a breast cancer is a luminal breast cancer (including luminal A and luminal B or normal-like breast cancer; LNLBC), or HER2-enriched breast cancer (H2EBC).

A subject having BC may exhibit one or more signs or symptoms of BC, for example the presence of cancerous cells (e.g., tumor cells), lumps on the breast, fever, swelling, bleeding, nausea and vomiting, and weight loss. In some embodiments, a subject having BC does not exhibit one or more signs or symptoms of BC. In some embodiments, a subject having BC has been diagnosed by a medical professional (e.g., a licensed physician) as having BC based upon one or more assays (e.g., clinical assays, molecular diagnostics, etc.) that indicate that the subject has BC, even in the absence of one or more signs or symptoms. In some embodiments, the intrinsic molecular subtype of a BC subject has been determined.

A subject suspected of having BC typically exhibits one or more signs or symptoms of BC. In some embodiments, a subject suspected of having BC exhibits one or more signs or symptoms of BC but has not been diagnosed by a medical professional (e.g., a licensed physician) and/or has not received a test result (e.g., a clinical assay, molecular diagnostic, etc.) indicating that the subject has BC.

A subject at risk of having BC may or may not exhibit one or more signs or symptoms of BC. In some embodiments, a subject at risk of having BC comprises one or more risk factors that increase the likelihood that the subject will develop BC. Examples of risk factors include the presence of pre-cancerous cells in a clinical sample, having one or more genetic mutations that predispose the subject to developing cancer (e.g., BC), taking one or more medications that increase the likelihood that the subject will develop cancer (e.g., BC), family history of BC, and the like.

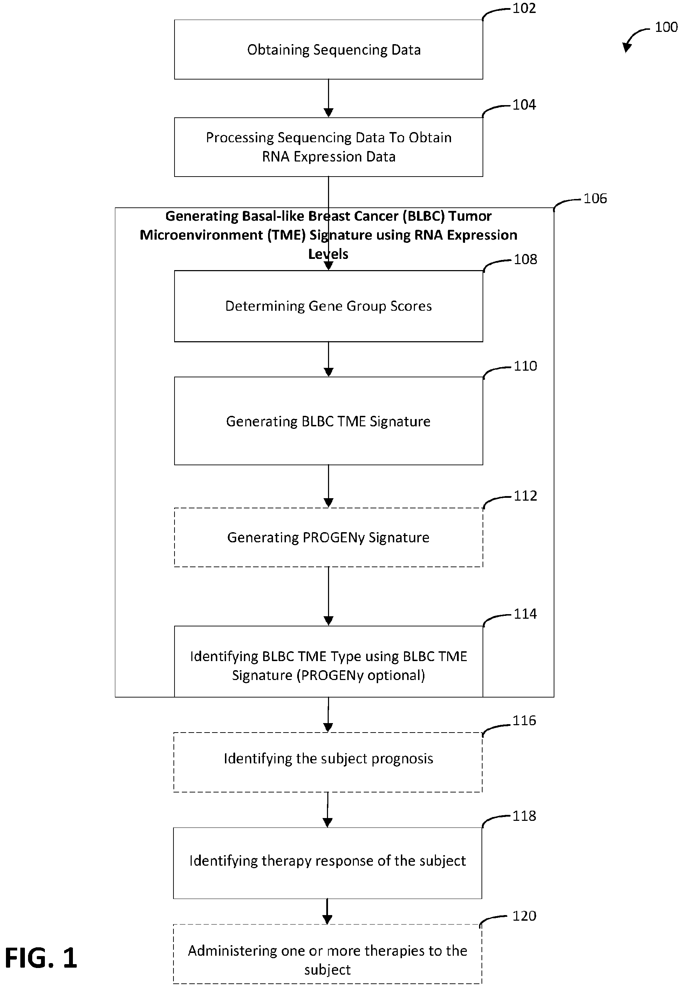

FIG. 1 is a flowchart of an illustrative process 100 for determining a BLBC TME signature for a subject, using the determined BLBC TME signature to identify the BLBC TME type for the subject, and using the BLBC TME type of the subject to identify whether or not the subject is likely to respond to immunotherapy.

Various (e.g., some or all) acts of process 100 may be implemented using any suitable computing device(s). For example, in some embodiments, one or more acts of the illustrative process 100 may be implemented in a clinical or laboratory setting. For example, one or more acts of the process 100 may be implemented on a computing device that is located within the clinical or laboratory setting. In some embodiments, the computing device may directly obtain RNA expression data from a sequencing apparatus located within the clinical or laboratory setting. For example, a computing device included in the sequencing apparatus may directly obtain the RNA expression data from the sequencing apparatus. In some embodiments, the computing device may indirectly obtain RNA expression data from a sequencing apparatus that is located within or external to the clinical or laboratory setting. For example, a computing device that is located within the clinical or laboratory setting may obtain expression data via a communication network, such as Internet or any other suitable network, as aspects of the technology described herein are not limited to any particular communication network.

Additionally or alternatively, one or more acts of the illustrative process 100 may be implemented in a setting that is remote from a clinical or laboratory setting. For example, the one or more acts of process 100 may be implemented on a computing device that is located externally from a clinical or laboratory setting. In this case, the computing device may indirectly obtain RNA expression data that is generated using a sequencing apparatus located within or external to a clinical or laboratory setting. For example, the expression data may be provided to computing device via a communication network, such as Internet or any other suitable network.

It should be appreciated that, in some embodiments, not all acts of process 100, as illustrated in FIG. 1, may be implemented using one or more computing devices. For example, the act 120 of administering one or more immunotherapies to the subject may be implemented manually (e.g., by a clinician).

Process 100 begins at act 102 where sequencing data for a subject is obtained. In some embodiments, the sequencing data may be obtained by sequencing a biological sample (e.g., breast biopsy and/or tumor tissue) obtained from the subject using any suitable sequencing technique. The sequencing data may include sequencing data of any suitable type, from any suitable source, and be in any suitable format. Examples of sequencing data, sources of sequencing data, and formats of sequencing data are described herein including in the section called "Obtaining RNA Expression Data."

As one illustrative example, in some embodiments, the sequencing data may comprise bulk sequencing data. The bulk sequencing data may comprise at least 1 million reads, at least 5 million reads, at least 10 million reads, at least 20 million reads, at least 50 million reads, or at least 100 million reads. In some embodiments, the sequencing data comprises bulk RNA sequencing (RNA-seq) data, single cell RNA sequencing (scRNA-seq) data, or next generation sequencing (NGS) data. In some embodiments, the sequencing data comprises microarray data.

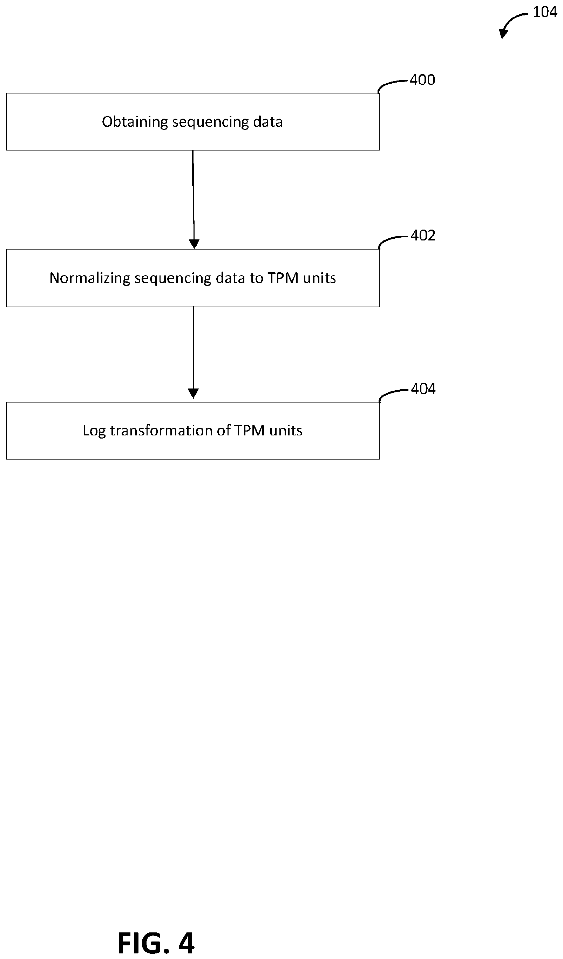

Next, process 100 proceeds to act 104, where the sequencing data obtained at act 102 is processed to obtain RNA expression data. This may be done in any suitable way and may involve normalizing bulk sequencing data to transcripts-per-million (TPM) units (or other units) and/or log transforming the RNA expression levels in TPM units. Converting the data to TPM units and normalization are described herein including with reference to FIG. 4.

Next, process 100 proceeds to act 106, where a basal-like breast cancer (BLBC) tumor microenvironment (TME) signature is generated for the subject using the RNA expression data generated at act 104 (e.g., from bulk-sequencing data, converted to TPM units and subsequently log-normalized, as described herein including with reference to FIG. 4).

As described herein, in some embodiments, a BLBC TME signature comprises two or more (e.g., 2, 3, 4, 5, 6, 7, 8, 9, 10, 11, 12, 13, 14, 15, 16, 17, 18, 19, 20, 21, 22, 23, 24, 25, 26, 27, 28, etc.) gene group scores. In some embodiments, the two or more gene group scores comprise gene group scores (which may also be referred to as gene group enrichment scores or gene group expression scores) for some or all of the gene groups shown in Table 1.

Accordingly, act 106 comprises: act 108 where the gene group scores are determined, act 110 where the BLBC TME signature is determined using the gene group determined at act 108, and act 114 where the BLBC TME type is determined by using the BLBC TME signature determined at act 110. In some embodiments, determining the gene group scores comprises determining, for each of multiple (e.g., some or all of the) gene groups listed in Table 1, a respective gene group score. In some embodiments, determining the gene group scores comprises determining respective gene group scores for 2, 3, 4, 5, 6, 7, 8, 9, 10, 11, 12, 13, 14, 15, 16, 17, 18, 19, 20, 21, 22, 23, 24, 25, 26, 27, or 28 gene groups (e.g., gene groups listed in Table 1). In some embodiments, determining the gene group scores further comprises determining one or more PROGENy signatures, as shown in act 112. The gene group score for a particular gene group may be determined using RNA expression levels for at least some of the genes in the gene group (e.g., the RNA expression levels obtained at act 104). The RNA expression levels is processed using a gene set enrichment analysis (GSEA) technique to determine the score for the particular gene group.

For example, in some embodiments, determining the BLBC TME signature comprises: determining gene group scores using the RNA expression levels for at least three genes from each of at least two of the gene groups, the gene groups including: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

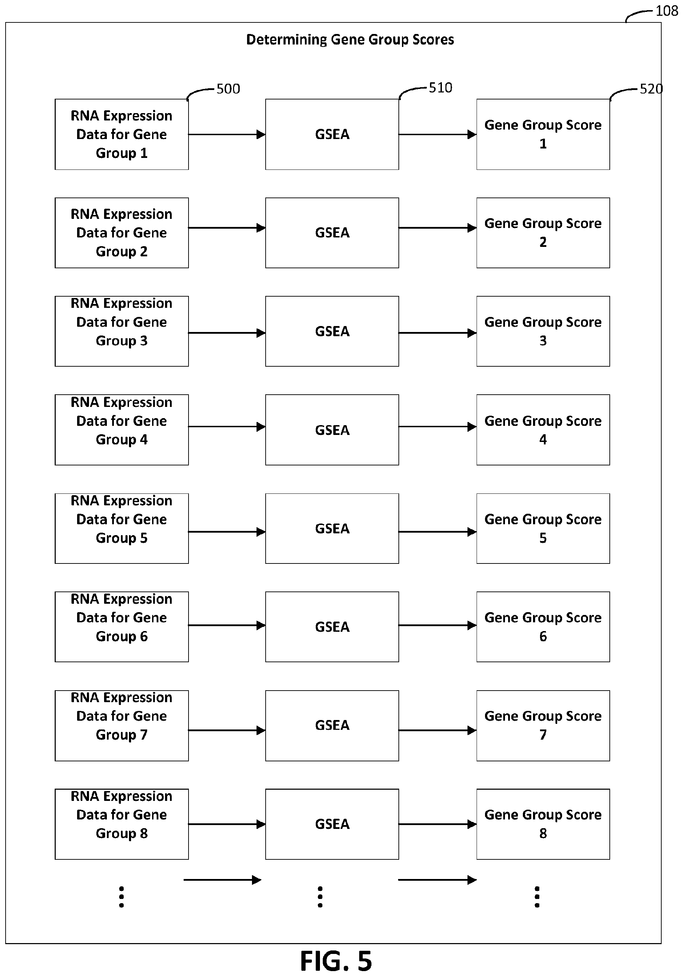

Aspects of determining the gene group scores are described herein, including with reference to FIG. 5 and in the Section titled "Gene Expression Signatures".

As described above, at act 110, the BLBC TME signature is generated. In some embodiments, the BLBC TME signature consists of only gene group scores for one or more (e.g., all) of the gene groups listed in Table 1. In some embodiments, the BLBC TME signature comprises gene group scores for at least 2, 3, 4, 5, 6, 7, 8, 9, 10, 11, 12, 13, 14, 15, 16, 17, 18, 19, 20, 21, 22, 23, 24, 25, 26, 27, or 28 gene groups listed in Table 1. In some embodiments, each gene group score for a particular gene group is determined using RNA expression levels of some or all (e.g., at least 2, 3, 4, 5, 6, 7, 8, 9, 10, 11, 12, 13, 14, 15, 16, 17, 18, 19, 20, etc.) of the genes of each gene group listed in Table 1. In other embodiments, the BLBC TME signature includes one or more other gene group scores in addition to the gene group scores listed in Table 1.

Next, process 100 proceeds to act 114, where a BLBC TME type is identified for the subject using the BLBC TME signature generated at act 110. This may be done in any suitable way. For example, in some embodiments, the each of the possible BLBC TME types is associated with a respective plurality of BLBC TME signature clusters. In such embodiments, a BLBC TME type for the subject may be identified by associating the BLBC TME signature of the subject with a particular one of the plurality of BLBC TME signature clusters; and identifying the BLBC TME type for the subject as the BLBC TME type corresponding to the particular one of the plurality of BLBC TME signature clusters to which the BLBC TME signature of the subject is associated. Examples of BLBC TME types are described herein. Aspects of identifying a BLBC TME type for a subject are described herein including in the section below titled "Generating TME Signature and Identifying TME Type."

In some embodiments, act 114 further comprising using one or more PROGENy signatures in addition to the BLBC TME signature to identify the BLBC TME type. In some embodiments, the one or more PROGENy signatures are selected from TGFb, NFkB, and/or VEGF signaling PROGENy signatures.

As described above, a subject's BLBC TME type is identified at act 114. In some embodiments, the BLBC TME type of a subject is identified to be one of the following BLBC TME types: Immune Enriched (IE) type, TLS (TLS) type (also referred to as B-cell enriched), Desert (D) type, Fibrotic (F) type, and Granulocyte enriched (G) type.

Next, process 100 proceeds to act 118, where the subject's likelihood of responding to a therapy is identified using the BLBC TME type identified at act 114. In some embodiments, when a subject is identified as having a BLBC TME type IE or BLBC TME type TLS at act 114, the subject is identified as having an increased likelihood of responding to an immunotherapy (e.g., a PD1 antibody, such as pembrolizumab) relative to a subject having other BLBC TME types, at act 118. In some embodiments, when a subject is identified as having a BLBC TME type F at act 114, the subject is identified as having an increased likelihood of responding to an anti-VEGF therapy relative to a subject having other BLBC TME types, at act 118. In some embodiments, when a subject is identified as having a BLBC TME type D at act 114, the subject is identified as having an increased likelihood of responding to a TKI relative to a subject having other BLBC TME types, at act 118. Aspects of identifying whether or not a subject is likely to respond to a therapy are described herein including in the section below titled "Therapeutic Indications."

In some embodiments, process 100 completes after act 118 completes. In some such embodiments, the determined BLBC TME signature and/or identified BLBC TME type, and/or the identified likelihood the subject will respond to a therapy may be stored for subsequent use, provided to one or more recipients (e.g., a clinician, a researcher, etc.), and/or used to update the BLBC TME signature clusters (as described hereinbelow).

However, in some embodiments, one or more other acts are performed after act 114. For example, in the illustrated embodiment of FIG. 1, process 100 may include one or more of optional acts 116, and 120 shown using dashed lines in FIG. 1. For example, at act 116, a prognosis may be identified for the subject. In another example, when a subject is identified as having BLBC TME type IE or BLBC TME type TLS at act 114, and/or identified as having an increased likelihood of responding to immunotherapy at act 118, the subject is administered one or more immunotherapies at act 120. Examples of immunotherapies and other therapies are provided herein.

It should be appreciated that although acts 112, 116, and 120 are indicated as optional in the example of FIG. 1, in other embodiments, one or more other acts may be optional (in addition to or instead of acts 112, 116, and 120). For example, in some embodiments, acts 102 and 104 may be optional (e.g., when the sequencing data is obtained and processed to obtain RNA expression data previously, process 100 may begin at act 106 by accessing the previously obtained RNA expression data). In some embodiments, the process 100 may comprise acts 102, 104, 106, 118 and 120, without acts 114 and 116. In some embodiments, the process 100 may comprise acts 102, 104, 106, 116, 114, 118, and 120 without act 116.

**Table 1. *Gene expression signatures used to generate BLBC TME Signatures***

| MHC I | HLA-C, TAPBP, HLA-B, B2M, TAP2, HLA-A, TAP1, NLRC5 |
|---|---|
| MHC II | HLA-DQB1, HLA-DMA, HLA-DMB, HLA-DRA, CIITA, HLA-DQA1, HLA-DPB1, HLA-DRB1, HLA-DPA1 |
| Coactivation molecules | TNFRSF4, CD27, CD80, CD40LG, TNFRSF9, CD40, CD28, ICOSLG, CD83, TNFSF9, CD70, TNFSF4, ICOS, CD86 |
| Effector cells | ZAP70, GZMB, GZMK, IFNG, FASLG, EOMES, TBX21, GZMA, CD8A, GNLY, PRF1, CD8B |
| NK cells | NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160 |
| T cells | TRBC2, CD3E, CD3G, ITK, CD28, TRBC1, TRAT1, TBX21, CD5, TRAC, CD3D |
| B cells | CD22, TNFRSF13C, STAP1, CD79B, PAX5, CR2, TNFRSF13B, CD79A, TNFRSF17, FCRL5, MS4A1, CD19, BLK |
| M1 signatures | CMKLR1, SOCS3, IRF5, NOS2, IL1B, IL12B, IL23A, TNF, IL12A |
| Th1 signature | IL21, TBX21, IL12RB2, CD40LG, IFNG, IL2, STAT4 |
| Antitumor cytokines | CCL3, IL21, IFNB1, IFNA2, TNF, TNFSF10 |

(continued)

| Checkpoint inhibition | PDCD1, BTLA, HAVCR2, CD274, VSIR, LAG3, TIGIT, PDCD1LG2, CTLA4 |
|---|---|
| Treg | IKZF2, TNFRSF18, IL10, FOXP3, CCR8, IKZF4, CTLA4 |
| Neutrophil signature | CD177, FFAR2, PGLYRP1, CXCR1, MPO, CXCR2, ELANE, CTSG, PRTN3, FCGR3B |
| Granulocyte traffic | CCR3, KITLG, CCL11, CXCL2, CXCR1, CXCR2, CXCL8, CXCL1, CXCL5 |
| MDSC | ARG1, IL6, CYBB, IL10, PTGS2, IDO1, IL4I1 |
| Macrophages | MRC1, SIGLEC1, MSR1, CD163, CSF1R, CD68, IL4I1, IL10 |
| Th2 signature | IL13, CCR4, IL10, IL4, IL5 |
| Protumor cytokines | TGFB2, MIF, IL6, TGFB3, TGFB1, IL22, IL10 |
| CAF | COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, LRP1 |
| Matrix | LAMC2, TNC, COL11A1, VTN, LAMB3, COL1A1, FN1, LAMA3, LGALS9, COL1A2, COL4A1, COL5A1, ELN, LGALS7, COL3A1 |
| Angiogenesis | VEGFC, VEGFA, PDGFC, KDR, CDH5, VEGFB, PGF, TEK, ANGPT2, CXCR2, FLT1, CXCL8, VWF, ANGPT1, CXCL5 |
| Endothelium | KDR, CDH5, NOS3, VCAM1, VWF, FLT1, MMRN1, CLEC14A, ENG, MMRN2 |
| Proliferation rate | CCND1, CCNB1, CETN3, CDK2, E2F1, AURKA, BUB1, AURKB, PLK1, MCM6, ESCO2, MYBL2, MKI67, MCM2, CCNE1 |
| EMT signature | CDH2, ZEB1, ZEB2, TWIST1, SNAI1, SNAI2, TWIST2 |
| TLS | CXCL13, CCL19, LAMP3, SELL, CXCR4, CCL21, CCR7, CD86, BCL6 |
| Follicular dendritic cells | FDCSP, SERPINE2, PRNP, PDPN, LTBR, BST1, CLU, C1S, C4A |
| Follicular B helper T cells | SH2D1A, IL6, MAF, CD40LG, IL21, ICOS, IL4, CD84, CXCR5, BCL6 |
| Granulocytes | CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, PRSS33 |

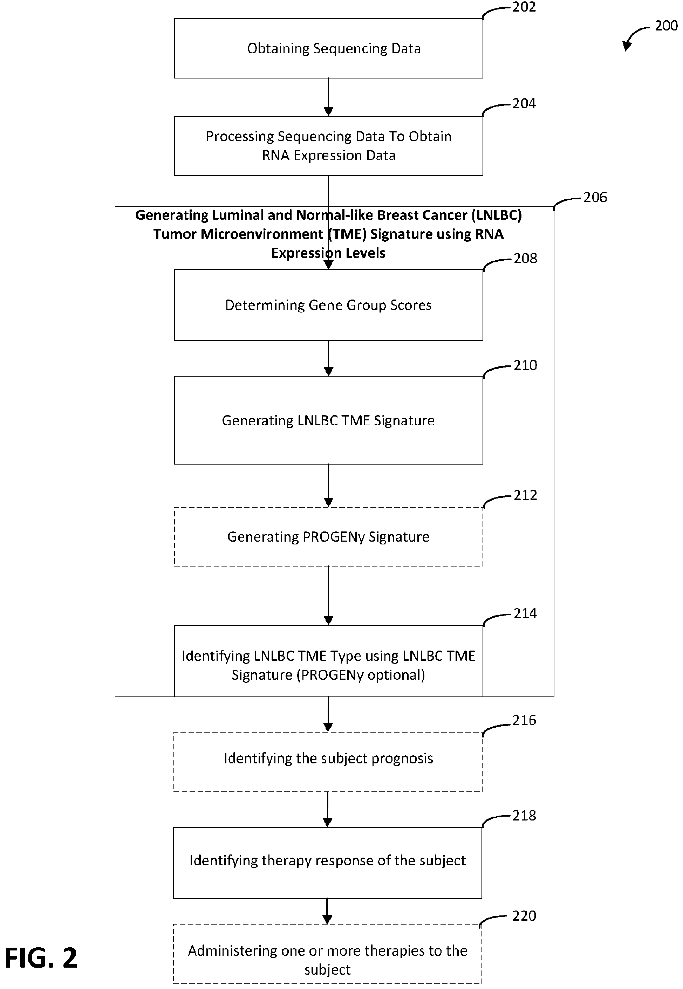

FIG. 2 is a flowchart of an illustrative process 200 for determining a LNLBC TME signature for a subject, using the determined LNLBC TME signature to identify the LNLBC TME type for the subject, and using the LNLBC TME type of the subject to identify whether or not the subject is likely to respond to immunotherapy.

Various (e.g., some or all) acts of process 200 may be implemented using any suitable computing device(s). For example, one or more acts of the illustrative process 200 may be implemented in a clinical or laboratory setting. For example, one or more acts of the process 200 may be implemented on a computing device that is located within the clinical or laboratory setting. In some embodiments, the computing device may directly obtain RNA expression data from a sequencing apparatus located within the clinical or laboratory setting. For example, a computing device included in the sequencing apparatus may directly obtain the RNA expression data from the sequencing apparatus. In some embodiments, the computing device may indirectly obtain RNA expression data from a sequencing apparatus that is located within or external to the clinical or laboratory setting. For example, a computing device that is located within the clinical or laboratory setting may obtain expression data via a communication network, such as Internet or any other suitable network, as aspects of the technology described herein are not limited to any particular communication network.

Additionally or alternatively, one or more acts of the illustrative process 200 may be implemented in a setting that is remote from a clinical or laboratory setting. For example, the one or more acts of process 200 may be implemented on a computing device that is located externally from a clinical or laboratory setting. In this case, the computing device may indirectly obtain RNA expression data that is generated using a sequencing apparatus located within or external to a clinical or laboratory setting. For example, the expression data may be provided to computing device via a communication network, such as Internet or any other suitable network.

It should be appreciated that not all acts of process 200, as illustrated in FIG. 2, may be implemented using one or more computing devices. For example, the act 220 of administering one or more therapies to the subject may be implemented manually (e.g., by a clinician).

Process 200 begins at act 202 where sequencing data for a subject is obtained. The sequencing data may be obtained by sequencing a biological sample (e.g., breast biopsy and/or tumor tissue) obtained from the subject using any suitable sequencing technique. The sequencing data may include sequencing data of any suitable type, from any suitable source, and be in any suitable format. Examples of sequencing data, sources of sequencing data, and formats of sequencing data are described herein including in the section called "Obtaining RNA Expression Data."

As one illustrative example, the sequencing data may comprise bulk sequencing data. The bulk sequencing data may comprise at least 1 million reads, at least 5 million reads, at least 10 million reads, at least 20 million reads, at least 50 million reads, or at least 100 million reads. In some examples, the sequencing data comprises bulk RNA sequencing (RNA-seq) data, single cell RNA sequencing (scRNA-seq) data, or next generation sequencing (NGS) data. In some examples, the sequencing data comprises microarray data.

Next, process 200 proceeds to act 204, where the sequencing data obtained at act 202 is processed to obtain RNA expression data. This may be done in any suitable way and may involve normalizing bulk sequencing data to transcripts-per-million (TPM) units (or other units) and/or log transforming the RNA expression levels in TPM units. Converting the data to TPM units and normalization are described herein including with reference to FIG. 4. However, the skilled person will recognize that the processes described in acts 104 and 204 may be identical.

Next, process 200 proceeds to act 206, where a luminal or normal-like breast cancer (LNLBC) tumor microenvironment (TME) signature is generated for the subject using the RNA expression data generated at act 204 (e.g., from bulk-sequencing data, converted to TPM units and subsequently log-normalized, as described herein including with reference to FIG. 4).

As described herein, a LNLBC TME signature comprises two or more (e.g., 2, 3, 4, 5, 6, 7, 8, 9, 10, 11, 12, 13, 14, 15, 16, 17, 18, 19, 20, 21, 22, 23, 24, etc.) gene group scores. In some examples, the two or more gene group scores comprise gene group scores (which may also be referred to as gene group enrichment scores or gene group expression scores) for some or all of the gene groups shown in Table 2.

Accordingly, act 206 comprises: act 208 where the gene group scores are determined, act 210 where the LNLBC TME signature is determined using the gene group determined at act 208, and act 214 where the LNLBC TME type is determined by using the LNLBC TME signature determined at act 210. In some examples, determining the gene group scores comprises determining, for each of multiple (e.g., some or all of the) gene groups listed in Table 2, a respective gene group score. In some examples, determining the gene group scores comprises determining respective gene group scores for 2, 3, 4, 5, 6, 7, 8, 9, 10, 11, 12, 13, 14, 15, 16, 17, 18, 19, 20, 21, 22, 23, 24, 25, 26, 27, or 28 gene groups (e.g., gene groups listed in Table 2). In some examples, determining the gene group scores further comprises determining one or more PROGENy signatures, as shown in act 212. The gene group score for a particular gene group may be determined using RNA expression levels for at least some of the genes in the gene group (e.g., the RNA expression levels obtained at act 204). The RNA expression levels may be processed using a gene set enrichment analysis (GSEA) technique to determine the score for the particular gene group.

For example, determining the LNLBC TME signature may comprise: determining gene group scores using the RNA expression levels for at least three genes from each of at least two of the gene groups, the gene groups including: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; T cell traffic group: CXCL10, CX3CL1, CX3CR1, CXCL11, CXCR3, CCL5, CCL3, CXCL9, and CCL4; B cells group: CD22, TNFRSF13C, STAP1, CD79B, PAX5, CR2, TNFRSF13B, CD79A, TNFRSF17, FCRL5, MS4A1, CD19, and BLK; M1 signatures group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Macrophages group: MRC1, SIGLEC1, MSR1, CD163, CSF1R, CD68, IL4I1, and IL10; Macrophage DC traffic group: CCL8, CSF1R, CSF1, CCR2, XCL1, XCR1, CCL2, and CCL7; Protumor cytokines group: TGFB2, MIF, IL6, TGFB3, TGFB1, IL22, and IL10; Cancer-associated fibroblasts (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Matrix remodeling group: ADAMTS4, ADAMTS5, CA9, LOX, MMP1, MMP11, MMP12, MMP2, MMP3, MMP7, MMP9, and PLOD2; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; and EMT signature group: CDH2, ZEB1, ZEB2, TWIST1, SNAI1, SNAI2, and TWIST2.

Aspects of determining the gene group scores are described herein, including with reference to FIG. 5 and in the Section titled "Gene Expression Signatures".

As described above, at act 210, the LNLBC TME signature is generated. In some examples, the LNLBC TME signature consists of only gene group scores for one or more (e.g., all) of the gene groups listed in Table 2. In some examples, the LNLBC TME signature comprises gene group scores for at least 2, 3, 4, 5, 6, 7, 8, 9, 10, 11, 12, 13, 14, 15, 16, 17, 18, 19, 20, 21, 22, 23, or 24 gene groups listed in Table 2. In some examples, each gene group score for a particular gene group is determined using RNA expression levels of some or all (e.g., at least 2, 3, 4, 5, 6, 7, 8, 9, 10, 11, 12, 13, 14, 15, 16, 17, 18, 19, 20, etc.) of the genes of each gene group listed in Table 2. In other examples, the LNLBC TME signature includes one or more other gene group scores in addition to the gene group scores listed in Table 2.

Next, process 200 proceeds to act 214, where a LNLBC TME type is identified for the subject using the LNLBC TME signature generated at act 210. This may be done in any suitable way. For example, the each of the possible LNLBC TME types may be associated with a respective plurality of LNLBC TME signature clusters. In such examples, a LNLBC TME type for the subject may be identified by associating the LNLBC TME signature of the subject with a particular one of the plurality of LNLBC TME signature clusters; and identifying the LNLBC TME type for the subject as the LNLBC TME type corresponding to the particular one of the plurality of LNLBC TME signature clusters to which the LNLBC TME signature of the subject is associated. Examples of LNLBC TME types are described herein. Aspects of identifying a LNLBC TME type for a subject are described herein including in the section below titled "Generating TME Signature and Identifying TME Type."

In some examples, act 214 further comprising using one or more PROGENy signatures in addition to the LNLBC TME signature to identify the LNLBC TME type. In some examples, the one or more PROGENy signatures comprises an Estrogen PROGENy signature.

As described above, a subject's LNLBC TME type is identified at act 214. In some examples, the LNLBC TME type of a subject is identified to be one of the following LNLBC TME types: Immune Desert (D) type, Fibrotic (F) type, Immune-Enriched Non-fibrotic (IE) type, Immune-Enriched Fibrotic (IE/F) type, and Angiogenic (E) type.

Next, process 200 proceeds to act 218, where the subject's likelihood of responding to a therapy is identified using the LNLBC TME type identified at act 214. In some examples, when a subject is identified as having a LNLBC TME type IE or LNLBC TME type IE/F at act 214, the subject is identified as having an increased likelihood of responding to an immunotherapy (e.g., a PD1 antibody, such as pembrolizumab) relative to a subject having other LNLBC TME types, at act 218. In some examples, when a subject is identified as having a LNLBC TME type F at act 214, the subject is identified as having an increased likelihood of responding to an anti-VEGF therapy relative to a subject having other LNLBC TME types, at act 218. Aspects of identifying whether or not a subject is likely to respond to a therapy are described herein including in the section below titled "Therapeutic Indications."

In some examples, process 200 completes after act 218 completes. In some such examples, the determined LNLBC TME signature and/or identified LNLBC TME type, and/or the identified likelihood the subject will respond to a therapy may be stored for subsequent use, provided to one or more recipients (e.g., a clinician, a researcher, etc.), and/or used to update the LNLBC TME signature clusters (as described hereinbelow).

However, in some examples, one or more other acts are performed after act 214. For example, in the illustrated embodiment of FIG. 2, process 200 may include one or more of optional acts 216, and 220 shown using dashed lines in FIG. 2. For example, at act 216, a prognosis may be identified for the subject. In another example, when a subject is identified as having LNLBC TME type IE or LNLBC TME type IE/F at act 214, and/or identified as having an increased likelihood of responding to immunotherapy at act 218, the subject is administered one or more immunotherapies at act 220. Examples of immunotherapies are provided herein.

It should be appreciated that although acts 212, 216, and 220 are indicated as optional in the example of FIG. 2, in other embodiments, one or more other acts may be optional (in addition to or instead of acts 212, 216, and 220). For example, in some embodiments, acts 202 and 204 may be optional (e.g., when the sequencing data is obtained and processed to obtain RNA expression data previously, process 200 may begin at act 206 by accessing the previously obtained RNA expression data). In some embodiments, the process 200 may comprise acts 202, 204, 206, 218 and 220, without acts 214 and 216. In some embodiments, the process 200 may comprise acts 202, 204, 206, 216, 214, 218, and 220 without act 216.

**Table 2. *Gene expression signatures used to generate LNLBC TME Signatures***

| | |
|---|---|
| MHC I | *HLA-C, TAPBP, HLA-B, B2M, TAP2, HLA-A, TAP1, NLRC5* |

(continued)

| | |
|---|---|
| MHC II | HLA-OQB1, HLA-DMA, HLA-DMB, HLA-DRA, CIITA, HLA-DQA1, HLA-DPB1, HLA-DRB1, HLA-DPA1 |
| Effector cells | ZAP70, GZMB, GZMK, IFNG, FASLG, EOMES, TBX21, GZMA, CD8A, GNLY, PRF1, CD8B |
| NK cells | NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160 |
| T cells | TRBC2, CD3E, CD3G, ITK, CD28, TRBC1, TRAT1, TBX21, CD5, TRAC, CD3D |
| T cell traffic | CXCL10, CX3CL1, CX3CR1, CXCL11, CXCR3, CCL5, CCL3, CXCL9, CCL4 |
| B cells | CD22, TNFRSF13C, STAP1, CD79B, PAX5, CR2, TNFRSF13B, CD79A, TNFRSF17, FCRL5, MS4A1, CD19, BLK |
| M1 signatures | CMKLR1, SOCS3, IRF5, NOS2, IL1B, IL12B, IL23A, TNF, IL12A |
| Th1 signature | IL21, TBX21, IL12RB2, CD40LG, IFNG, IL2, STAT4 |
| Antitumor cytokines | CCL3, IL21, IFNB1, IFNA2, TNF, TNFSF10 |
| Checkpoint inhibition | PDCD1, BTLA, HAVCR2, CD274, VSIR, LAG3, TIGIT, PDCD1LG2, CTLA4 |
| Treg | IKZF2, TNFRSF18, IL10, FOXP3, CCR8, IKZF4, CTLA4 |
| Granulocyte traffic | CCR3, KITLG, CCL11, CXCL2, CXCR1, CXCR2, CXCL8, CXCL1, CXCL5 |
| MDSC | ARG1, IL6, CYBB, IL10, PTGS2, IDO1, IL4I1 |
| Macrophages | MRC1, SIGLEC1, MSR1, CD163, CSF1R, CD68, IL4I1, IL10 |
| Macrophage DC traffic | CCL8, CSF1R, CSF1, CCR2, XCL1, XCR1, CCL2, CCL7 |
| Protumor cytokines | TGFB2, MIF, IL6, TGFB3, TGFB1, IL22, IL10 |
| CAF | COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, LRP1 |
| Matrix | LAMC2, TNC, COL11A1, VTN, LAMB3, COL1A1, FN1, LAMA3, LGALS9, COL1A2, COL4A1, COL5A1, ELN, LGALS7, COL3A1 |
| Matrix remodeling | ADAMTS4, ADAMTS5, CA9, LOX, MMP1, MMP11, MMP12, MMP2, MMP3, MMP7, MMP9, PLOD2 |
| Angiogenesis | VEGFC, VEGFA, PDGFC, KDR, CDH5, VEGFB, PGF, TEK, ANGPT2, CXCR2, FLT1, CXCL8, VWF, ANGPT1, CXCL5 |
| Endothelium | KDR, CDH5, NOS3, VCAM1, VWF, FLT1, MMRN1, CLEC14A, ENG, MMRN2 |
| Proliferation rate | CCND1, CCNB1, CETN3, CDK2, E2F1, AURKA, BUB1, AURKB, PLK1, MCM6, ESCO2, MYBL2, MKI67, MCM2, CCNE1 |
| EMT signature | CDH2, ZEB1, ZEB2, TWIST1, SNAI1, SNAI2, TWIST2 |

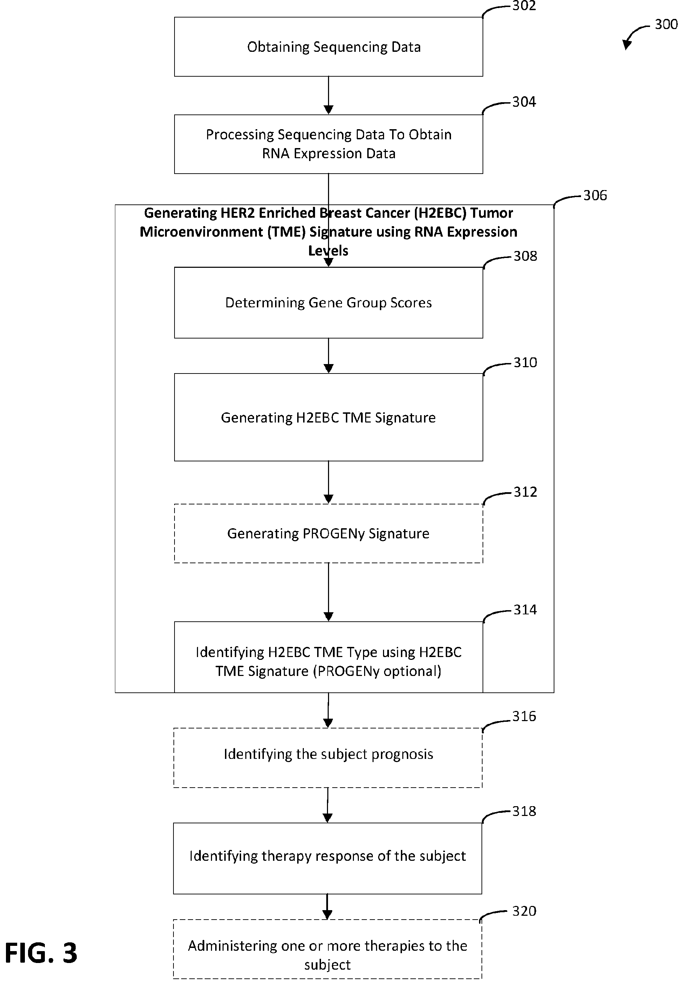

FIG. 3 is a flowchart of an illustrative process 300 for determining a H2EBC TME signature for a subject, using the determined H2EBC TME signature to identify the H2EBC TME type for the subject, and using the H2EBC TME type of the subject to identify whether or not the subject is likely to respond to immunotherapy.

Various (e.g., some or all) acts of process 300 may be implemented using any suitable computing device(s). For example, ione or more acts of the illustrative process 300 may be implemented in a clinical or laboratory setting. For example, one or more acts of the process 300 may be implemented on a computing device that is located within the clinical or laboratory setting. The computing device may directly obtain RNA expression data from a sequencing apparatus located within the clinical or laboratory setting. For example, a computing device included in the sequencing apparatus may directly obtain the RNA expression data from the sequencing apparatus. In some examples, the computing device may indirectly obtain RNA expression data from a sequencing apparatus that is located within or external to the clinical or laboratory setting. For example, a computing device that is located within the clinical or laboratory setting may obtain expression data via a communication network, such as Internet or any other suitable network, as aspects of the technology described herein are not limited to any particular communication network.

Additionally or alternatively, one or more acts of the illustrative process 300 may be implemented in a setting that is remote from a clinical or laboratory setting. For example, the one or more acts of process 300 may be implemented on a computing device that is located externally from a clinical or laboratory setting. In this case, the computing device may indirectly obtain RNA expression data that is generated using a sequencing apparatus located within or external to a clinical or laboratory setting. For example, the expression data may be provided to computing device via a communication network, such as Internet or any other suitable network.

It should be appreciated that not all acts of process 300, as illustrated in FIG. 3, may be implemented using one or more computing devices. For example, the act 320 of administering one or more therapies to the subject may be implemented manually (e.g., by a clinician).

Process 300 begins at act 302 where sequencing data for a subject is obtained. In some examples, the sequencing data may be obtained by sequencing a biological sample (e.g., breast biopsy and/or tumor tissue) obtained from the subject using any suitable sequencing technique. The sequencing data may include sequencing data of any suitable type, from any suitable source, and be in any suitable format. Examples of sequencing data, sources of sequencing data, and formats of sequencing data are described herein including in the section called "Obtaining RNA Expression Data."

As one illustrative example, the sequencing data may comprise bulk sequencing data. The bulk sequencing data may comprise at least 1 million reads, at least 5 million reads, at least 10 million reads, at least 20 million reads, at least 50 million reads, or at least 100 million reads. In some examples, the sequencing data comprises bulk RNA sequencing (RNA-seq) data, single cell RNA sequencing (scRNA-seq) data, or next generation sequencing (NGS) data. In some examples, the sequencing data comprises microarray data.

Next, process 300 proceeds to act 304, where the sequencing data obtained at act 302 is processed to obtain RNA expression data. This may be done in any suitable way and may involve normalizing bulk sequencing data to transcripts-per-million (TPM) units (or other units) and/or log transforming the RNA expression levels in TPM units. Converting the data to TPM units and normalization are described herein including with reference to FIG. 4. However, the skilled person will recognize that the processes described in acts 104 and 304 may be identical.

Next, process 300 proceeds to act 306, where a HER2-enriched breast cancer (H2EBC) tumor microenvironment (TME) signature is generated for the subject using the RNA expression data generated at act 304 (e.g., from bulk-sequencing data, converted to TPM units and subsequently log-normalized, as described herein including with reference to FIG. 4).

As described herein, in some examples, a H2EBC TME signature comprises two or more (e.g., 2, 3, 4, 5, 6, 7, 8, 9, 10, 11, 12, 13, 14, 15, 16, 17, 18, 19, 20, 21, 22, 23, 24, 25, 26, 27, 28, 29, etc.) gene group scores. In some examples, the two or more gene group scores comprise gene group scores (which may also be referred to as gene group enrichment scores or gene group expression scores) for some or all of the gene groups shown in Table 3.

Accordingly, act 306 comprises: act 308 where the gene group scores are determined, act 310 where the H2EBC TME signature is determined using the gene group determined at act 308, and act 314 where the H2EBC TME type is determined by using the H2EBC TME signature determined at act 310. In some examples, determining the gene group scores comprises determining, for each of multiple (e.g., some or all of the) gene groups listed in Table 3, a respective gene group score. In some examples, determining the gene group scores comprises determining respective gene group scores for 2, 3, 4, 5, 6, 7, 8, 9, 10, 11, 12, 13, 14, 15, 16, 17, 18, 19, 20, 21, 22, 23, 24, 25, 26, 27, or 28 gene groups (e.g., gene groups listed in Table 3). In some examples, determining the gene group scores further comprises determining one or more PROGENy signatures, as shown in act 312. The gene group score for a particular gene group may be determined using RNA expression levels for at least some of the genes in the gene group (e.g., the RNA expression levels obtained at act 304). The RNA expression levels may be processed using a gene set enrichment analysis (GSEA) technique to determine the score for the particular gene group.

For example determining the H2EBC TME signature may comprise: determining gene group scores using the RNA expression levels for at least three genes from each of at least two of the gene groups, the gene groups including: Coactivation molecules group: TNFRSF4, CD27, CD80, CD40LG, TNFRSF9, CD40, CD28, ICOSLG, CD83, TNFSF9, CD70, TNFSF4, ICOS, and CD86; MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; T cell traffic group: CXCL10, CX3CL1, CX3CR1, CXCL11, CXCR3, CCL5, CCL3, CXCL9, and CCL4; B cells group: CD22, TNFRSF13C, STAP1, CD79B, PAX5, CR2, TNFRSF13B, CD79A, TNFRSF17, FCRL5, MS4A1, CD19, and BLK; M1 signatures group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Treg traffic group: CCR10, CCL28, CCL17, CCR4, CCL22, CCR8, and CCL1; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; MDSC traffic group: CXCL8, CSF3R, CXCL12, CSF1R, CSF2RA, IL6R, CSF1, CCL26, CXCR2, IL6, CXCR4, CCL15, CXCL5, CSF2, and CSF3; Macrophages group: MRC1, SIGLEC1, MSR1, CD163, CSF1R, CD68, IL4I1, and IL10; Macrophage DC traffic group: CCL8, CSF1R, CSF1, CCR2, XCL1, XCR1, CCL2, and CCL7; Th2 signature group: IL13, CCR4, IL10, IL4, and IL5; Protumor cytokines group: TGFB2, MIF, IL6, TGFB3, TGFB1, IL22, and IL10; Cancer-associated fibroblasts (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Matrix remodeling group: ADAMTS4, ADAMTS5, CA9, LOX, MMP1, MMP11, MMP12, MMP2, MMP3, MMP7, MMP9, and PLOD2; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; and EMT signature group: CDH2, ZEB1, ZEB2, TWIST1, SNAI1, SNAI2, and TWIST2.

Aspects of determining the gene group scores are described herein, including with reference to FIG. 5 and in the Section titled "Gene Expression Signatures".

As described above, at act 310, the H2EBC TME signature is generated. In some examples, the H2EBC TME signature consists of only gene group scores for one or more (e.g., all) of the gene groups listed in Table 3. In some examples, the H2EBC TME signature comprises gene group scores for at least 2, 3, 4, 5, 6, 7, 8, 9, 10, 11, 12, 13, 14, 15, 16, 17, 18, 19, 20, 21, 22, 23, 24, 25, 26, 27, 28, or 29 gene groups listed in Table 3. In some examples, each gene group score for a particular gene group is determined using RNA expression levels of some or all (e.g., at least 2, 3, 4, 5, 6, 7, 8, 9, 10, 11, 12, 13, 14, 15, 16, 17, 18, 19, 20, etc.) of the genes of each gene group listed in Table 3. In other examples, the H2EBC TME signature includes one or more other gene group scores in addition to the gene group scores listed in Table 3.

Next, process 300 proceeds to act 314, where a H2EBC TME type is identified for the subject using the H2EBC TME signature generated at act 310. This may be done in any suitable way. For example, in some examples, the each of the possible H2EBC TME types is associated with a respective plurality of H2EBC TME signature clusters. In such examples, a H2EBC TME type for the subject may be identified by associating the H2EBC TME signature of the subject with a particular one of the plurality of H2EBC TME signature clusters; and identifying the H2EBC TME type for the subject as the H2EBC TME type corresponding to the particular one of the plurality of H2EBC TME signature clusters to which the H2EBC TME signature of the subject is associated. Examples of H2EBC TME types are described herein. Aspects of identifying a H2EBC TME type for a subject are described herein including in the section below titled "Generating TME Signature and Identifying TME Type".

In some examples, act 314 further comprising using one or more PROGENy signatures in addition to the H2EBC TME signature to identify the H2EBC TME type. In some examples, the one or more PROGENy signatures comprises an Androgen PROGENy signature, Estrogen PROGENy signature, and/or EGFR PROGENy signature.

As described above, a subject's H2EBC TME type is identified at act 314. In some examples, the H2EBC TME type of a subject is identified to be one of the following H2EBC TME types: Immune Desert (D) type, Fibrotic (F) type, Immune-Enriched, Non-fibrotic (IE) type, Moderately Immune-Enriched (IE-med) type, and Endothelium enriched (End-Ar-H) type.

Next, process 300 proceeds to act 318, where the subject's likelihood of responding to a therapy is identified using the H2EBC TME type identified at act 314. In some examples, when a subject is identified as having a H2EBC TME type IE or H2EBC TME type IE-med at act 314, the subject is identified as having an increased likelihood of responding to an immunotherapy (e.g., a PD1 antibody, such as pembrolizumab) relative to a subject having other H2EBC TME types, at act 318. In some examples, when a subject is identified as having a H2EBC TME type F at act 314, the subject is identified as having an increased likelihood of responding to an anti-VEGF therapy relative to a subject having other H2EBC TME types, at act 318. Aspects of identifying whether or not a subject is likely to respond to a therapy are described herein including in the section below titled "Therapeutic Indications."

In some examples, process 300 completes after act 318 completes. In some such examples, the determined H2EBC TME signature and/or identified H2EBC TME type, and/or the identified likelihood the subject will respond to a therapy may be stored for subsequent use, provided to one or more recipients (e.g., a clinician, a researcher, etc.), and/or used to update the H2EBC TME signature clusters (as described hereinbelow).

However, in some examples, one or more other acts are performed after act 314. For example, in the illustrated embodiment of FIG. 3, process 300 may include one or more of optional acts 316, and 320 shown using dashed lines in FIG. 3. For example, at act 316, a prognosis may be identified for the subject. In another example, when a subject is identified as having H2EBC TME type IE or H2EBC TME type IE-med at act 314, and/or identified as having an increased likelihood of responding to immunotherapy at act 318, the subject is administered one or more immunotherapies at act 320. Examples of immunotherapies are provided herein.

It should be appreciated that although acts 312, 316, and 320 are indicated as optional in the example of FIG. 3, in other embodiments, one or more other acts may be optional (in addition to or instead of acts 312, 316, and 320). For example, acts 302 and 304 may be optional (e.g., when the sequencing data is obtained and processed to obtain RNA expression data previously, process 300 may begin at act 306 by accessing the previously obtained RNA expression data). In some examples, the process 300 may comprise acts 302, 304, 306, 318 and 320, without acts 314 and 316. In some examples, the process 300 may comprise acts 302, 304, 306, 316, 314, 318, and 320 without act 316.

**Table 3. *Gene expression signatures used to generate H2EBC TME Signatures***

| | |
|---|---|
| Coactivation molecules | TNFRSF4, CD27, CD80, CD40LG, TNFRSF9, CD40, CD28, ICOSLG, CD83, TNFSF9, CD70, TNFSF4, ICOS, CD86 |
| MHC I | HLA-C, TAPBP, HLA-B, B2M, TAP2, HLA-A, TAP1, NLRC5 |
| MHC II | HLA-DQB1, HLA-DMA, HLA-DMB, HLA-DRA, CIITA, HLA-DQA1, HLA-DPB1, HLA-DRB1, HLA-DPA1 |
| Effector cells | ZAP70, GZMB, GZMK, IFNG, FASLG, EOMES, TBX21, GZMA, CD8A, GNLY, PRF1, CD8B |
| NK cells | NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160 |
| T cells | TRBC2, CD3E, CD3G, ITK, CD28, TRBC1, TRAT1, TBX21, CD5, TRAC, CD3D |
| T cell traffic | CL10, CX3CL1, CX3CR1, CXCL11, CXCR3, CCL5, CCL3, CXCL9, CCL4 |
| B cells | CD22, TNFRSF13C, STAP1, CD79B, PAX5, CR2, TNFRSF13B, CD79A, TNFRSF17, FCRL5, MS4A1, CD19, BLK |
| M1 signatures | CMKLR1, SOCS3, IRF5, NOS2, IL1B, IL12B, IL23A, TNF, IL12A |
| Th1 signature | IL21, TBX21, IL12RB2, CD40LG, IFNG, IL2, STAT4 |
| Antitumor cytokines | CCL3, IL21, IFNB1, IFNA2, TNF, TNFSF10 |
| Checkpoint inhibition | PDCD1, BTLA, HAVCR2, CD274, VSIR, LAG3, TIGIT, PDCD1LG2, CTLA4 |
| Treg | IKZF2, TNFRSF18, IL10, FOXP3, CCR8, IKZF4, CTLA4 |
| T reg traffic | CCR10, CCL28, CCL17, CCR4, CCL22, CCR8, CCL1 |
| Neutrophil signature | CD177, FFAR2, PGLYRP1, CXCR1, MPO, CXCR2, ELANE, CTSG, PRTN3, FCGR3B |
| Granulocyte traffic | CCR3, KITLG, CCL11, CXCL2, CXCR1, CXCR2, CXCL8, CXCL1, CXCL5 |
| MDSC | ARG1, IL6, CYBB, IL10, PTGS2, IDO1, IL4I1 |
| MDSC traffic | CXCL8, CSF3R, CXCL12, CSF1R, CSF2RA, IL6R, CSF1, CCL26, CXCR2, IL6, CXCR4, CCL15, CXCL5, CSF2, CSF3 |
| Macrophages | MRC1, SIGLEC1, MSR1, CD163, CSF1R, CD68, IL4I1, IL10 |
| Macrophage DC traffic | CCL8, CSF1R, CSF1, CCR2, XCL1, XCR1, CCL2, CCL7 |
| Th2 signature | IL13, CCR4, IL10, IL4, IL5 |
| Protumor cytokines | TGFB2, MIF, IL6, TGFB3, TGFB1, IL22, IL10 |
| CAF | COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, LRP1 |
| Matrix | LAMC2, TNC, COL11A1, VTN, LAMB3, COL1A1, FN1, LAMA3, LGALS9, COL1A2, COL4A1, COL5A1, ELN, LGALS7, COL3A1 |
| Matrix remodeling | ADAMTS4, ADAMTS5, CA9, LOX, MMP1, MMP11, MMP12, MMP2, MMP3, MMP7, MMP9, PLOD2 |
| Angiogenesis | VEGFC, VEGFA, PDGFC, KDR, CDH5, VEGFB, PGF, TEK, ANGPT2, CXCR2, FLT1, CXCL8, VWF, ANGPT1, CXCL5 |
| Endothelium | KDR, CDH5, NOS3, VCAM1, VWF, FLT1, MMRN1, CLEC14A, ENG, MMRN2 |

(continued)

| | |
|---|---|
| Coactivation molecules | *TNFRSF4, CD27, CD80, CD40LG, TNFRSF9, CD40, CD28, ICOSLG, CD83, TNFSF9, CD70, TNFSF4, ICOS, CD86* |
| Proliferation rate | *CCND1, CCNB1, CETN3, CDK2, E2F1, AURKA, BUB1, AURKB, PLK1, MCM6, ESCO2, MYBL2, MKI67, MCM2, CCNE1* |
| EMT signature | *CDH2, ZEB1, ZEB2, TWIST1,* SNAI1, *SNAI2, TWIST2* |

*Biological Samples*

Aspects of the disclosure relate to methods for determining a breast cancer TME type of a subject by obtaining sequencing data from a biological sample that has been obtained from the subject.

The biological sample may be any type of sample comprising tumor tissue, including, for example, a sample of one or more cells, one or more pieces of tissue(s) or organ(s). In some embodiments, the biological sample comprises breast tissue sample of the subject. In some embodiments, a breast tissue sample comprises one or more cell types derived from a breast (e.g., epithelial cells, secretory luminal cells, basal/myoepithelial cells, etc.). In some embodiments, a breast tissue sample comprises tumor cells.

In some embodiments, a tissue sample may have been previously obtained from a subject using a surgical procedure (*e.g.*, laparoscopic surgery, microscopically controlled surgery, or endoscopy), bone marrow biopsy, punch biopsy, endoscopic biopsy, or needle biopsy (e.g., a fine-needle aspiration, core needle biopsy, vacuum-assisted biopsy, or image-guided biopsy).

A sample of lymph node or blood, in some embodiments, refers to a sample comprising cells, e.g., cells from a blood sample or lymph node sample. In some embodiments, the sample comprises non-cancerous cells. In some embodiments, the sample comprises pre-cancerous cells. In some embodiments, the sample comprises cancerous cells. In some embodiments, the sample comprises blood cells. In some embodiments, the sample comprises lymph node cells. In some embodiments, the sample comprises lymph node cells and blood cells.

A sample of blood may be a sample of whole blood or a sample of fractionated blood. In some embodiments, the sample of blood comprises whole blood. In some embodiments, the sample of blood comprises fractionated blood. In some embodiments, the sample of blood comprises buffy coat. In some embodiments, the sample of blood comprises serum. In some embodiments, the sample of blood comprises plasma. In some embodiments, the sample of blood comprises a blood clot.

In some embodiments, a sample of blood is collected to obtain the cell-free nucleic acid (e.g., cell-free DNA) in the blood.

In some embodiments, the sample may be from a cancerous tissue or an organ or a tissue or organ suspected of having one or more cancerous cells. In some embodiments, a sample from a subject (*e.g.,* a biopsy from a subject) may include both healthy and cancerous cells and/or tissue. In certain embodiments, one sample will be taken from a subject for analysis. In some embodiments, more than one (*e.g.,* 2, 3, 4, 5, 6, 7, 8, 9, 10, 11, 12, 13, 14, 15, 16, 17, 18, 19, 20, or more) samples may be taken from a subject for analysis. In some embodiments, one sample from a subject will be analyzed. In certain embodiments, more than one (*e.g.,* 2, 3, 4, 5, 6, 7, 8, 9, 10, 11, 12, 13, 14, 15, 16, 17, 18, 19, 20, or more) samples may be analyzed. If more than one sample from a subject is analyzed, the samples may be procured at the same time (*e.g.,* more than one sample may be taken in the same procedure), or the samples may be taken at different times (*e.g.,* during a different procedure including a procedure 1, 2, 3, 4, 5, 6, 7, 8, 9, 10 days; 1, 2, 3, 4, 5, 6, 7, 8, 9, 10 weeks; 1, 2, 3, 4, 5, 6, 7, 8, 9, 10 months, 1, 2, 3, 4, 5, 6, 7, 8, 9, 10 years, or 1, 2, 3, 4, 5, 6, 7, 8, 9, 10 decades after a first procedure). A second or subsequent sample may be taken or obtained from the same region (*e.g.,* from the same tumor or area of tissue) or a different region (including, *e.g.,* a different tumor). A second or subsequent sample may be taken or obtained from the subject after one or more treatments, and may be taken from the same region or a different region. As a non-limiting example, the second or subsequent sample may be useful in determining whether the cancer in each sample has different characteristics (*e.g.,* in the case of samples taken from two physically separate tumors in a patient) or whether the cancer has responded to one or more treatments (*e.g.,* in the case of two or more samples from the same tumor prior to and subsequent to a treatment).

Any of the biological samples described herein may be obtained from the subject using any known technique. See, for example, the following publications on collecting, processing, and storing biological samples: Biospecimens and biorepositories: from afterthought to science by Vaught et al. (Cancer Epidemiol Biomarkers Prev. 2012 Feb;21(2):253-5), and Biological sample collection, processing, storage and information management by Vaught and Henderson (IARC Sci Publ. 2011;(163):23-42).

Any of the biological samples from a subject described herein may be stored using any method that preserves stability of the biological sample. In some embodiments, preserving the stability of the biological sample means inhibiting components (e.g., DNA, RNA, protein, or tissue structure or morphology) of the biological sample from degrading until they are measured so that when measured, the measurements represent the state of the sample at the time of obtaining it from the subject. In some embodiments, a biological sample is stored in a composition that is able to penetrate the same and protect components (e.g., DNA, RNA, protein, or tissue structure or morphology) of the biological sample from degrading. As used herein, degradation is the transformation of a component from one form to another form such that the first form is no longer detected at the same level as before degradation.

In some embodiments, the biological sample is stored using cryopreservation. Non-limiting examples of cryopreservation include, but are not limited to, step-down freezing, blast freezing, direct plunge freezing, snap freezing, slow freezing using a programmable freezer, and vitrification. In some embodiments, the biological sample is stored using lyophilization. In some embodiments, a biological sample is placed into a container that already contains a preservant (e.g., RNALater to preserve RNA) and then frozen (e.g., by snap-freezing), after the collection of the biological sample from the subject. In some embodiments, such storage in frozen state is done immediately after collection of the biological sample. In some embodiments, a biological sample may be kept at either room temperature or 4°C for some time (e.g., up to an hour, up to 8 h, or up to 1 day, or a few days) in a preservant or in a buffer without a preservant, before being frozen.

Non-limiting examples of preservants include formalin solutions, formaldehyde solutions, RNALater or other equivalent solutions, TriZol or other equivalent solutions, DNA/RNA Shield or equivalent solutions, EDTA (e.g., Buffer AE (10 mM Tris·Cl; 0.5 mM EDTA, pH 9.0)) and other coagulants, and Acids Citrate Dextrose (e.g., for blood specimens).

In some embodiments, special containers may be used for collecting and/or storing a biological sample. For example, a vacutainer may be used to store blood. In some embodiments, a vacutainer may comprise a preservant (e.g., a coagulant, or an anticoagulant). In some embodiments, a container in which a biological sample is preserved may be contained in a secondary container, for the purpose of better preservation, or for the purpose of avoid contamination.

Any of the biological samples from a subject described herein may be stored under any condition that preserves stability of the biological sample. In some embodiments, the biological sample is stored at a temperature that preserves stability of the biological sample. In some embodiments, the sample is stored at room temperature (e.g., 25 °C). In some embodiments, the sample is stored under refrigeration *(e.g.,* 4 °C). In some embodiments, the sample is stored under freezing conditions (e.g., -20 °C). In some embodiments, the sample is stored under ultralow temperature conditions (e.g., -50 °C to -800 °C). In some embodiments, the sample is stored under liquid nitrogen (e.g., -1700 °C). In some embodiments, a biological sample is stored at -60°C to -8-°C (e.g., -70°C) for up to 5 years (e.g., up to 1 month, up to 2 months, up to 3 months, up to 4 months, up to 5 months, up to 6 months, up to 7 months, up to 8 months, up to 9 months, up to 10 months, up to 11 months, up to 1 year, up to 2 years, up to 3 years, up to 4 years, or up to 5 years). In some embodiments, a biological sample is stored as described by any of the methods described herein for up to 20 years (e.g., up to 5 years, up to 10 years, up to 15 years, or up to 20 years).

*Obtaining RNA Expression Data*

Aspects of the disclosure relate to methods of determining a breast cancer TME type of a subject using sequencing data or RNA expression data obtained from a biological sample from the subject.

The RNA expression data used in methods described herein typically is derived from sequencing data obtained from the biological sample.

The sequencing data may be obtained from the biological sample using any suitable sequencing technique and/or apparatus. In some embodiments, the sequencing apparatus used to sequence the biological sample may be selected from any suitable sequencing apparatus known in the art including, but not limited to, Illumina™, SOLid™, Ion Torrent™, PacBio™, a nanopore-based sequencing apparatus, a Sanger sequencing apparatus, or a 454™ sequencing apparatus. In some embodiments, sequencing apparatus used to sequence the biological sample is an Illumina sequencing (e.g., NovaSeq™, NextSeq™, HiSeq™, MiSeq™, or MiniSeq™) apparatus.

After the sequencing data is obtained, it is processed in order to obtain the RNA expression data. RNA expression data may be acquired using any method known in the art including, but not limited to whole transcriptome sequencing, whole exome sequencing, total RNA sequencing, mRNA sequencing, targeted RNA sequencing, RNA exome capture sequencing, next generation sequencing, and/or deep RNA sequencing. In some embodiments, RNA expression data may be obtained using a microarray assay.

In some embodiments, the sequencing data is processed to produce RNA expression data. In some embodiments, RNA sequence data is processed by one or more bioinformatics methods or software tools, for example RNA sequence quantification tools (e.g., Kallisto) and genome annotation tools (e.g., Gencode v23), in order to produce expression data. The Kallisto software is described in Nicolas L Bray, Harold Pimentel, Páll Melsted and Lior Pachter, Near-optimal probabilistic RNA-seq quantification, Nature Biotechnology 34, 525-527 (2016), doi:10.1038/nbt.3519.

In some embodiments, microarray expression data is processed using a bioinformatics R package, such as "affy" or "limma," in order to produce expression data. The "affy" software is described in Bioinformatics. 2004 Feb 12;20(3):307-15. doi: 10.1093/bioinformatics/btg405. "affy--analysis of Affymetrix GeneChip data at the probe level" by Laurent Gautier 1, Leslie Cope, Benjamin M Bolstad, Rafael A Irizarry PMID: 14960456 DOI: 10.1093/bioinformatics/btg405. The "limma" software is described in Ritchie ME, Phipson B, Wu D, Hu Y, Law CW, Shi W, Smyth GK "limma powers differential expression analyses for RNA-sequencing and microarray studies." Nucleic Acids Res. 2015 Apr 20;43(7):e47. 20. doi.org/10.1093/nar/gkv007PMID: 25605792, PMCID: PMC4402510.

In some embodiments, sequencing data and/or expression data comprises more than 5 kilobases (kb). In some embodiments, the size of the obtained RNA data is at least 10 kb. In some embodiments, the size of the obtained RNA sequencing data is at least 100 kb. In some embodiments, the size of the obtained RNA sequencing data is at least 500 kb. In some embodiments, the size of the obtained RNA sequencing data is at least 1 megabase (Mb). In some embodiments, the size of the obtained RNA sequencing data is at least 10 Mb. In some embodiments, the size of the obtained RNA sequencing data is at least 100 Mb. In some embodiments, the size of the obtained RNA sequencing data is at least 500 Mb. In some embodiments, the size of the obtained RNA sequencing data is at least 1 gigabase (Gb). In some embodiments, the size of the obtained RNA sequencing data is at least 10 Gb. In some embodiments, the size of the obtained RNA sequencing data is at least 100 Gb. In some embodiments, the size of the obtained RNA sequencing data is at least 500 Gb.

In some embodiments, the expression data is acquired through bulk RNA sequencing. Bulk RNA sequencing may include obtaining expression levels for each gene across RNA extracted from a large population of input cells (e.g., a mixture of different cell types.) In some embodiments, the expression data is acquired through single cell sequencing (e.g., scRNA-seq). Single cell sequencing may include sequencing individual cells.

In some embodiments, bulk sequencing data comprises at least 1 million reads, at least 5 million reads, at least 10 million reads, at least 20 million reads, at least 50 million reads, or at least 100 million reads. In some embodiments, bulk sequencing data comprises between 1 million reads and 5 million reads, 3 million reads and 10 million reads, 5 million reads and 20 million reads, 10 million reads and 50 million reads, 30 million reads and 100 million reads, or 1 million reads and 100 million reads (or any number of reads including, and between).

In some embodiments, the expression data comprises next-generation sequencing (NGS) data. In some embodiments, the expression data comprises microarray data.

Expression data (e.g., indicating expression levels) for a plurality of genes may be used for any of the methods or compositions described herein. The number of genes which may be examined may be up to and inclusive of all the genes of the subject. In some embodiments, expression levels may be determined for all of the genes of a subject. As a non-limiting example, four or more, five or more, six or more, seven or more, eight or more, nine or more, ten or more, eleven or more, twelve or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 35 or more, 40 or more, 50 or more, 60 or more, 70 or more, 80 or more, 90 or more, 100 or more, 125 or more, 150 or more, 175 or more, 200 or more, 225 or more, 250 or more, 275 or more, or 300 or more genes may be used for any evaluation described herein. As another set of non-limiting examples, the expression data may include, for each gene group listed in Table 1, Table 2, or Table 3, expression data for at least 5, at least 10, at least 15, at least 20, or at least 25 genes selected from each gene group.

In some embodiments, RNA expression data is obtained by accessing the RNA expression data from at least one computer storage medium on which the RNA expression data is stored. Additionally or alternatively, in some embodiments, RNA expression data may be received from one or more sources via a communication network of any suitable type. For example, in some embodiment, the RNA expression data may be received from a server (e.g., a SFTP server, or Illumina BaseSpace).

The RNA expression data obtained may be in any suitable format, as aspects of the technology described herein are not limited in this respect. For example, in some embodiments, the RNA expression data may be obtained in a text-based file (e.g., in a FASTQ, FASTA, BAM, or SAM format). In some embodiments, a file in which sequencing data is stored may contains quality scores of the sequencing data. In some embodiments, a file in which sequencing data is stored may contain sequence identifier information.

Expression data, in some embodiments, includes gene expression levels. Gene expression levels may be detected by detecting a product of gene expression such as mRNA and/or protein. In some embodiments, gene expression levels are determined by detecting a level of a mRNA in a sample. As used herein, the terms "determining" or "detecting" may include assessing the presence, absence, quantity and/or amount (which can be an effective amount) of a substance within a sample, including the derivation of qualitative or quantitative concentration levels of such substances, or otherwise evaluating the values and/or categorization of such substances in a sample from a subject.

FIG. 4 shows an exemplary process 104 for processing sequencing data to obtain RNA expression data from sequencing data. Process 104 is analogous to processes 204 and 304, shown in FIGs. 2 and 3, respectively. Process 104 may be performed by any suitable computing device or devices, as aspects of the technology described herein are not limited in this respect. For example, process 104 may be performed by a computing device part of a sequencing apparatus. In other embodiments, process 104 may be performed by one or more computing devices external to the sequencing apparatus.

Process 104 begins at act 400, where sequencing data is obtained from a biological sample obtained from a subject. The sequencing data is obtained by any suitable method, for example, using any of the methods described herein including in the Section titled "Biological Samples."

In some embodiments, the sequencing data obtained at act 400 comprises RNA-seq data. In some embodiments, the biological sample comprises blood or tissue. In some embodiments, the biological sample comprises one or more tumor cells, for example, one or more breast tumor cells.

Next, process 104 proceeds to act 402 where the sequencing data obtained at act 400 is normalized to transcripts per kilobase million (TPM) units. The normalization may be performed using any suitable software and in any suitable way. For example, in some embodiments, TPM normalization may be performed according to the techniques described in Wagner et al. (Theory Biosci. (2012) 131:281-285). In some embodiments, the TPM normalization may be performed using a software package, such as, for example, the gcrma package. Aspects of the gcrma package are described in Wu J, Gentry RIwcfJMJ (2021). "gcrma: Background Adjustment Using Sequence Information. R package version 2.66.0.". In some embodiments, RNA expression level in TPM units for a particular gene may be calculated according to the following formula:

$$A \cdot \frac{1}{\sum(A)} \cdot 10^6$$

*Where*

$$A = \frac{total\ reads\ mapped\ to\ gene \cdot 10^3}{gene\ length\ in\ bp}$$

Next, process 104 proceeds to act 404, where the RNA expression levels in TPM units (as determined at act 402) may be log transformed. Process 104 is illustrative and there are variations. For example, in some embodiments, one or both of acts 402 and 404 may be omitted. Thus, in some embodiments, the RNA expression levels may not be normalized to transcripts per million units and may, instead, be converted to another type of unit (e.g., reads per kilobase million (RPKM) or fragments per kilobase million (FPKM) or any other suitable unit). Additionally or alternatively, in some embodiments, the log transformation may be omitted. Instead, no transformation may be applied in some embodiments, or one or more other transformations may be applied in lieu of the log transformation.

RNA expression data obtained by process 104 can include the sequence data generated by a sequencing protocol (e.g., the series of nucleotides in a nucleic acid molecule identified by next-generation sequencing, sanger sequencing, etc.) as well as information contained therein (e.g., information indicative of source, tissue type, etc.) which may also be considered information that can be inferred or determined from the sequence data. In some embodiments, expression data obtained by process 104 can include information included in a FASTA file, a description and/or quality scores included in a FASTQ file, an aligned position included in a BAM file, and/or any other suitable information obtained from any suitable file.

*Gene Expression Signatures*

Aspects of the disclosure relate to processing of expression data to determine one or more gene expression signatures (e.g., a breast cancer TME signature). Expression data (e.g., RNA expression data) is processed using a computing device to determine the one or more gene expression signatures. In some embodiments, the computing device may be operated by a user such as a doctor, clinician, researcher, patient, or other individual. For example, the user may provide the expression data as input to the computing device (e.g., by uploading a file), and/or may provide user input specifying processing or other methods to be performed using the expression data.

In some embodiments, expression data may be processed by one or more software programs running on computing device.

The claimed methods comprise an act of determining a BLBC TME signature comprising gene group scores for respective gene groups in a plurality of gene groups. In some embodiments, a BLBC TME signature comprises gene group scores for at least one (e.g., 1, 2, 3, 4, 5, 6, 7, 8, 9, 10, 11, 12, 13, 14, 15, 16, 17, 18, 19, 20, 21, 22, 23, 24, 25, 26, 27, or 28) of the gene groups listed in Table 1. In some embodiments not part of the claims, methods described herein comprise an act of determining a LNLBC TME signature comprising gene group scores for respective gene groups in a plurality of gene groups. In some embodiments not part of the claims, a LNLBC TME signature comprises gene group scores for at least one (e.g., 1, 2, 3, 4, 5, 6, 7, 8, 9, 10, 11, 12, 13, 14, 15, 16, 17, 18, 19, 20, 21, 22, 23, or 24) of the gene groups listed in Table 2. In some embodiments not part of the claims, methods described herein comprise an act of determining a H2EBC TME signature comprising gene group scores for respective gene groups in a plurality of gene groups. In some embodiments not part of the claims, a H2EBC TME signature comprises gene group scores for at least one (e.g., 1, 2, 3, 4, 5, 6, 7, 8, 9, 10, 11, 12, 13, 14, 15, 16, 17, 18, 19, 20, 21, 22, 23, 24, 25, 26, 27, 28, or 29) of the gene groups listed in Table 3.

The number of genes in a gene group used to determine a gene group score may vary. In some embodiments, all RNA expression levels for all genes in a particular gene group may be used to determine a gene group score for the particular gene group. In other embodiments, RNA expression data for fewer than all genes may be used (e.g., RNA expression levels for at least two genes, at least three genes, at least five genes, between 2 and 10 genes, between 5 and 15 genes, between 3 and 30 genes, or any other suitable range within these ranges).

In some embodiments, a TME signature comprises a gene group score for the MHC I group. In some embodiments, this gene group score may be calculated using RNA expression levels of at least three genes (e.g., at least 3, at least 4, at least 5, at least 6, or at least 7) in the MCH I group, which is defined by its constituent genes: *HLA-C, TAPBP, HLA-B, B2M, TAP2, HLA-A, TAP1,* and *NLRC5.*

In some embodiments, a TME signature comprises a gene group score for the Angiogenesis group. In some embodiments, this gene group score may be calculated using RNA expression levels of at least three (e.g., at least 3, at least 4, at least 5, at least 6, at least 7, at least 8, at least 9, at least 10, at least 11, at least 12, at least 13, or at least 14) genes in the Angiogenesis group, which is defined by its constituent genes: *VEGFC, VEGFA, PDGFC, KDR, CDH5, VEGFB, PGF, TEK, ANGPT2, CXCR2, FLT1, CXCL8, VWF, ANGPT1,* and *CXCL5.*

In some embodiments, a TME signature comprises a gene group score for the Antitumor cytokines group. In some embodiments, this gene group score may be calculated using RNA expression levels of at least three (e.g., at least 3, at least 4, or at least 5) genes in the Antitumor cytokines group, which is defined by its constituent genes: *CCL3, IL21, IFNB1, IFNA2, TNF,* and *TNFSF10.*

In some embodiments, a TME signature comprises a gene group score for the B cells group. In some embodiments, this gene group score may be calculated using RNA expression levels of at least three (e.g., at least 3, at least 4, at least 5, at least 6, at least 7, at least 8, at least 9, at least 10, at least 11, at least 12, or at least 13) genes in the B cells group, which is defined by its constituent genes: *CD22, TNFRSF13C, STAP1, CD79B, PAX5, CR2, TNFRSF13B, CD79A, TNFRSF17, FCRL5, MS4A1, CD19,* and *BLK.*

In some embodiments, a TME signature comprises a gene group score for the CAF group. In some embodiments, this gene group score may be calculated using RNA expression levels of at least three (e.g., at least 3, at least 4, at least 5, at least 6, at least 7, at least 8, at least 9, at least 10, at least 11, at least 12, at least 13, at least 14, at least 15, at least 16, at least 17, or at least 18) genes in the CAF group, which is defined by its constituent genes: *COL6A3, PDGFRB, COL6A1, MFAP5, COL5A1, FAP, PDGFRA, FGF2, ACTA2, COL6A2, FBLN1, CD248, COL1A1, MMP2, COLIA2, MMP3, LUM, CXCL12,* and *LRP1.*

In some embodiments, a TME signature comprises a gene group score for the checkpoint inhibition group. In some embodiments, this gene group score may be calculated using RNA expression levels of at least three (e.g., at least 3, at least 4, at least 5, at least 6, at least 7, at least 8, or at least 9) genes in the checkpoint inhibition group, which is defined by its constituent genes: *PDCD1, BTLA, HAVCR2, CD274, VSIR, LAG3, TIGIT, PDCD1LG2,* and *CTLA4.*

In some embodiments, a TME signature comprises a gene group score for the coactivation molecules group. In some embodiments, this gene group score may be calculated using RNA expression levels of at least three (e.g., at least 3, at least 4, at least 5, at least 6, at least 7, at least 8, at least 9, at least 10, at least 11, at least 12, or at least 13) genes in the coactivation molecules group, which is defined by its constituent genes: *TNFRSF4, CD27, CD80, CD40LG, TNFRSF9, CD40, CD28, ICOSLG, CD83, TNFSF9, CD70, TNFSF4, ICOS,* and *CD86.*

In some embodiments, a TME signature comprises a gene group score for the effector cells group. In some embodiments, this gene group score may be calculated using RNA expression levels of at least three (e.g., at least 3, at least 4, at least 5, at least 6, at least 7, at least 8, at least 9, at least 10, or at least 11) genes in the effector cells group, which is defined by its constituent genes: *ZAP70, GZMB, GZMK, IFNG, FASLG, EOMES, TBX21, GZMA, CD8A, GNLY, PRF1,* and *CD8B.*

In some embodiments, a TME signature comprises a gene group score for the EMT signature group. In some embodiments, this gene group score may be calculated using RNA expression levels of at least three (e.g., at least 3, at least 4, at least 5, or at least 6) genes in the EMT signature group, which is defined by its constituent genes: *CDH2, ZEB1, ZEB2, TWIST1, SNAI1, SNAI2,* and *TWIST2.*

In some embodiments, a TME signature comprises a gene group score for the endothelium group. In some embodiments, this gene group score may be calculated using RNA expression levels of at least three (e.g., at least 3, at least 4, at least 5, at least 6, at least 7, at least 8, or at least 9) genes in the endothelium group, which is defined by its constituent genes: *KDR, CDH5, NOS3, VCAM1, VWF, FLT1, MMRN1, CLEC14A, ENG,* and *MMRN2.*

In some embodiments, a TME signature comprises a gene group score for the follicular B helper T cells group. In some embodiments, this gene group score may be calculated using RNA expression levels of at least three (e.g., at least 3, at least 4, at least 5, at least 6, at least 7, at least 8, or at least 9) genes in the follicular B helper T cells group, which is defined by its constituent genes: *SH2D1A, IL6, MAF, CD40LG, IL21, ICOS, IL4, CD84, CXCR5,* and *BCL6.*

In some embodiments, a TME signature comprises a gene group score for the follicular dendritic cells group. In some embodiments, this gene group score may be calculated using RNA expression levels of at least three (e.g., at least 3, at least 4, at least 5, at least 6, at least 7, or at least 8) genes in the follicular dendritic cells group, which is defined by its constituent genes: *FDCSP, SERPINE2, PRNP, PDPN, LTBR, BST1, CLU, C1S, C4A.*

In some embodiments, a TME signature comprises a gene group score for the granulocyte traffic group. In some embodiments, this gene group score may be calculated using RNA expression levels of at least three (e.g., at least 3, at least 4, at least 5, at least 6, at least 7, or at least 8) genes in the granulocyte traffic group, which is defined by its constituent genes: *CCR3, KITLG, CCL11, CXCL2, CXCR1, CXCR2, CXCL8, CXCL1,* and *CXCL5.*

In some embodiments, a TME signature comprises a gene group score for the granulocytes group. In some embodiments, this gene group score may be calculated using RNA expression levels of at least three (e.g., at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, or at least 31) genes in the granulocytes group, which is defined by its constituent genes: *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* and *PRSS33.*

In some embodiments, a TME signature comprises a gene group score for the M1 signatures group. In some embodiments, this gene group score may be calculated using RNA expression levels of at least three (e.g., at least 3, at least 4, at least 5, at least 6, at least 7, or at least 8) genes in the M1 signatures group, which is defined by its constituent genes: *CMKLR1, SOCS3, IRF5, NOS2, IL1B, IL12B, IL23A, TNF,* and *IL12A.*

In some embodiments, a TME signature comprises a gene group score for the macrophages group. In some embodiments, this gene group score may be calculated using RNA expression levels of at least three (e.g., at least 3, at least 4, at least 5, at least 6, or at least 7) genes in the macrophages group, which is defined by its constituent genes: *MRC1, SIGLEC1, MSR1, CD163, CSF1R, CD68, IL4I1,* and *IL10.*

In some embodiments, a TME signature comprises a gene group score for the matrix group. In some embodiments, this gene group score may be calculated using RNA expression levels of at least three (e.g., at least 3, at least 4, at least 5, at least 6, at least 7, at least 8, at least 9, at least 10, at least 11, at least 12, at least 13, or at least 14) genes in the matrix group, which is defined by its constituent genes: *LAMC2, TNC, COL11A1, VTN, LAMB3, COL1A1, FN1, LAMA3, LGALS9, COL1A2, COL4A1, COL5A1, ELN, LGALS7,* and *COL3A1.*

In some embodiments, a TME signature comprises a gene group score for the MDSC group. In some embodiments, this gene group score may be calculated using RNA expression levels of at least three (e.g., at least 3, at least 4, at least 5, or at least 6) genes in the MDSC group, which is defined by its constituent genes: *ARG1, IL6, CYBB, IL10, PTGS2, IDO1,* and *IL4I1.*

In some embodiments, a TME signature comprises a gene group score for the MHC II group. In some embodiments, this gene group score may be calculated using RNA expression levels of at least three (e.g., at least 3, at least 4, at least 5, at least 6, at least 7, or at least 8) genes in the MHC II group, which is defined by its constituent genes: *HLA-DQB1, HLA-DMA, HLA-DMB, HLA-DRA, CIITA, HLA-DQA1, HLA-DPB1, HLA-DRB1,* and *HLA-DPA1.*

In some embodiments, a TME signature comprises a gene group score for the neutrophil signature group. In some embodiments, this gene group score may be calculated using RNA expression levels of at least three (e.g., at least 3, at least 4, at least 5, at least 6, at least 7, at least 8, or at least 9) genes in the neutrophil signature group, which is defined by its constituent genes: *CD177, FFAR2, PGLYRP1, CXCR1, MPO, CXCR2, ELANE, CTSG, PRTN3,* and *FCGR3B.*

In some embodiments, a TME signature comprises a gene group score for the NK cells group. In some embodiments, this gene group score may be calculated using RNA expression levels of at least three (e.g., at least 3, at least 4, at least 5, at least 6, at least 7, at least 8, at least 9, at least 10, at least 11, at least 12, at least 13, at least 14, at least 15, or at least 16) genes in the NK cells group, which is defined by its constituent genes: *NKG7, FGFBP2, CD244, KLRK1, KIR2DL4, CD226, KLRF1, GNLY, GZMB, KLRC2, NCR1, GZMH, IFNG, SH2D1B, NCR3, EOMES,* and *CD160.*

In some embodiments, a TME signature comprises a gene group score for the proliferation rate group. In some embodiments, this gene group score may be calculated using RNA expression levels of at least three (e.g., at least 3, at least 4, at least 5, at least 6, at least 7, at least 8, at least 9, at least 10, at least 11, at least 12, at least 13, or at least 14) genes in the proliferation rate group, which is defined by its constituent genes: *CCND1, CCNB1, CETN3, CDK2, E2F1, AURKA, BUB1, AURKB, PLK1, MCM6, ESCO2, MYBL2, MKI67, MCM2,* and *CCNE1.*

In some embodiments, a TME signature comprises a gene group score for the protumor cytokines group. In some embodiments, this gene group score may be calculated using RNA expression levels of at least three (e.g., at least 3, at least 4, at least 5, or at least 6) genes in the protumor cytokines group, which is defined by its constituent genes: *TGFB2, MIF, IL6, TGFB3, TGFB1, IL22,* and *IL10.*

In some embodiments, a TME signature comprises a gene group score for the T cells group. In some embodiments, this gene group score may be calculated using RNA expression levels of at least three (e.g., at least 3, at least 4, at least 5, at least 6, at least 7, at least 8, at least 9, or at least 10) genes in the T cells group, which is defined by its constituent genes: *TRBC2, CD3E, CD3G, ITK, CD28, TRBC1, TRAT1, TBX21, CD5, TRAC,* and *CD3D.*

In some embodiments, a TME signature comprises a gene group score for the Th1 signature group. In some embodiments, this gene group score may be calculated using RNA expression levels of at least three (e.g., at least 3, at least 4, at least 5, or at least 6) genes in the Th1 signature group, which is defined by its constituent genes: *IL21, TBX21, IL12RB2, CD40LG, IFNG, IL2,* and *STAT4.*

In some embodiments, a TME signature comprises a gene group score for the Th2 signature group. In some embodiments, this gene group score may be calculated using RNA expression levels of at least three (e.g., at least 3 or at least 4) genes in the Th2 signature group, which is defined by its constituent genes: *IL13, CCR4, IL10, IL4,* and *IL5.*

In some embodiments, a TME signature comprises a gene group score for the TLS group. In some embodiments, this gene group score may be calculated using RNA expression levels of at least three (e.g., at least 3, at least 4, at least 5, at least 6, at least 7, or at least 8) genes in the TLS group, which is defined by its constituent genes: *CXCL13, CCL19, LAMP3, SELL, CXCR4, CCL21, CCR7, CD86,* and *BCL6.*

In some examples, a TME signature comprises a gene group score for the T reg group. In some embodiments, this gene group score may be calculated using RNA expression levels of at least three (e.g., at least 3, at least 4, at least 5, or at least 6) genes in the T reg group, which is defined by its constituent genes: *IKZF2, TNFRSF18, IL10, FOXP3, CCR8, IKZF4,* and *CTLA4.*

In some examples, a TME signature comprises a gene group score for the Macrophage DC traffic group. In some embodiments, this gene group score may be calculated using RNA expression levels of at least three (e.g., at least 3, at least 4, at least 5, at least 6, or at least 7) genes in the macrophage DC traffic group, which is defined by its constituent genes: *CCL8, CSF1R, CSF1, CCR2, XCL1, XCR1, CCL2,* and *CCL7.*

In some examples, a TME signature comprises a gene group score for the matrix remodeling group. In some embodiments, this gene group score may be calculated using RNA expression levels of at least three (e.g., at least 3, at least 4, at least 5, at least 6, at least 7, at least 8, at least 9, or at least 10) genes in the matrix remodeling group, which is defined by its constituent genes: *ADAMTS4, ADAMTS5, CA9, LOX, MMP1, MMP11, MMP12, MMP2, MMP3, MMP7, MMP9,* and *PLOD2.*

In some examples, a TME signature comprises a gene group score for the T cell traffic group. In some embodiments, this gene group score may be calculated using RNA expression levels of at least three (e.g., at least 3, at least 4, at least 5, at least 6, at least 7, or at least 8) genes in the T cell traffic group, which is defined by its constituent genes: *CXCL10, CX3CL1, CX3CR1, CXCL11, CXCR3, CCL5, CCL3, CXCL9,* and *CCL4.*

In some examples, a TME signature comprises a gene group score for the MDSC traffic group. In some embodiments, this gene group score may be calculated using RNA expression levels of at least three (e.g., at least 3, at least 4, at least 5, or at least 6) genes in the MDSC group, which is defined by its constituent genes: *ARG1, IL6, CYBB, IL10, PTGS2, IDO1,* and *IL4I1.*

In some examples, a TME signature comprises a gene group score for the T reg traffic group. In some embodiments, this gene group score may be calculated using RNA expression levels of at least three (e.g., at least 3, at least 4, at least 5, or at least 6) genes in the T reg traffic group, which is defined by its constituent genes: *CCR10, CCL28, CCL17, CCR4, CCL22, CCR8,* and *CCL1.*

In some embodiments, determining a BLBC TME signature comprises determining a respective gene group score for each of at least two of the following gene groups, using, for a particular gene group, RNA expression levels for at least three genes in the particular gene group to determine the gene group score for the particular group, the gene groups including: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Coactivation molecules group: TNFRSF4, CD27, CD80, CD40LG, TNFRSF9, CD40, CD28, ICOSLG, CD83, TNFSF9, CD70, TNFSF4, ICOS, and CD86; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; B cells group: CD22, TNFRSF13C, STAP1, CD79B, PAX5, CR2, TNFRSF13B, CD79A, TNFRSF17, FCRL5, MS4A1, CD19, and BLK; M1 signature group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Th2 signature group: IL13, CCR4, IL10, IL4, and IL5; Protumor cytokines group: TGFB2, MIF, IL6, TGFB3, TGFB1, IL22, and IL10; Cancer-associated fibroblast (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; EMT signature group: CDH2, ZEB1, ZEB2, TWIST1, SNAI1, SNAI2, and TWIST2; Tertiary Lymphoid Structure (TLS) group: CXCL13, CCL19, LAMP3, SELL, CXCR4, CCL21, CCR7, CD86, and BCL6; Follicular dendritic cells group: FDCSP, SERPINE2, PRNP, PDPN, LTBR, BST1, CLU, C1S, and C4A; Follicular B helper T cells group: SH2D1A, IL6, MAF, CD40LG, IL21, ICOS, IL4, CD84, CXCR5, and BCL6; and Granulocytes group: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, and PRSS33.

In some embodiments, determining a LNLBC TME signature comprises determining a respective gene group score for each of at least two of the following gene groups, using, for a particular gene group, RNA expression levels for at least three genes in the particular gene group to determine the gene group score for the particular group, the gene groups including: MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; T cell traffic group: CXCL10 , CX3CL1, CX3CR1, CXCL11, CXCR3, CCL5, CCL3, CXCL9, and CCL4; B cells group: CD22, TNFRSF13C, STAP1, CD79B, PAX5, CR2, TNFRSF13B, CD79A, TNFRSF17, FCRL5, MS4A1, CD19, and BLK; M1 signatures group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; Macrophages group: MRC1, SIGLEC1, MSR1, CD163, CSF1R, CD68, IL4I1, and IL10; Macrophage DC traffic group: CCL8, CSF1R, CSF1, CCR2, XCL1, XCR1, CCL2, and CCL7; Protumor cytokines group: TGFB2, MIF, IL6, TGFB3, TGFB1, IL22, and IL10; Cancer-associated fibroblasts (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Matrix remodeling group: ADAMTS4, ADAMTS5, CA9, LOX, MMP1, MMP11, MMP12, MMP2, MMP3, MMP7, MMP9, and PLOD2; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; and EMT signature group: CDH2, ZEB1, ZEB2, TWIST1, SNAI1, SNAI2, and TWIST2

In some examples, determining a H2EBC TME signature comprises determining a respective gene group score for each of at least two of the following gene groups, using, for a particular gene group, RNA expression levels for at least three genes in the particular gene group to determine the gene group score for the particular group, the gene groups including: Coactivation molecules group: TNFRSF4, CD27, CD80, CD40LG, TNFRSF9, CD40, CD28, ICOSLG, CD83, TNFSF9, CD70, TNFSF4, ICOS, and CD86; MHC I group: HLA-C, TAPBP, HLA-B, B2M, TAP2, HLA-A, TAP1, and NLRC5; MHC II group: HLA-DQB1, HLA-DMA, HLA-DMB, HLA-DRA, CIITA, HLA-DQA1, HLA-DPB1, HLA-DRB1, and HLA-DPA1; Effector cells group: ZAP70, GZMB, GZMK, IFNG, FASLG, EOMES, TBX21, GZMA, CD8A, GNLY, PRF1, and CD8B; NK cells group: NKG7, FGFBP2, CD244, KLRK1, KIR2DL4, CD226, KLRF1, GNLY, GZMB, KLRC2, NCR1, GZMH, IFNG, SH2D1B, NCR3, EOMES, and CD160; T cells group: TRBC2, CD3E, CD3G, ITK, CD28, TRBC1, TRAT1, TBX21, CD5, TRAC, and CD3D; T cell traffic group: CXCL10, CX3CL1, CX3CR1, CXCL11, CXCR3, CCL5, CCL3, CXCL9, and CCL4; B cells group: CD22, TNFRSF13C, STAP1, CD79B, PAX5, CR2, TNFRSF13B, CD79A, TNFRSF17, FCRL5, MS4A1, CD19, and BLK; M1 signatures group: CMKLR1, SOCS3, IRF5, NOS2, IL1B, IL12B, IL23A, TNF, and IL12A; Th1 signature group: IL21, TBX21, IL12RB2, CD40LG, IFNG, IL2, and STAT4; Antitumor cytokines group: CCL3, IL21, IFNB1, IFNA2, TNF, and TNFSF10; Checkpoint inhibition group: PDCD1, BTLA, HAVCR2, CD274, VSIR, LAG3, TIGIT, PDCD1LG2, and CTLA4; Treg group: IKZF2, TNFRSF18, IL10, FOXP3, CCR8, IKZF4, and CTLA4; Treg traffic group: CCR10, CCL28, CCL17, CCR4, CCL22, CCR8, and CCL1; Neutrophil signature group: CD177, FFAR2, PGLYRP1, CXCR1, MPO, CXCR2, ELANE, CTSG, PRTN3, and FCGR3B; Granulocyte traffic group: CCR3, KITLG, CCL11, CXCL2, CXCR1, CXCR2, CXCL8, CXCL1, and CXCL5; MDSC group: ARG1, IL6, CYBB, IL10, PTGS2, IDO1, and IL4I1; MDSC traffic group: CXCL8, CSF3R, CXCL12, CSF1R, CSF2RA, IL6R, CSF1, CCL26, CXCR2, IL6, CXCR4, CCL15, CXCL5, CSF2, and CSF3; Macrophages group: MRC1, SIGLEC1, MSR1, CD163, CSF1R, CD68, IL4I1, and IL10; Macrophage DC traffic group: CCL8, CSF1R, CSF1, CCR2, XCL1, XCR1, CCL2, and CCL7; Th2 signature group: IL13, CCR4, IL10, IL4, and IL5; Protumor cytokines group: TGFB2, MIF, IL6, TGFB3, TGFB1, IL22, and IL10; Cancer-associated fibroblasts (CAF) group: COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, and LRP1; Matrix group: LAMC2, TNC, COL11A1, VTN, LAMB3, COL1A1, FN1, LAMA3, LGALS9, COL1A2, COL4A1, COL5A1, ELN, LGALS7, and COL3A1; Matrix remodeling group: ADAMTS4, ADAMTS5, CA9, LOX, MMP1, MMP11, MMP12, MMP2, MMP3, MMP7, MMP9, and PLOD2; Angiogenesis group: VEGFC, VEGFA, PDGFC, KDR, CDH5, VEGFB, PGF, TEK, ANGPT2, CXCR2, FLT1, CXCL8, VWF, ANGPT1, and CXCL5; Endothelium group: KDR, CDH5, NOS3, VCAM1, VWF, FLT1, MMRN1, CLEC14A, ENG, and MMRN2; Proliferation rate group: CCND1, CCNB1, CETN3, CDK2, E2F1, AURKA, BUB1, AURKB, PLK1, MCM6, ESCO2, MYBL2, MKI67, MCM2, and CCNE1; and EMT signature group: CDH2, ZEB1, ZEB2, TWIST1, SNAI1, SNAI2, and TWIST2.

Lists of gene groups are provided in Tables 1, 2, and 3 at the end of the specification.

As described above, aspects of the disclosure relate to determining a breast cancer TME signature for a subject. That signature may include gene group scores (e.g., gene group scores generated using RNA expression data for gene groups listed in Table 1, Table 2, and/or Table 3). Aspects of determining of TME signatures is described next with reference to FIG. 5.

A TME signature comprises gene group scores generated using a gene set enrichment analysis (GSEA) technique to determine a gene group score for one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, or 29) gene groups listed in Table 1. In some embodiments, a TME signature comprises gene group scores generated using a gene set enrichment analysis (GSEA) technique to determine a gene group score for eight or more (e.g., 8, 9, 10, 11, 12, 13, 14, 15, 16, 17, 18, 19, or 20) gene groups listed in Table 1. In some embodiments not part of the claims, a TME signature comprises gene group scores generated using a gene set enrichment analysis (GSEA) technique to determine a gene group score for eight or more (e.g., 8, 9, 10, 11, 12, 13, 14, 15, 16, 17, 18, 19, or 20) gene groups listed in Table 2. In some embodiments not part of the claims, a TME signature comprises gene group scores generated using a gene set enrichment analysis (GSEA) technique to determine a gene group score for eight or more (e.g., 8, 9, 10, 11, 12, 13, 14, 15, 16, 17, 18, 19, or 20) gene groups listed in Table 3.

In some embodiments, each gene group score is generated using a gene set enrichment analysis (GSEA) technique, using RNA expression levels of at least some genes in the gene group. In some embodiments, using a GSEA technique comprises using single-sample GSEA. Aspects of single sample GSEA (ssGSEA) are described in Barbie et al. Nature. 2009 Nov 5; 462(7269): 108-112. In some embodiments, ssGSEA is performed according to the following formula:

$$ssGSEA\ score\ =\ \frac{\sum\limits_{i}^{N} r_i^{1.25}}{\sum\limits_{i}^{N} r_i^{0.25}}\ -\ \frac{(M - N + 1)}{2}$$

where $r_i$ represents the rank of the ith gene in expression matrix, where $N$ represents the number of genes in the gene set (e.g., the number of genes in the first gene group when ssGSEA is being used to determine a gene group score for the first gene group using expression levels of the genes in the first gene group), and where M represents total number of genes in expression matrix. Additional, suitable techniques of performing GSEA are known in the art and are contemplated for use in the methods described herein without limitation. In some embodiments, a TME signature is calculated by performing ssGSEA on expression data from a plurality of subjects, for example expression data from one or more cohorts of subjects, such as TCGA, Metabric, FUSCCTNBC, GSE103091, GSE106977, GSE21653, GSE25066, GSE41998, GSE47994, GSE81538, GSE96058, etc., in order to produce a plurality of enrichment scores.

FIG. 5 depicts an illustrative example of how gene group scores may be determined as part of act 108 of process 100, act 208 of process 200, and act 308 of process 300. As shown in the example of FIG. 5, a "TME signature" comprises multiple gene group scores 520 determined for respective multiple gene groups. Each gene group score, for a particular gene group, is computed by performing GSEA 510 (e.g., using ssGSEA) on RNA expression data for one or more (e.g., at least two, at least three, at least four, at least five, at least six, etc., or all) genes in the particular gene group 500.

For example, as shown in FIG. 5, a gene group score (labelled "Gene Group Score 1") for gene group 1 (e.g., the T reg group) is computed from RNA expression data for one or more genes in gene group 1. As another example, a gene group score (labelled "Gene Group Score 2") for gene group 2 (e.g., the T cells group) is computed from RNA expression data for one or more genes in gene group 2. As another example, a gene group score (labelled "Gene Group Score 3") for gene group 3 (e.g., the NK cells group) is computed from RNA expression data for one or more genes in gene group 3. As another example, a gene group score (labelled "Gene Group Score 4") for gene group 4 (e.g., the B cells group) is computed from RNA expression data for one or more genes in gene group 4. As another example, a gene group score (labelled "Gene Group Score 5") for gene group 5 (e.g., the MDSC group) is computed from RNA expression data for one or more genes in gene group 5. As another example, a gene group score (labelled "Gene Group Score 6") for gene group 6 (e.g., the CAF group) is computed from RNA expression data for one or more genes in gene group 6. As another example, a gene group score (labelled "Gene Group Score 7") for gene group 7 (e.g., the Proliferation rate group) is computed from RNA expression data for one or more genes in gene group 7. As another example, a gene group score (labelled "Gene Group Score 8") for gene group 8 (e.g., the coactivation molecules group) is computed from RNA expression data for one or more genes in gene group 8.

Although the example of FIG. 5 shows that the gene expression group score includes eight gene group scores for a respective set of eight gene groups, it should be appreciated that in other embodiments, the first gene expression signature may include scores for any suitable number of groups (e.g., not just 8; the number of groups could be fewer or greater than 8). As indicated by the vertical ellipsis in FIG. 5, determining gene group scores of a TME signature may comprise determining gene group scores for 9, 10, 11, 12, 13, 14, 15, 16, 17, 18, 19, 20, or more gene groups using RNA expression data from one or more respective genes in each respective gene group, as aspects of the technology described herein are not limited in this respect. In another example, a TME signature may include scores for only a subset of the gene groups listed in Table 1. As another example, the gene expression group score may include one or more scores for one or more gene groups other than those gene groups listed in Table 1 (e.g. in addition to the score(s) for the groups in Table 1 o, for example the gene groups listed in Table 2 or Table 3).

In some embodiments, RNA expression levels for a particular gene group may be embodied in at least one data structure having fields storing the expression levels. The data structure or data structures may be provided as input to software comprising code that implements a GSEA technique (e.g., the ssGSEA technique) and processes the expression levels in the at least one data structure to compute a score for the particular gene group.

The number of genes in a gene group used to determine a gene group score may vary. In some embodiments, all RNA expression levels for all genes in a particular gene group may be used to determine a gene group score for the particular gene group. In other embodiments, RNA expression data for fewer than all genes may be used (e.g., RNA expression levels for at least two genes, at least three genes, at least five genes, between 2 and 10 genes, between 5 and 15 genes, or any other suitable range within these ranges).

In some embodiments, RNA expression levels for a particular gene group may be embodied in at least one data structure having fields storing the expression levels. The data structure or data structures may be provided as input to software comprising code that is configured to perform suitable scaling (e.g., median scaling) to produce a score for the particular gene group.

In some embodiments, ssGSEA is performed on expression data comprising three or more (e.g., 3, 4, 5, 6, 7, 8, 9, 10, 11, 12, 13, 14, 15, 16, 17, 18, 19, or 20) gene groups set forth in Table 1, Table 2, or Table 3. In some embodiments, each of the gene groups separately comprises one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or more) genes listed in Table 1, Table 2, or Table 3. In some embodiments, a TME signature is produced by performing ssGSEA on all of the gene groups in Table 1, each gene group including all listed genes in Table 1. In some embodiments, a TME signature is produced by performing ssGSEA on all of the gene groups in Table 2, each gene group including all listed genes in Table 2. In some embodiments, a TME signature is produced by performing ssGSEA on all of the gene groups in Table 3, each gene group including all listed genes in Table 3.

In some embodiments, one or more (e.g., a plurality) of enrichment scores are normalized in order to produce a TME signature for the expression data (e.g., expression data of the subject or of a cohort of subjects). In some embodiments, the enrichment scores are normalized by median scaling. In some embodiments, the enrichment scores are normalized by rank estimation and median scaling. In some embodiments, median scaling comprises clipping the range of enrichment scores, for example clipping to about -1.0 to about +1.0, -2.0 to about +3.0, -3.0 to about +3.0, -4.0 to +4.0, -5.0 to about +5.0. In some embodiments, median scaling produces a TME signature of the subject.

In some embodiments, a TME signature of a subject processed using a clustering algorithm to identify a tumor microenvironment type (e.g. a BLBC TME type, LNLBC TME type, H2EBC TME type). In some embodiments, the clustering comprises unsupervised clustering. In some embodiments, the unsupervised clustering comprises a dense clustering approach. In some embodiments, the unsupervised clustering comprises a hierarchical clustering approach. In some embodiments, clustering comprises calculating intersample similarity (e.g., using a Pearson correlation coefficient that, for example, may take on values in the range of [-1,1]), converting the distance matrix into a graph where each sample forms a node and two nodes form an edge with a weight equal to their Pearson correlation coefficient, removing edges with weight lower than a specified threshold, and applying a Louvain community detection algorithm to calculate graph partitioning into clusters. In some embodiments, the optimum weight threshold for observed clusters was calculated by employing minimum DaviesBouldin, maximum Calinski-Harabasz, and Silhouette techniques. In some embodiments, separations with low-populated clusters (< 5% of samples) are excluded.

In some embodiments, a TME signature of a subject is compared to pre-existing clusters of TME types and assigned a TME type based on that comparison.

Some aspects of determining gene group scores for gene groups are also described in U.S. Patent Publication No. 2020-0273543, entitled "SYSTEMS AND METHODS FOR GENERATING, VISUALIZING AND CLASSIFYING MOLECULAR FUNCTIONAL PROFILES".

*Generating TME Signature and Identifying TME Type*

As described herein, FIGs. 1-3 illustrate the determination of a subject's breast cancer TME signature, identification of the subject's TME type using the TME signature, and identification of whether the subject is likely to respond to a therapy based on the identified TME type.

As described herein, in some embodiments, one of a plurality of different breast cancer TME types may be identified for the subject using the TME signature determined for the subject using the techniques described herein. In some embodiments, the plurality of BLBC TME types comprises an Immune Enriched (IE) type, TLS (TLS) type (also referred to as B-cell enriched), Desert (D) type, Fibrotic (F) type, and Granulocyte enriched (G) type, as described herein and further below. In some embodiments not part of the claims, the plurality of LNLBC TME types comprises an Immune Desert (D) type, Fibrotic (F) type, Immune-Enriched Non-fibrotic (IE) type, Immune-Enriched Fibrotic (IE/F) type, and Angiogenic (E) type, as described herein and further below. In some embodiments not part of the claims, the plurality of H2EBC TME types comprises an Immune Desert (D) type, Fibrotic (F) type, Immune-Enriched, Non-fibrotic (IE) type, Moderately Immune-Enriched (IE-med) type, and Endothelium enriched (End-Ar-H) type, as described herein and further below.

In some embodiments, each of the plurality of TME types is associated with a respective TME signature cluster in a plurality of TME signature clusters. The TME type for a subject may be determined by: (1) associating the TME signature of the subject with a particular one of the plurality of TME signature clusters; and (2) identifying the TME type for the subject as the TME type corresponding to the particular one of the plurality of TME signature clusters to which the TME signature of the subject is associated.

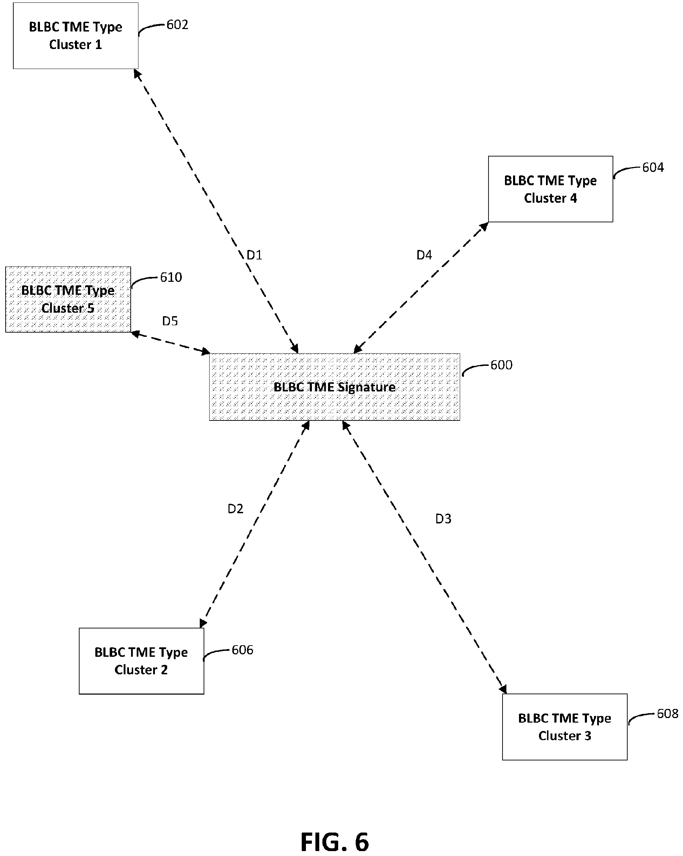

FIG. 6 shows an illustrative BLBC TME signature 600. In some embodiments not part of the claims, the signature 600 is a LNLBC TME signature or a H2EBC TME signature. In some embodiments, the TME signature (BLBC TME signature) comprises at least eight gene group scores for gene groups listed in Table 1. In other embodiments not part of the claims, the TME signature comprises at least eight gene group scores for gene groups listed in Table 2, or Table 3. However, it should be appreciated, that a TME signature may include fewer scores than the number of scores shown in FIG. 5 (e.g., by omitting scores for one or more of the gene groups listed in Table 1, Table 2, or Table 3) or more scores than the number of scores shown in FIG. 5 (e.g., by including scores for one or more other gene groups in addition to or instead of the gene groups listed in Table 1, Table 2, or Table 3). In some embodiments, a TME signature may be embodied in at least one data structure comprising fields storing the gene group scores part of the TME signature.

In some embodiments, the TME signature clusters may be generated by: (1) obtaining TME signatures (using the techniques described herein) for a plurality of subjects; and (2) clustering the TME signatures so obtained into the plurality of clusters. Any suitable clustering technique may be used for this purpose including, but not limited to, a dense clustering algorithm, spectral clustering algorithm, k-means clustering algorithm, hierarchical clustering algorithm, and/or an agglomerative clustering algorithm.

For example, intersample similarity may be calculated using a Pearson correlation. A distance matrix may be converted into a graph where each sample forms a node and two nodes form an edge with a weight equal to their Pearson correlation coefficient. Edges with weight lower than a specified threshold may be removed. A Louvain community detection algorithm may be applied to calculate graph partitioning into clusters. To mathematically determine the optimum weight threshold for observed clusters minimum DaviesBouldin, maximum Calinski-Harabasz, and Silhouette techniques may be employed. Separations with low-populated clusters (< 5% of samples) may be excluded.

Accordingly, in some embodiments, generating the TME signature clusters involves: (A) obtaining multiple sets of RNA expression data obtained by sequencing biological samples from multiple respective subjects, each of the multiple sets of RNA expression data indicating RNA expression levels for genes in a first plurality of gene groups (including one or more of the gene groups in Table 1); (B) generating multiple TME signatures from the multiple sets of RNA expression data, each of the multiple TME signatures comprising gene group scores for respective gene groups, the generating comprising, for each particular one of the multiple TME signatures: (i) determining the TME signature by determining the gene group scores using the RNA expression levels in the particular set of RNA expression data for which the particular one TME signature is being generated, and (ii) clustering the multiple signatures to obtain the plurality of TME signature clusters.

The resulting TME signature clusters may each contain any suitable number of TME signatures (e.g., at least 10, at least 100, at least 500, at least 500, at least 1000, at least 5000, between 100 and 10,000, between 500 and 20,000, or any other suitable range within these ranges), as aspects of the technology described herein are not limited in this respect. The number of TME signature clusters in this example is five. And although, in some embodiments, it may be possible that the number of clusters is different, it should be appreciated that an important aspect of the present disclosure is the inventors' discovery that certain intrinsic types of breast cancer (e.g., BLBC, LNLBC, H2EBC) may be characterized into five types based upon the generation of TME signatures using methods described herein.

For example, as shown in FIG. 6, a subject's BLBC TME signature 600 may be associated with one of five BLBC TME clusters: 602, 604, 606, 608, and 610. Each of the clusters 602, 604, 606, 608, and 610 may be associated with respective BLBC TME type. In this example, the BLBC TME signature 600 is compared to each cluster (e.g., using a distance-based comparison or any other suitable metric) and, based on the result of the comparison, the BLBC TME signature 600 is associated with the closest signature cluster (when a distance-based comparison is performed, or the "closest" in the sense of whatever metric or measure of distance is used). In this example, BLBC TME signature 600 is associated with BLBC TME Type Cluster 5 610 (as shown by the consistent shading) because the measure of distance D5 between the BLBC TME signature 600 and (e.g., a centroid or other point representative of) cluster 610 is smaller than the measures of the distance D1, D2, D3, and D4 between the BLBC TME signature 600 and (e.g., a centroid or other point(s) representative of) clusters 602, 604, 606, and 608, respectively.

In some embodiments, a subject's TME signature may be associated with one of five breast cancer TME signature clusters by using a machine learning technique (e.g., such as k-nearest neighbors (KNN) or any other suitable classifier) to assign the TME signature to one of the five breast cancer TME signature clusters. The machine learning technique may be trained to assign TME signatures on the meta-cohorts represented by the signatures in the clusters.

In some embodiments, BLBC TME types comprise an Immune Enriched (IE) type, TLS (TLS) type (also referred to as B-cell enriched), Desert (D) type, Fibrotic (F) type, and Granulocyte enriched (G) type. In some embodiments, LNLBC TME types comprise an Immune Desert (D) type, Fibrotic (F) type, Immune-Enriched Non-fibrotic (IE) type, Immune-Enriched Fibrotic (IE/F) type, and Angiogenic (E) type. In some embodiments, H2EBC TME types comprises an Immune Desert (D) type, Fibrotic (F) type, Immune-Enriched, Non-fibrotic (IE) type, Moderately Immune-Enriched (IE-med) type, and Endothelium enriched (End-Ar-H) type. The breast cancer TME types described herein may be described by qualitative characteristics, for example high signals for certain gene expression signatures or scores or low signals for certain other gene expression signatures or scores. In some embodiments, a "high" signal refers to a gene expression signal or score (e.g., an enrichment score) that is at least 1-fold, 2-fold, 3-fold, 4-fold, 5-fold, 6-fold, 7-fold, 8-fold, 9-fold, 10-fold, 20-fold, 50-fold, 100-fold, 1000-fold, or more increased relative to the score of the same gene or gene group in a subject having a different type of breast cancer (e.g., a different TME type within the same intrinsic subtype of cancer, for example BLBC, LNLBC, or H2EBC). In some embodiments, a "low" signal refers to a gene expression signal or score (e.g., an enrichment score,) that is at least 1-fold, 2-fold, 3-fold, 4-fold, 5-fold, 6-fold, 7-fold, 8-fold, 9-fold, 10-fold, 20-fold, 50-fold, 100-fold, 1000-fold, or more decreased relative to the score of the same gene or gene group in a subject having a different type of TME (e.g., a different TME type within the same intrinsic subtype of cancer, for example BLBC, LNLBC, or H2EBC).

The tumor microenvironment of BC may contain variable numbers of immune cells, stromal cells, blood vessels and extracellular matrix.

In some embodiments, BLBC TME types comprise an Immune Enriched (IE) type, TLS (TLS) type (also referred to as B-cell enriched), Desert (D) type, Fibrotic (F) type, and Granulocyte enriched (G) type.

In some embodiments, BLBC Granulocyte (G) type TME is characterized by a high percentage of M1 macrophages, granulocytes, and cytokines regulating granulocyte traffic. In some embodiments, G type TME comprises a NFkB signaling pathway that is upregulated relative to other BLBC TME types. In some embodiments, G type BLBC TME comprises a high tumor proliferation rate signal relative to other BLBC TME types.

In some embodiments, the BLBC Immune-Enriched, Non-fibrotic (IE) TME type is characterized by abundant immune-active infiltrate-containing cytotoxic effector cells. In some embodiments, BLBC IE samples comprise an immune-inflamed. In some embodiments, BLBC IE type comprises a low percentage of malignant cells relative to other BLBC TME types. In some embodiments IE type BLBC TME type is associated with good prognosis.

In some embodiments, BLBC Fibrotic (F) TME type is highly fibrotic (e.g., relative to other BLBC TME types) with dense collagen formation. In some embodiments, F type BLBC TME samples comprise minimal leukocyte/lymphocyte infiltration (non-inflamed) relative to other BLBC TME types. In some embodiments, F type BLBC TME comprises high levels of angiogenesis relative to other BLBC TME types. In some embodiments, Cancer-associated fibroblasts (CAF) are abundant in BLBC F type TME samples. In some embodiments, BLBC TME type F is associated with poor prognosis.

In some embodiments, BLBC Immune Desert (D) type TME contains the highest percentage of malignant cells (relative to other BLBC TME types). In some embodiments, D type BLBC TME samples are characterized by minimal leukocyte/lymphocyte infiltration or absence of leukocyte/lymphocyte infiltration. In some embodiments, D type BLBC TME comprises an immune non-inflamed, immune desert phenotype. In some embodiments, D type BLBC TME type is associated with a high tumor proliferation rate and poor prognosis.

In some embodiments, BLBC B-Cell-Enriched, Tertiary Lymphoid Structure (TLS)-like (TLS) TME type is characterized by high levels of immune infiltrate, high vascularity, and a significant number of B cells, relative to other BLBC TME types. In some embodiments, BLBC TLS type TME comprises a medium prevalence of stromal and fibrotic elements relative to other BLBC TME types. In some embodiments, BLBC TME TLS type is associated with good prognosis.

In some embodiments not part of the claims, LNLBC TME types comprise an Immune Desert (D) type, Fibrotic (F) type, Immune-Enriched Non-fibrotic (IE) type, Immune-Enriched Fibrotic (IE/F) type, and Angiogenic (E) type.

In some examples, LNLBC Immune-Enriched, Fibrotic (IE/F) TME type is characterized by increased vascularization and a high level of immune infiltrate relative to other LNLBC TME types. In some examples, LNLBC IE/F TME type comprises an immune-inflamed phenotype. In some examples, LNLBC IE/F type TME comprises a low percentage of malignant cells relative to other LNLBC TME types. In some examples, LNLBC TME type IE/F is associated with low estrogen expression and a low tumor proliferation rate relative to other LNLBC TME types.

In some examples, LNLBC Immune-Enriched, Non-fibrotic (IE) TME type is characterized by abundant immune-active infiltrate-containing cytotoxic effector cells and regulatory T cells relative to other LNLBC TME types. In some examples, LNLBC IE type TME comprises an immune-inflamed phenotype. In some examples, LNLBC TME type IE is associated with a poor prognosis for patients on hormone therapy.

In some examples, LNLBC Fibrotic (F) type TME is highly fibrotic (relative to other LNLBC TME types) with dense collagen formation. In some embodiments, LNLBC type TME is characterized by minimal leukocyte/lymphocyte infiltration relative to other LNLBC TME types. In some examples, LNLBC F type TME comprises a non-inflamed phenotype. In some examples, Cancer-associated fibroblasts (CAF) are abundant in LNLBC F type TME samples. In some examples, epithelial-mesenchymal transition (EMT) is present in F type LNLBC TME samples.

In some examples, LNLBC Immune Desert (D) type LNLBC TME contains the highest percentage of malignant cells relative to other LNLBC TME types. In some examples, leukocyte/lymphocyte infiltration is minimal or completely absent compared to other LNLBC TME types. In some examples, LNLBC D type TME comprise an immune non-inflamed, immune desert phenotype. In some examples, type D LNLBC TME samples are characterized by high estrogen expression. In some examples, LNLBC type D TME is associated with a high tumor proliferation rate relative to other LNLBC TME types.

In some examples, LNLBC Angiogenic (E) TME type is characterized by intense angiogenesis and medium levels of immune infiltrate relative to other LNLBC TME types. In some examples, Cancer-associated fibroblasts (CAF) are abundant in LNLBC E type TME samples. In some examples, epithelial-mesenchymal transition (EMT) is present in E type LNLBC samples. In some examples, type E TME comprises high levels of pro-tumor cytokines relative to other LNLBC TME types. In some examples, LNLBC TME type E comprises low estrogen expression and a low tumor proliferation rate relative to other LNLBC TME types. In some examples, LNLBC E type is associated with a good prognosis for patients on hormone therapy.

In some embodiments not part of the claims, H2EBC TME types comprises an Immune Desert (D) type, Fibrotic (F) type, Immune-Enriched, Non-fibrotic (IE) type, Moderately Immune-Enriched (IE-med) type, and Endothelium enriched (End-Ar-H) type.

In some examples, H2EBC Immune Desert (D) type TME contains the highest percentage of malignant cells relative to other H2EBC TME types. In some embodiments, leukocyte/lymphocyte infiltration is minimal or completely absent in H2EBC D type TME samples. In some examples, H2EBC type D TME comprises an immune non-inflamed, immune desert phenotype. In some examples, type D H2EBC TME is characterized by high estrogen pathway activity compared to other H2EBC TME types.

In some examples, H2EBC Moderately Immune-Enriched (IE-med) type TME is characterized by a moderate number of tumor-infiltrating immune cells, including B cells, cytotoxic effector cells, and regulatory T cells, relative to other H2EBC TME types. In some embodiments, the level of immune cell abundance in IE-med type TME samples is lower than in the IE type H2EBC TME. In some examples, a low level of vascularization is present in H2EBC IE-med samples relative to other H2EBC TME types. In some examples, H2EBC TME type IE-med is associated with a poor prognosis on chemotherapy.

In some examples, H2EBC Immune-Enriched, Non-fibrotic (IE) type TME is characterized by abundant immune-active infiltrate containing cytotoxic effector cells and regulatory T cells relative to other H2EBC TME types. In some examples, IE type H2EBC TME comprises an immune-inflamed phenotype, with vascularization. In some examples, the percentage of malignant cells in H2EBC IE type TME is low relative to other H2EBC TME types. In some examples, H2EBC IE type TME is associated with a good prognosis on chemotherapy.

In some examples, Fibrotic, Hypoxic (F) type TME is highly vascularized relative to other H2EBC TME types. In some examples, samples of H2EBC F type TME are characterized by dense collagen formations and epithelial-mesenchymal transition (EMT). In some examples, H2EBC F type TME comprises an immune non-inflamed phenotype. Cancer-associated fibroblasts (CAF) are abundant in this H2EBC TME type relative to other H2EBC TME types.

In some examples, H2EBC Endothelium-rich (End-Ar-H) type TME is characterized by the highest number of endothelial cells relative to other H2EBC TME types. In some embodiments, End-Ar-H type is associated with angiogenesis and epithelial-mesenchymal transition (EMT). In some examples, End-Ar-H samples comprise an immune non-inflamed phenotype. In some examples, End-Ar-H samples comprise pro-tumor cytokines. In some embodiments, End-Ar-H TME type is characterized by high androgen pathway activity and a low tumor proliferation rate relative to other H2EBC TME types. In some examples, the End-Ar-H H2EBC TME type is characterized by higher vascularization relative to other H2EBC TME types.

Tables 4-6 below describe examples of breast cancer TME signatures and gene group scores produced by ssGSEA analysis and normalization (e.g., median scaling) of expression data from one or more breast cancer subjects.

Table 4: Representative gene group score values for BLBC TME Types

| 1st Quartile | B-Cell-Enriched, TLS-like | Fibrotic | Granulocyte-Enriched | Immune Desert | Immune-Enriched, Non-fibrotic |
|---|---|---|---|---|---|
| MHC I | -0.013772 | 1.195517 | -0.30691 | 1.868011 | 0.310702 |
| MHC II | 0.089579 | 0.940065 | -0.98134 | 2.331025 | 0.228533 |
| Coactivation molecules | 0.471358 | 0.810785 | -0.340691 | 1.218577 | -0.074828 |
| Effector cells | 0.383054 | 0.781105 | -0.386779 | 1.001363 | 0.322425 |
| NK cells | 0.412038 | 0.740612 | -0.290136 | 1.173359 | 0.101175 |
| T cells | 0.383088 | 0.912746 | -0.490777 | 1.571505 | 0.115368 |
| B cells | 0.839816 | 0.823325 | -0.541291 | 0.867954 | -0.383564 |
| M1 signatures | -0.106299 | 0.891427 | 0.368548 | 1.154469 | -0.184765 |
| Th1 signature | 0.501204 | -1.02013 | -0.435158 | 1.085056 | 0.26494 |
| Antitumor cytokines | -0.134263 | 0.918883 | 0.328389 | 1.505027 | 0.113096 |
| Checkpoint inhibition | 0.379976 | 0.809873 | -0.323628 | 1.062542 | 0.128878 |
| Treg | 0.319099 | 0.900727 | -0.442572 | 1.045394 | 0.074351 |
| Neutrophil signature | -0.538689 | 0.692874 | -0.17063 | 0.998408 | -0.610051 |
| Granulocyte traffic | -0.617585 | 0.863299 | 0.76044 | 0.670625 | -0.381876 |
| MDSC | 0.06531 | 0.859666 | -0.147172 | 1.477085 | -0.1146 |
| Macrophages | -0.068111 | 0.615238 | -0.105753 | 1.460345 | -0.185848 |
| Th2 signature | -0.013797 | -0.89074 | -0.092029 | 1.304006 | -0.35512 |
| Protumor cytokines | -0.079504 | 0.072129 | -0.35075 | -1.20093 | -1.08095 |
| CAF | -0.316691 | 0.478458 | -0.32214 | 1.262119 | -1.49582 |
| Matrix | -0.31461 | 0.293479 | -0.476573 | 1.027741 | -1.570277 |
| Angiogenesis | -0.35908 | 0.348278 | -0.150087 | 0.781442 | -1.170951 |
| Endothelium | 0.043821 | 0.076047 | -0.918279 | 1.281854 | -0.933164 |
| Proliferation rate | -1.304074 | 1.280098 | 0.332367 | 0.489096 | 0.001694 |
| EMT signature | -0.417742 | 0.280867 | -0.551044 | 1.073161 | -1.109461 |
| TLS | 0.637026 | 0.620717 | -0.534448 | 1.486764 | -0.030314 |
| Follicular dendritic cells | -0.008343 | 0.620564 | -0.614763 | 2.149971 | -0.594461 |
| Follicular B helper T cells | 0.563762 | 0.523048 | -0.85098 | -1.42135 | -0.105815 |
| Granulocytes | -0.491617 | 0.697456 | 0.369863 | 1.255692 | -0.671726 |
| | | | | | |
| Median | B-Cell-Enriched, TLS-like | Fibrotic | Granulocyte-Enriched | Immune Desert | Immune-Enriched, Non-fibrotic |

(continued)

| 1st Quartile | B-Cell-Enriched, TLS-like | Fibrotic | Granulocyte-Enriched | Immune Desert | Immune-Enriched, Non-fibrotic |
|---|---|---|---|---|---|
| MHC I | 0.4388 | 0.545877 | 0.059941 | 1.069063 | 0.599759 |
| MHC II | 0.518856 | 0.229051 | -0.040952 | 1.742506 | 0.51775 |
| Coactivation molecules | 0.832481 | 0.213117 | 0.050329 | -0.93182 | 0.549353 |
| Effector cells | 0.750858 | 0.424397 | -0.035661 | 0.867026 | 0.899943 |
| NK cells | 0.677002 | 0.442623 | 0.112637 | 0.789593 | 0.678327 |
| T cells | 0.715254 | 0.361191 | -0.220634 | 1.254844 | 0.451614 |
| B cells | 1.605124 | 0.364844 | -0.277231 | 0.588113 | 0.359428 |
| M1 signatures | 0.523866 | 0.436623 | 0.878541 | -0.67449 | 0.480742 |
| Th1 signature | 0.871187 | 0.493218 | -0.04452 | 0.680496 | 0.842014 |
| Antitumor cytokines | 0.450992 | 0.533655 | 0.554197 | 1.063449 | 0.642374 |
| Checkpoint inhibition | 1.019152 | 0.395752 | 0.08691 | 0.817885 | 0.791015 |
| Treg | 0.852125 | 0.266814 | -0.095805 | 0.406351 | 0.544114 |
| Neutrophil signature | 0.245832 | -0.03296 | 0.606084 | 0.375399 | 0.167506 |
| Granulocyte traffic | -0.079501 | 0.121515 | 2.033171 | 0.035641 | -0.035897 |
| MDSC | 0.633459 | -0.30715 | 0.488374 | 0.960012 | 0.38603 |
| Macrophages | 0.629024 | 0.162259 | 0.531652 | 1.074313 | 0.311797 |
| Th2 signature | 0.65462 | 0.186634 | 0.142503 | 0.709894 | 0.543895 |
| Protumor cytokines | 0.126044 | 0.901149 | 0.048368 | 0.807342 | -0.539016 |
| CAF | 0.114671 | 0.838173 | 0.128034 | 0.556796 | -1.052595 |
| Matrix | 0.151675 | 0.769624 | 0.137905 | 0.367892 | -0.978111 |
| Angiogenesis | 0.211272 | 0.882755 | 0.233441 | 0.402501 | -0.729234 |
| Endothelium | 0.549846 | 0.728809 | -0.383032 | 0.668995 | -0.561547 |
| Proliferation rate | -0.714623 | 0.465644 | 0.673913 | 0.223454 | 0.39582 |
| EMT signature | 0.100662 | 1.008029 | -0.12421 | 0.382939 | -0.846386 |
| TLS | 1.042933 | 0.239931 | -0.248545 | 0.979078 | 0.407842 |
| Follicular dendritic cells | 0.470502 | 0.399273 | -0.109886 | -1.57116 | 0.045007 |
| Follicular B helper T cells | 0.864423 | 0.180106 | -0.122911 | 0.829971 | 0.402736 |
| Granulocytes | -0.031271 | -0.10476 | 1.409272 | 0.458858 | -0.402059 |
| | | | | | |
| 3rd quartile | B-Cell-Enriched, TLS-like | Fibrotic | Granulocyte-Enriched | Immune Desert | Immune-Enriched, Non-fibrotic |
| MHC I | 0.856531 | 0.050552 | 0.417453 | 0.283614 | 0.887408 |
| MHC II | 0.832287 | 0.124013 | 0.479371 | 0.961926 | 0.74535 |

(continued)

| 1st Quartile | B-Cell-Enriched, TLS-like | Fibrotic | Granulocyte-Enriched | Immune Desert | Immune-Enriched, Non-fibrotic |
|---|---|---|---|---|---|
| Coactivation molecules | 1.36251 | 0.230676 | 0.430194 | 0.486974 | 1.389481 |
| Effector cells | 1.450718 | 0.029469 | 0.593618 | 0.314249 | 1.024273 |
| NK cells | 1.386844 | 0.221935 | 0.491981 | 0.386613 | 1.050958 |
| T cells | 1.28236 | 0.221771 | 0.20432 | 0.567645 | 0.766395 |
| B cells | 1.958389 | 0.59268 | 0.094922 | 0.373209 | 0.851565 |
| M1 signatures | 0.951001 | 0.171254 | 1.363807 | 0.392281 | 1.472314 |
| Th1 signature | 1.315333 | 0.104994 | 0.19002 | 0.217872 | 1.296162 |
| Antitumor cytokines | 0.764427 | 0.046086 | 1.059702 | 0.238053 | 1.123654 |
| Checkpoint inhibition | 1.577002 | 0.051167 | 0.539108 | 0.384169 | 1.391379 |
| Treg | 1.294313 | 0.258976 | 0.412065 | 0.126482 | 0.93952 |
| Neutrophil signature | 0.925393 | 0.519809 | 2.178384 | 0.002887 | 1.062851 |
| Granulocyte traffic | 0.361208 | 0.625462 | 2.531776 | 0.305622 | 0.991968 |
| MDSC | 0.938029 | 0.048645 | 1.099426 | 0.333219 | 0.930869 |
| Macrophages | 1.05575 | 0.405549 | 0.98854 | 0.536937 | 0.996389 |
| Th2 signature | 1.49488 | 0.330998 | 0.608121 | 0.315611 | 1.117524 |
| Protumor cytokines | 0.519058 | 1.479138 | 0.656721 | -0.28671 | 0.028825 |
| CAF | 0.625054 | 1.03302 | 0.443802 | 0.234699 | -0.063872 |
| Matrix | 0.469206 | 1.131938 | 0.5805 | 0.15359 | -0.17732 |
| Angiogenesis | 0.477757 | 1.289833 | 1.126464 | 0.082292 | -0.284289 |
| Endothelium | 1.141564 | 1.257356 | 0.148922 | 0.180322 | 0.158205 |
| Proliferation rate | -0.05994 | 0.089251 | 1.052134 | 0.807769 | 0.749714 |
| EMT signature | 0.682099 | 1.424015 | 0.239926 | 0.207197 | -0.224901 |
| TLS | 1.510424 | 0.447178 | 0.207576 | 0.237317 | 0.688721 |
| Follicular dendritic cells | 0.95364 | 0.643892 | 0.428894 | 0.473376 | 0.591679 |
| Follicular B helper T cells | 1.462768 | 0.319308 | 0.567517 | 0.460211 | 0.857215 |
| Granulocytes | 0.364079 | 0.505298 | 1.992993 | 0.166947 | 0.683427 |

Table 5: Representative gene group score values for LNLBC TME Types

| 1st quartile | Fibrotic | Angiogenic E | Immune Desert | Immune-Enriched, Fibrotic | Immune-Enriched, Non-fibrotic |
|---|---|---|---|---|---|
| Angiogenesis | -0.36924 | 0.619367 | -0.93161 | -0.08422 | -1.18524 |
| Endothelium | -0.48902 | 0.653813 | -0.95409 | 0.409219 | -0.87163 |
| CAF | 0.196003 | 0.271577 | -1.63976 | -0.13826 | -1.60395 |

(continued)

| 1st quartile | Fibrotic | Angiogenic E | Immune Desert | Immune-Enriched, Fibrotic | Immune-Enriched, Non-fibrotic |
|---|---|---|---|---|---|
| Matrix | 0.230641 | -0.047042 | -1.33207 | -0.46171 | -1.53722 |
| Matrix remodeling | -0.00045 | -0.160366 | -1.44037 | -0.45366 | -0.90332 |
| Protumor cytokines | -0.11498 | 0.410832 | -1.23501 | 0.018665 | -0.92041 |
| Granulocyte traffic | -0.71115 | -0.431087 | -0.82976 | -0.54128 | -0.68992 |
| Macrophages | -0.72912 | -0.483917 | -1.11209 | -0.04233 | -0.05145 |
| Macrophage DC traffic | -0.95065 | -0.163372 | -1.13977 | 0.14808 | -0.04177 |
| MDSC | -0.92618 | -0.323607 | -1.0875 | 0.200996 | 0.032503 |
| Treg | -0.95094 | -1.135712 | -0.7443 | 0.067593 | 0.245039 |
| M1 signatures | -0.83963 | -0.411836 | -0.96707 | 0.086466 | -0.13385 |
| MHC II | -1.00199 | -0.19787 | -2 | 0.311961 | -0.13784 |
| Antitumor cytokines | -0.90753 | -0.513848 | -0.96816 | -0.16751 | -0.04416 |
| B cells | -0.84962 | -0.255643 | -0.82526 | 0.292477 | -0.20918 |
| NK cells | -0.92198 | -0.273009 | -0.91213 | 0.413535 | 0.422618 |
| Checkpoint inhibition | -0.96549 | -0.287392 | -1.26521 | 0.33935 | 0.152629 |
| Effector cells | -0.91518 | -0.263565 | -1.03844 | 0.479078 | 0.186756 |
| T cells | -0.97974 | -0.292555 | -1.21074 | 0.434941 | 0.100981 |
| Th1 signature | -0.836 | -0.400943 | -0.9927 | 0.414641 | 0.251526 |
| T cell traffic | -0.96579 | -0.195315 | -1.1588 | 0.524879 | 0.130298 |
| MHC I | -0.86838 | -0.664172 | -0.92453 | -0.13929 | 0.098891 |
| EMT signature | -0.08029 | 0.228207 | -1.22124 | -0.0607 | -1.06962 |
| Proliferation rate | -0.63669 | -1.305355 | 0.102428 | -1.05696 | -0.02149 |
| | | | | | |
| Median | Fibrotic | Angiogenic E | Immune Desert | Immune-Enriched, Fibrotic | Immune-Enriched, Non-fibrotic |
| Angiogenesis | 0.13089 | 1.089985 | -0.41034 | 0.427333 | -0.68822 |
| Endothelium | -0.08616 | 1.200428 | -0.43036 | 0.861957 | -0.45203 |
| CAF | 0.528727 | 0.712068 | -0.90689 | 0.266423 | -0.66533 |
| Matrix | 0.657581 | 0.452114 | -0.74865 | 0.135737 | -0.70492 |
| Matrix remodeling | 0.5564 | 0.346783 | -0.56107 | 0.098105 | -0.27868 |
| Protumor cytokines | 0.321563 | 0.757651 | -0.71326 | 0.572426 | -0.40169 |
| Granulocyte traffic | -0.02405 | 0.270705 | -0.26557 | 0.170967 | -0.03399 |
| Macrophages | -0.28629 | 0.1134 | -0.54525 | 0.512782 | 0.594743 |
| Macrophage DC traffic | -0.32357 | 0.290999 | -0.71422 | 0.7047 | 0.47269 |
| MDSC | -0.42952 | 0.135352 | -0.57882 | 0.693916 | 0.616412 |
| Treg | -0.31546 | -0.704732 | -0.20383 | 0.424024 | 0.888721 |
| M1 signatures | -0.30831 | 0.155659 | -0.55989 | 0.709953 | 0.419937 |
| MHC II | -0.33581 | 0.245489 | -1.2503 | 0.627193 | 0.487676 |

(continued)

| Median | Fibrotic | Angiogenic E | Immune Desert | Immune-Enriched, Fibrotic | Immune-Enriched, Non-fibrotic |
|---|---|---|---|---|---|
| Antitumor cytokines | -0.29894 | 0.086875 | -0.43325 | 0.328085 | 0.482788 |
| B cells | -0.51901 | 0.268217 | -0.48954 | 1.201947 | 0.476807 |
| NK cells | -0.54757 | 0.128339 | -0.55265 | 1.132914 | 0.98596 |
| Checkpoint inhibition | -0.43772 | 0.137738 | -0.61535 | 1.025923 | 0.745253 |
| Effector cells | -0.57352 | 0.153858 | -0.63497 | 1.3018 | 0.870473 |
| T cells | -0.52281 | 0.219783 | -0.7133 | 0.989078 | 0.605005 |
| Th1 signature | -0.5124 | -0.018822 | -0.55245 | 1.04191 | 0.940718 |
| T cell traffic | -0.49037 | 0.09156 | -0.70828 | 0.875596 | 0.707156 |
| MHC I | -0.3142 | -0.129865 | -0.34889 | 0.421479 | 0.811843 |
| EMT signature | 0.442752 | 0.810241 | -0.74304 | 0.410865 | -0.37901 |
| Proliferation rate | -0.17258 | -0.779981 | 0.604379 | -0.66437 | 0.539958 |
| | | | | | |
| 3rd Quartile | Fibrotic | Angiogenic E | Immune Desert | Immune-Enriched, Fibrotic | Immune-Enriched, Non-fibrotic |
| Angiogenesis | 0.692275 | 1.60827 | 0.235934 | 1.06062 | -0.16499 |
| Endothelium | 0.550623 | 1.746773 | 0.077353 | 1.296412 | 0.003981 |
| CAF | 0.785389 | 1.037361 | -0.29004 | 0.666753 | 0.106049 |
| Matrix | 1.135632 | 0.91872 | -0.09359 | 0.596676 | 0.065372 |
| Matrix remodeling | 1.114831 | 0.980053 | 0.000055 | 0.649909 | 0.649363 |
| Protumor cytokines | 0.972247 | 1.445929 | -0.28195 | 1.166077 | 0.103233 |
| Granulocyte traffic | 0.581148 | 0.875169 | 0.443668 | 0.748585 | 0.544824 |
| Macrophages | 0.302605 | 0.726814 | -0.01145 | 0.953983 | 1.052275 |
| Macrophage DC traffic | 0.146293 | 0.784784 | -0.16614 | 1.199187 | 1.000149 |
| MDSC | 0.126419 | 0.760034 | -0.19834 | 1.496923 | 1.444611 |
| Treg | 0.217277 | -0.126469 | 0.341343 | 1.061922 | 1.625762 |
| M1 signatures | 0.193782 | 0.866331 | -0.07859 | 1.289762 | 1.074833 |
| MHC II | 0.188742 | 0.589235 | -0.4559 | 1.059064 | 0.866353 |
| Antitumor cytokines | 0.381603 | 0.628777 | 0.10998 | 1.201661 | 1.087728 |
| B cells | 0.07168 | 0.850737 | -0.03099 | 2 | 1.463667 |
| NK cells | -0.21137 | 0.546466 | -0.15555 | 1.908609 | 1.864799 |
| Checkpoint inhibition | 0.002177 | 0.573729 | -0.26911 | 1.829039 | 1.762289 |
| Effector cells | -0.16534 | 0.61714 | -0.31702 | 1.989068 | 1.878515 |
| T cells | -0.03355 | 0.573534 | -0.27133 | 1.559133 | 1.199031 |
| Th1 signature | -0.02777 | 0.471833 | -0.14758 | 1.934534 | 1.910713 |
| T cell traffic | -0.00507 | 0.541907 | -0.2101 | 1.369119 | 1.201642 |
| MHC I | 0.26045 | 0.184581 | 0.37491 | 1.021562 | 1.444376 |
| EMT signature | 0.9745 | 1.264448 | -0.22145 | 0.879767 | 0.099785 |

(continued)

| 3rd Quartile | Fibrotic | Angiogenic E | Immune Desert | Immune-Enriched, Fibrotic | Immune-Enriched, Non-fibrotic |
|---|---|---|---|---|---|
| Proliferation rate | 0.362708 | -0.225406 | 1.123565 | -0.17663 | 1.024834 |

Table 6: Representative gene group score values for H2EBC TME Types

| 1st quartile | Fibrotic, Hypoxic | End-Ar-H | Immune Desert | Immune-Enriched, Non-fibrotic | Moderately Immune-Enriched |
|---|---|---|---|---|---|
| MHC I | -0.50996 | -1.028705 | -1.25939 | 0.379773 | -0.214935 |
| MHC II | -0.34436 | -0.379657 | -2 | 0.064105 | -0.042585 |
| Coactivation molecules | -0.32097 | -0.386773 | -1.21996 | -0.05712 | -0.291095 |
| Effector cells | -0.65523 | -0.573295 | -1.19283 | 0.450238 | -0.107277 |
| T cell traffic | -0.64523 | -1.006717 | -1.82082 | 0.120647 | -0.028718 |
| NK cells | -0.72467 | -0.607152 | -1.1673 | 0.51681 | -0.063312 |
| T cells | -0.63059 | -0.457309 | -1.41539 | 0.260505 | -0.181872 |
| B cells | -0.77462 | -0.207271 | -0.99811 | 0.27495 | -0.022677 |
| M1 signatures | -0.51568 | -0.837693 | -1.76069 | -0.32961 | -0.316423 |
| Th1 signature | -0.7237 | -0.761138 | -1.07605 | 0.336966 | 0.028795 |
| Antitumor cytokines | -0.37841 | -1.301966 | -1.27443 | -0.19857 | -0.062392 |
| Checkpoint inhibition | -0.62843 | -0.617112 | -1.31882 | 0.228292 | -0.04212 |
| Treg | -0.59164 | -0.764338 | -1.0296 | 0.39409 | -0.178607 |
| T reg traffic | -1.0424 | -0.21563 | -1.21014 | -0.0321 | -0.131326 |
| Neutrophil signature | -0.46345 | -0.916782 | -0.69067 | -0.27786 | -0.391887 |
| Granulocyte traffic | -0.71757 | -0.674164 | -0.73869 | -0.5264 | -0.513552 |
| MDSC | -0.33399 | -0.829623 | -1.17973 | 0.03466 | -0.034828 |
| MDSC traffic | 0.160562 | -0.307156 | -1.385 | -0.44947 | -0.69471 |
| Macrophages | -0.05704 | -0.815826 | -1.13472 | -0.38766 | -0.456883 |
| Macrophage DC traffic | -0.13815 | -0.483296 | -1.37643 | -0.06402 | -0.373982 |
| Th2 signature | -0.80658 | -0.909667 | -0.80331 | 0.05669 | -0.007402 |
| Protumor cytokines | 0.178807 | 0.00501 | -0.88006 | -0.90399 | -1.232665 |
| CAF | 0.445249 | 0.026559 | -0.58611 | -1.72946 | -1.128358 |
| Matrix | 0.375355 | -0.12893 | -0.4501 | -1.30491 | -1.068014 |
| Matrix remodeling | 0.12767 | -0.963133 | -0.41921 | -1.02318 | -0.961999 |
| Angiogenesis | 0.39538 | 0.161302 | -0.70316 | -1.10971 | -0.576715 |
| Endothelium | -0.33554 | 0.446186 | -0.96566 | -0.93995 | -0.712441 |
| Proliferation rate | -0.68437 | -1.558976 | -0.30026 | -0.31655 | 0.089707 |
| EMT signature | 0.415636 | -0.036412 | -0.72712 | -1.09096 | -0.749257 |
| | | | | | |

(continued)

| Median | Fibrotic, Hypoxic | Angiogenic E | Immune Desert | Immune-Enriched, Non-fibrotic | Moderately Immune-Enriched |
|---|---|---|---|---|---|
| MHC I | -0.12811 | -0.485408 | -0.75787 | 0.718462 | 0.227725 |
| MHC II | -0.0594 | -0.237289 | -1.52001 | 0.772672 | 0.470783 |
| Coactivation molecules | 0.07168 | -0.206707 | -0.78413 | 0.861883 | 0.343689 |
| Effector cells | -0.40364 | -0.022328 | -0.96834 | 0.94198 | 0.305983 |
| T cell traffic | -0.11423 | -0.433898 | -1.3495 | 0.626189 | 0.202896 |
| NK cells | -0.44831 | -0.093501 | -0.89709 | 1.011695 | 0.293172 |
| T cells | -0.31119 | 0.16262 | -1.06743 | 0.74497 | 0.303976 |
| B cells | -0.59984 | 0.023371 | -0.71784 | 1.051779 | 0.544435 |
| M1 signatures | 0.272214 | -0.368761 | -1.25043 | 0.208889 | 0.131041 |
| Th1 signature | -0.29888 | -0.105487 | -0.95917 | 0.801748 | 0.484556 |
| Antitumor cytokines | 0.272224 | -0.551443 | -0.93915 | 0.515326 | 0.258733 |
| Checkpoint inhibition | -0.20706 | -0.337757 | -0.91787 | 1.125389 | 0.439868 |
| Treg | -0.41773 | -0.528091 | -0.6378 | 0.793172 | 0.320043 |
| T reg traffic | -0.43576 | 0.080441 | -0.88045 | 0.512966 | 0.126069 |
| Neutrophil signature | 0.066821 | -0.012836 | -0.21043 | 0.371707 | -0.194622 |
| Granulocyte traffic | 0.275167 | 0.295816 | -0.48693 | 0.070392 | 0.454397 |
| MDSC | 0.021514 | -0.499829 | -0.89362 | 0.490726 | 0.262227 |
| MDSC traffic | 0.961655 | 0.001749 | -0.99655 | 0.216713 | -0.165377 |
| Macrophages | 0.305488 | -0.530369 | -0.61255 | 0.319173 | 0.300468 |
| Macrophage DC traffic | 0.187631 | -0.228383 | -1.07524 | 0.674737 | 0.287228 |
| Th2 signature | -0.26724 | -0.088883 | -0.32463 | 0.440613 | 0.656094 |
| Protumor cytokines | 0.648698 | 0.652725 | -0.68926 | -0.0984 | -0.508638 |
| CAF | 0.683589 | 0.283612 | -0.28693 | -0.90469 | -0.475546 |
| Matrix | 0.714603 | 0.380983 | 0.084721 | -0.70781 | -0.622031 |
| Matrix remodeling | 0.670734 | -0.386139 | 0.02158 | -0.65735 | 0.235042 |
| Angiogenesis | 0.741704 | 0.631906 | -0.28919 | -0.43285 | -0.195982 |
| Endothelium | 0.107818 | 0.947673 | -0.64606 | -0.04333 | -0.364012 |
| Proliferation rate | -0.26581 | -0.946444 | 0.506088 | 0.386563 | 0.19457 |
| EMT signature | 0.736558 | 0.676782 | -0.52808 | -0.35552 | -0.23523 |
| | | | | | |
| 3rd quartile | Fibrotic, Hypoxic | Angiogenic E | Immune Desert | Immune-Enriched, Non-fibrotic | Moderately Immune-Enriched |
| MHC I | 0.419841 | -0.021644 | -0.0348 | 1.094373 | 0.601802 |
| MHC II | 0.445526 | 0.264838 | -0.86696 | 0.932629 | 0.578364 |
| Coactivation molecules | 0.388757 | 0.181029 | -0.54994 | 1.684568 | 0.763755 |
| Effector cells | -0.10134 | 0.585693 | -0.67755 | 1.392372 | 0.514871 |
| T cell traffic | 0.176427 | 0.509381 | -0.93828 | 0.938324 | 0.448058 |

(continued)

| 3rd quartile | Fibrotic, Hypoxic | Angiogenic E | Immune Desert | Immune-Enriched, Non-fibrotic | Moderately Immune-Enriched |
|---|---|---|---|---|---|
| NK cells | -0.03344 | 0.370933 | -0.64212 | 1.414526 | 0.620201 |
| T cells | -0.02911 | 0.705969 | -0.75481 | 1.266674 | 0.682386 |
| B cells | -0.32283 | 1.129845 | -0.35977 | 1.924102 | 0.778214 |
| M1 signatures | 0.894738 | 0.329647 | -0.77894 | 1.081893 | 0.4589 |
| Th1 signature | -0.07611 | 0.113575 | -0.66746 | 1.144752 | 0.678717 |
| Antitumor cytokines | 1.141645 | 0.014635 | -0.59875 | 0.943408 | 0.620808 |
| Checkpoint inhibition | 0.101434 | 0.262815 | -0.39776 | 1.557846 | 1.014721 |
| Treg | -0.20999 | -0.040648 | 0.434791 | 1.583633 | 0.801358 |
| T reg traffic | 0.350959 | 0.623394 | -0.52286 | 1.150516 | 0.951535 |
| Neutrophil signature | 1.145782 | 1.447738 | 0.314626 | 0.810644 | 0.323271 |
| Granulocyte traffic | 1.878027 | 0.614887 | 0.093277 | 0.747848 | 0.871462 |
| MDSC | 0.347407 | 0.187718 | -0.5654 | 0.767224 | 0.575757 |
| MDSC traffic | 1.137178 | 0.787931 | -0.72856 | 0.879935 | 0.317335 |
| Macrophages | 0.688573 | 0.035164 | -0.31152 | 0.784939 | 0.839137 |
| Macrophage DC traffic | 0.540477 | 0.102071 | -0.39507 | 0.920481 | 0.738431 |
| Th2 signature | 0.155824 | 0.67449 | -0.22749 | 1.431603 | 0.924031 |
| Protumor cytokines | 0.958121 | 1.459723 | -0.18583 | 0.359691 | -0.230075 |
| CAF | 1.025111 | 0.797566 | 0.014949 | 0.020443 | 0.317489 |
| Matrix | 1.067071 | 0.885291 | 0.500804 | -0.18006 | -0.343393 |
| Matrix remodeling | 1.346909 | 0.540641 | 0.415946 | 0.174138 | 0.667884 |
| Angiogenesis | 1.200546 | 1.375148 | 0.060622 | 0.251565 | 0.002108 |
| Endothelium | 0.553925 | 1.656375 | -0.27833 | 0.644128 | 0.521416 |
| Proliferation rate | 0.39812 | -0.627743 | 0.796286 | 0.928018 | 0.574112 |
| EMT signature | 1.561173 | 1.059518 | -0.2147 | 0.055389 | 0.342763 |

The present disclosure describes methods for identifying a subject having, suspected of having, or at risk of having BC as having an increased likelihood of having a good prognosis (e.g., as measured by overall survival (OS) or progression-free survival (PFS). In some embodiments, the method comprises determining a BC TME type of the subject as described herein.

In some examples, the methods comprise identifying the subject as having a decreased risk of BC progression relative to other BC TME types. In some examples, "decreased risk of BC progression" may indicate better prognosis of BC or decreased likelihood of having advanced disease in a subject. In some examples, "decreased risk of BC progression" may indicate that the subject who has BC is expected to be more responsive to certain treatments. For instance, "decreased risk of BC progression" indicates that a subject is at least 10%, 20%, 30%, 40%, 50%, 60%, 70%, 80%, 90%, or 100% likely to experience a progression-free survival event (e.g., relapse, retreatment, or death) than another BC patient or population of BC patients (e.g., patients having BC, but not the same BC TME type as the subject).

In some examples, the methods further comprise identifying the subject as having an increased risk of BC progression relative to other BC TME types. In some examples, "increased risk of BC progression" may indicate less positive prognosis of BC or increased likelihood of having advanced disease in a subject. In some examples, "increased risk of BC progression" may indicate that the subject who has BC is expected to be less responsive or unresponsive to certain treatments and show less or no improvements of disease symptoms. For instance, "increased risk of BC progression" indicates that a subject is at least 10%, 20%, 30%, 40%, 50%, 60%, 70%, 80%, 90%, or 100% more likely to experience a progression-free survival event (e.g., relapse, retreatment, or death) than another BC patient or population of BC patients (e.g., patients having BC, but not the same BC TME type as the subject).

In some examples, the methods described herein comprise the use of at least one computer hardware processor to perform the determination.

In some examples, the present disclosure provides a method for providing a prognosis, predicting survival, or stratifying patient risk of a subject suspected of having, or at risk of having BC. In some embodiments, the method comprises determining a BC TME type of the subject as described herein.

*Updating TME Clusters Based on New Data*

Techniques for generating breast cancer TME clusters are described herein. It should be appreciated that the TME clusters may be updated as additional TME signatures are computed for patients. In some embodiments, the TME signature of the subject is one of a threshold number TME signatures for a threshold number of subjects. In some embodiments, when the threshold number of TME signatures is generated the TME signature clusters are updated. For example, once a threshold number of new TME signatures are obtained (e.g., 1 new signature, 10 new signatures, 100 new signatures, 500 new signatures, any suitable threshold number of signatures in the range of 10-1,000 signatures), the new signatures may be combined with the TME signatures previously used to generate the TME clusters and the combined set of old and new TME signatures may be clustered again (e.g., using any of the clustering algorithms described herein or any other suitable clustering algorithm) to obtain an updated set of TME signature clusters.

In this way, data obtained from a future patient may be analyzed in a way that takes advantage of information learned from patients whose TME signature was computed prior to that of the future patient. In this sense, the machine learning techniques described herein (e.g., the unsupervised clustering machine learning techniques) are adaptive and learn with the accumulation of new patient data. This facilitates improved characterization of the TME type that future patients may have and may improve the selection of treatment for those patients.

*Therapeutic Indications*

The disclosure also describes methods of identifying or selecting a therapeutic agent for a subject based upon determination of the subject's breast cancer TME type. The disclosure is based, in part, on the recognition that subjects having combinations of intrinsic breast cancer type (e.g., BLBC, LNLBC, H2EBC) and certain TME types of those cancers (e.g., BLBC G type, BLBC IE type, BLBC TLS type, LNLBC IE type, LNLBC IE/F type, H2EBC IE type, H2EBC IE-med type) have an increased likelihood of responding to certain therapies (e.g., immunotherapeutic agents) relative to subjects having other combinations of intrinsic breast cancer and TME types. The disclosure is also based, in part, on the recognition that subjects having TME type F (e.g., BLBC F type, LNLBC F type, H2EBC F type) have an increased likelihood of responding to anti-VEGF therapies relative to subjects having other combinations of intrinsic breast cancer and TME types. The disclosure also relates to the recognition that subject having BLBC type D may respond to tyrosine kinase inhibitors (TKIs).

In some embodiments, the therapeutic agents are immuno-oncology (IO) agents. An IO agent may be a small molecule, peptide, protein (e.g., antibody, such as monoclonal antibody), interfering nucleic acid, or a combination of any of the foregoing. In some embodiments, the IO agents comprise a PD1 inhibitor, PD-L1 inhibitor, or PD-L2 inhibitor. Examples of IO agents include but are not limited to cemiplimab, nivolumab, pembrolizumab, avelumab, durvalumab, atezolizumab, BMS1166, BMS202, etc. In some embodiments, the IO agents comprise a combination of atezolizumab and albumin-bound paclitaxel, pembrolizumab and albumin-bound paclitaxel, pembrolizumab and paclitaxel, or pembrolizumab and Gemcitabine and Carboplatin.

In some embodiments, the therapeutic agents are tyrosine kinase inhibitors (TKIs). A TKI may be a small molecule, peptide, protein (e.g., antibody, such as monoclonal antibody), interfering nucleic acid, or a combination of any of the foregoing. Examples of TKIs include but are not limited to Axitinib (Inlyta®), Cabozantinib (Cabometyx®), Imatinib mesylate (Gleevec®), Dasatinib (Sprycel®), Nilotinib (Tasigna®), Bosutinib (Bosulif®), Sunitinib (Sutent®), etc. In some embodiments, the TKI inhibitor comprises neratinib, apatinib, toripalimab and anlotinib, or anlotinib.

In some embodiments, the therapeutic agents are anti-VEGF agents. An anti-VEGF agent may be a small molecule, peptide, protein (e.g., antibody, such as monoclonal antibody), interfering nucleic acid, or a combination of any of the foregoing. Examples of anti-VEGF therapies include but are not limited to Bevacizumab (Avastin®), Sunitinib, Sorafenib, Pazopanib, etc. In some embodiments, the anti-VEGF agent comprises liposomal doxorubicin, bevacizumab, and everolimus.

In some embodiments not part of the claims, methods described by the disclosure further comprise a step of administering one or more therapeutic agents to the subject based upon the determination of the subject's TME type. In some embodiments not part of the claims, a subject is administered one or more (e.g., 1, 2, 3, 4, 5, or more) IO agents. In some embodiments not part of the claims, a subject is administered one or more (e.g., 1, 2, 3, 4, 5, or more) TKIs. In some embodiments not part of the claims, a subject is administered one or more (e.g., 1, 2, 3, 4, 5, or more) anti-VEGF agents.

Aspects of the disclosure not part of the claims relate to methods of treating a subject having (or suspected or at risk of having) breast cancer based upon a determination of the breast cancer TME type of the subject. In some embodiments not part of the claims, the methods comprise administering one or more (e.g., 1, 2, 3, 4, 5, or more) therapeutic agents to the subject. In some embodiments not part of the claims, the therapeutic agent (or agents) administered to the subject are selected from small molecules, peptides, nucleic acids, radioisotopes, cells (e.g., CAR T-cells, etc.), and combinations thereof. Examples of therapeutic agents include chemotherapies (e.g., cytotoxic agents, etc.), immunotherapies (e.g., immune checkpoint inhibitors, such as PD-1 inhibitors, PD-L1 inhibitors, etc.), antibodies (e.g., anti-HER2 antibodies), cellular therapies (e.g. CAR T-cell therapies), gene silencing therapies (e.g., interfering RNAs, CRISPR, etc.), antibody-drug conjugates (ADCs), and combinations thereof.

In some embodiments not part of the claims, a subject is administered an effective amount of a therapeutic agent. "An effective amount" as used herein refers to the amount of each active agent required to confer therapeutic effect on the subject, either alone or in combination with one or more other active agents. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons, or for virtually any other reasons.

Empirical considerations, such as the half-life of a therapeutic compound, generally contribute to the determination of the dosage. For example, antibodies that are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally (but not necessarily) based on treatment, and/or suppression, and/or amelioration, and/or delay of a cancer. Alternatively, sustained continuous release formulations of an anti-cancer therapeutic agent may be appropriate. Various formulations and devices for achieving sustained release are known in the art.

In some embodiments not part of the claims, dosages for an anti-cancer therapeutic agent as described herein may be determined empirically in individuals who have been administered one or more doses of the anti-cancer therapeutic agent. Individuals may be administered incremental dosages of the anti-cancer therapeutic agent. To assess efficacy of an administered anti-cancer therapeutic agent, one or more aspects of a cancer (e.g., tumor microenvironment, tumor formation, tumor growth, or TME types, etc.) may be analyzed.

Generally, for administration of any of the anti-cancer antibodies described herein, an initial candidate dosage may be about 2 mg/kg. For the purpose of the present disclosure, a typical daily dosage might range from about any of 0.1 $\mu$g/kg to 3 $\mu$g /kg to 30 $\mu$g /kg to 300 $\mu$g /kg to 3 mg/kg, to 30 mg/kg to 100 mg/kg or more, depending on the factors mentioned above. For repeated administrations over several days or longer, depending on the condition, the treatment is sustained until a desired suppression or amelioration of symptoms occurs or until sufficient therapeutic levels are achieved to alleviate a cancer, or one or more symptoms thereof. An exemplary dosing regimen comprises administering an initial dose of about 2 mg/kg, followed by a weekly maintenance dose of about 1 mg/kg of the antibody, or followed by a maintenance dose of about 1 mg/kg every other week. However, other dosage regimens may be useful, depending on the pattern of pharmacokinetic decay that the practitioner (e.g., a medical doctor) wishes to achieve. For example, dosing from one-four times a week is contemplated. In some embodiments, dosing ranging from about 3 $\mu$g /mg to about 2 mg/kg (such as about 3 $\mu$g /mg, about 10 $\mu$g /mg, about 30 $\mu$g /mg, about 100 $\mu$g /mg, about 300 $\mu$g /mg, about 1 mg/kg, and about 2 mg/kg) may be used. In some embodiments, dosing frequency is once every week, every 2 weeks, every 4 weeks, every 5 weeks, every 6 weeks, every 7 weeks, every 8 weeks, every 9 weeks, or every 10 weeks; or once every month, every 2 months, or every 3 months, or longer. The progress of this therapy may be monitored by conventional techniques and assays and/or by monitoring TME types as described herein. The dosing regimen (including the therapeutic used) may vary over time.

Dosing of immuno-oncology agents is well-known, for example as described by Louedec et al. Vaccines (Basel). 2020 Dec; 8(4): 632. For example, dosages of pembrolizumab, for example, include administration of 200 mg every 3 weeks or 400 mg every 6 weeks, by infusion over 30 minutes.

Dosing of TKIs is also well-known, for example as described by Gerritse et al. Cancer Treat Rev. 2021 Jun;97:102171. doi: 10.1016/j.ctrv.2021.102171. Combination dosing of TKIs and IO agents is also known, for example as described by Rassy et al. Ther Adv Med Oncol. 2020; 12: 1758835920907504.

When the anti-cancer therapeutic agent is not an antibody, it may be administered at the rate of about 0.1 to 300 mg/kg of the weight of the patient divided into one to three doses, or as disclosed herein. In some embodiments not part of the claims, for an adult patient of normal weight, doses ranging from about 0.3 to 5.00 mg/kg may be administered. The particular dosage regimen, e.g., dose, timing, and/or repetition, will depend on the particular subject and that individual's medical history, as well as the properties of the individual agents (such as the half-life of the agent, and other considerations well known in the art).

For the purpose of the present disclosure, the appropriate dosage of an anti-cancer therapeutic agent will depend on the specific anti-cancer therapeutic agent(s) (or compositions thereof) employed, the type and severity of cancer, whether the anti-cancer therapeutic agent is administered for preventive or therapeutic purposes, previous therapy, the patient's clinical history and response to the anti-cancer therapeutic agent, and the discretion of the attending physician. Typically, the clinician will administer an anti-cancer therapeutic agent, such as an antibody, until a dosage is reached that achieves the desired result.

Administration of an anti-cancer therapeutic agent can be continuous or intermittent, depending, for example, upon the recipient's physiological condition, whether the purpose of the administration is therapeutic or prophylactic, and other factors known to skilled practitioners. The administration of an anti-cancer therapeutic agent (e.g., an anti-cancer antibody) may be essentially continuous over a preselected period of time or may be in a series of spaced dose, e.g., either before, during, or after developing cancer.

As used herein, the term "treating" refers to the application or administration of a composition including one or more active agents to a subject, who has a cancer, a symptom of a cancer, or a predisposition toward a cancer, with the purpose to cure, heal, alleviate, relieve, alter, remedy, ameliorate, improve, or affect the cancer or one or more symptoms of breast cancer, or the predisposition toward breast cancer.

Alleviating breast cancer includes delaying the development or progression of the disease, or reducing disease severity. Alleviating the disease does not necessarily require curative results. As used therein, "delaying" the development of a disease (e.g., a cancer) means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given time frame and/or reduces extent of the symptoms in a given time frame, when compared to not using the method. Such comparisons are typically based on clinical studies, using a number of subjects sufficient to give a statistically significant result.

"Development" or "progression" of a disease means initial manifestations and/or ensuing progression of the disease. Development of the disease can be detected and assessed using clinical techniques known in the art. Alternatively, or in addition to the clinical techniques known in the art, development of the disease may be detectable and assessed based on other criteria. However, development also refers to progression that may be undetectable. For purpose of this disclosure, development or progression refers to the biological course of the symptoms. "Development" includes occurrence, recurrence, and onset. As used herein "onset" or "occurrence" of a cancer includes initial onset and/or recurrence.

Examples of the antibody anti-cancer agents include, but are not limited to, alemtuzumab (Campath), trastuzumab (Herceptin), Ibritumomab tiuxetan (Zevalin), Brentuximab vedotin (Adcetris), Ado-trastuzumab emtansine (Kadcyla), blinatumomab (Blincyto), Bevacizumab (Avastin), Cetuximab (Erbitux), ipilimumab (Yervoy), nivolumab (Opdivo), pembrolizumab (Keytruda), atezolizumab (Tecentriq), avelumab (Bavencio), durvalumab (Imfinzi), and panitumumab (Vectibix).

Examples of an immunotherapy include, but are not limited to, a PD-1 inhibitor or a PD-L1 inhibitor, a CTLA-4 inhibitor, adoptive cell transfer, therapeutic cancer vaccines, oncolytic virus therapy, T-cell therapy, and immune checkpoint inhibitors.

Examples of radiation therapy include, but are not limited to, ionizing radiation, gamma-radiation, neutron beam radiotherapy, electron beam radiotherapy, proton therapy, brachytherapy, systemic radioactive isotopes, and radiosensitizers.

Examples of a surgical therapy include, but are not limited to, a curative surgery (e.g., tumor removal surgery), a preventive surgery, a laparoscopic surgery, and a laser surgery.

Examples of the chemotherapeutic agents include, but are not limited to, R-CHOP, Carboplatin or Cisplatin, Docetaxel, Gemcitabine, Nab-Paclitaxel, Paclitaxel, Pemetrexed, and Vinorelbine. Additional examples of chemotherapy include, but are not limited to, Platinating agents, such as Carboplatin, Oxaliplatin, Cisplatin, Nedaplatin, Satraplatin, Lobaplatin, Triplatin, Tetranitrate, Picoplatin, Prolindac, Aroplatin and other derivatives; Topoisomerase I inhibitors, such as Camptothecin, Topotecan, irinotecan/SN38, rubitecan, Belotecan, and other derivatives; Topoisomerase II inhibitors, such as Etoposide (VP-16), Daunorubicin, a doxorubicin agent (e.g., doxorubicin, doxorubicin hydrochloride, doxorubicin analogs, or doxorubicin and salts or analogs thereof in liposomes), Mitoxantrone, Aclarubicin, Epirubicin, Idarubicin, Amrubicin, Amsacrine, Pirarubicin, Valrubicin, Zorubicin, Teniposide and other derivatives; Antimetabolites, such as Folic family (Methotrexate, Pemetrexed, Raltitrexed, Aminopterin, and relatives or derivatives thereof); Purine antagonists (Thioguanine, Fludarabine, Cladribine, 6-Mercaptopurine, Pentostatin, clofarabine, and relatives or derivatives thereof) and Pyrimidine antagonists (Cytarabine, Floxuridine, Azacitidine, Tegafur, Carmofur, Capacitabine, Gemcitabine, hydroxyurea, 5-Fluorouracil (5FU), and relatives or derivatives thereof); Alkylating agents, such as Nitrogen mustards (e.g., Cyclophosphamide, Melphalan, Chlorambucil, mechlorethamine, Ifosfamide, mechlorethamine, Trofosfamide, Prednimustine, Bendamustine, Uramustine, Estramustine, and relatives or derivatives thereof); nitrosoureas (e.g., Carmustine, Lomustine, Semustine, Fotemustine, Nimustine, Ranimustine, Streptozocin, and relatives or derivatives thereof); Triazenes (e.g., Dacarbazine, Altretamine, Temozolomide, and relatives or derivatives thereof); Alkyl sulphonates (e.g., Busulfan, Mannosulfan, Treosulfan, and relatives or derivatives thereof); Procarbazine; Mitobronitol, and Aziridines (e.g., Carboquone, Triaziquone, ThioTEPA, triethylenemalamine, and relatives or derivatives thereof); Antibiotics, such as Hydroxyurea, Anthracyclines (e.g., doxorubicin agent, daunorubicin, epirubicin and relatives or derivatives thereof); Anthracenediones (e.g., Mitoxantrone and relatives or derivatives thereof); Streptomyces family antibiotics (e.g., Bleomycin, Mitomycin C, Actinomycin, and Plicamycin); and ultraviolet light.

In some aspects, the disclosure provides a method for treating breast cancer (BC), the method comprising administering one or more therapeutic agents (e.g., one or more anti-cancer agents, such as one or more immunotherapeutic agents) to a subject identified as having a particular breast cancer TME type, wherein the breast cancer TME type of the subject has been identified by method as described by the disclosure.

*Reports*

In some embodiments, methods disclosed herein comprise generating a report for assisting with the preparation of recommendation for prognosis and/or treatment. The generated report can provide summary of information, so that the clinician can identify the RC TME type or suitable therapy. The report as described herein may be a paper report, an electronic record, or a report in any format that is deemed suitable in the art. The report may be shown and/or stored on a computing device known in the art (e.g., handheld device, desktop computer, smart device, website, etc.). The report may be shown and/or stored on any device that is suitable as understood by a skilled person in the art.

In some embodiments, methods disclosed herein can be used for commercial diagnostic purposes. For example, the generated report may include, but is limited to, information concerning expression levels of one or more genes from any of the gene groups described herein, clinical and pathologic factors, patient's prognostic analysis, predicted response to the treatment, classification of the RC TME environment (e.g., as belonging to one of the types described herein), the alternative treatment recommendation, and/or other information. In some embodiments, the methods and reports may include database management for the keeping of the generated reports. For instance, the methods as disclosed herein can create a record in a database for the subject (e.g., subject 1, subject 2, etc.) and populate the specific record with data for the subject. In some embodiments, the generated report can be provided to the subject and/or to the clinicians. In some embodiments, a network connection can be established to a server computer that includes the data and report for receiving or outputting. In some embodiments, the receiving and outputting of the date or report can be requested from the server computer.

*Computer Implementation*

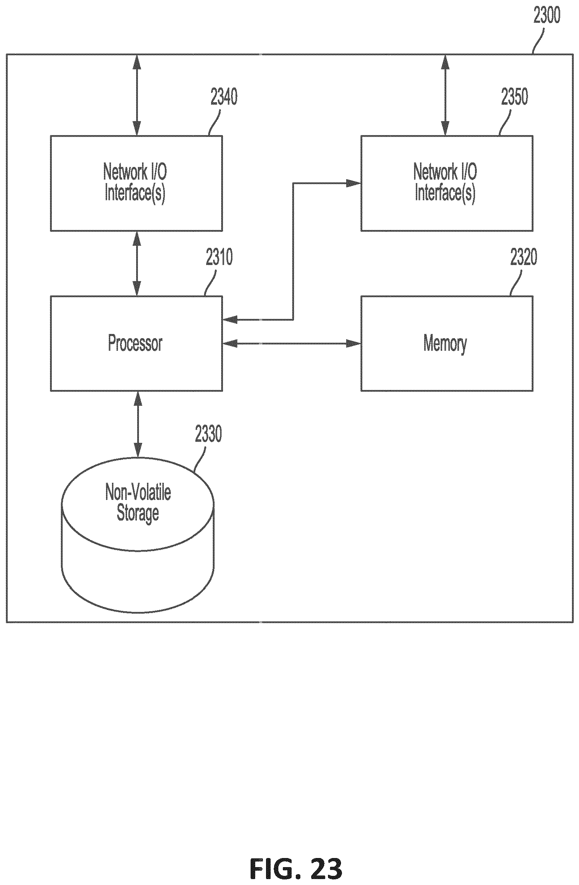

An illustrative implementation of a computer system 2300 that may be used in connection with any of the embodiments of the technology described herein (e.g., such as the method of FIG. 1, FIG. 2, or FIG. 3) is shown in FIG. 23. The computer system 2300 includes one or more processors 2310 and one or more articles of manufacture that comprise non-transitory computer-readable storage media (e.g., memory 2320 and one or more non-volatile storage media 2330). The processor 2310 may control writing data to and reading data from the memory 2320 and the non-volatile storage device 2330 in any suitable manner, as the aspects of the technology described herein are not limited to any particular techniques for writing or reading data. To perform any of the functionality described herein, the processor 2310 may execute one or more processor-executable instructions stored in one or more non-transitory computer-readable storage media (e.g., the memory 2320), which may serve as non-transitory computer-readable storage media storing processor-executable instructions for execution by the processor 2310.

Computing device 2300 may also include a network input/output (I/O) interface 2340 via which the computing device may communicate with other computing devices (e.g., over a network), and may also include one or more user I/O interfaces 2350, via which the computing device may provide output to and receive input from a user. The user I/O interfaces may include devices such as a keyboard, a mouse, a microphone, a display device (e.g., a monitor or touch screen), speakers, a camera, and/or various other types of I/O devices.

The above-described embodiments can be implemented in any of numerous ways. For example, the embodiments may be implemented using hardware, software, or a combination thereof. When implemented in software, the software code can be executed on any suitable processor (e.g., a microprocessor) or collection of processors, whether provided in a single computing device or distributed among multiple computing devices. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the embodiments described herein comprises at least one computer-readable storage medium (e.g., RAM, ROM, EEPROM, flash memory or other memory technology, CD-ROM, digital versatile disks (DVD) or other optical disk storage, magnetic cassettes, magnetic tape, magnetic disk storage or other magnetic storage devices, or other tangible, non-transitory computer-readable storage medium) encoded with a computer program (i.e., a plurality of executable instructions) that, when executed on one or more processors, performs the above-discussed functions of one or more embodiments. The computer-readable medium may be transportable such that the program stored thereon can be loaded onto any computing device to implement aspects of the techniques discussed herein. In addition, it should be appreciated that the reference to a computer program which, when executed, performs any of the above-discussed functions, is not limited to an application program running on a host computer. Rather, the terms computer program and software are used herein in a generic sense to reference any type of computer code (e.g., application software, firmware, microcode, or any other form of computer instruction) that can be employed to program one or more processors to implement aspects of the techniques discussed herein.

The foregoing description of implementations provides illustration and description but is not intended to be exhaustive or to limit the implementations to the precise form disclosed. Modifications and variations are possible in light of the above teachings or may be acquired from practice of the implementations. In other implementations the methods depicted in these figures may include fewer operations, different operations, differently ordered operations, and/or additional operations. Further, non-dependent blocks may be performed in parallel.

It will be apparent that example aspects, as described above, may be implemented in many different forms of software, firmware, and hardware in the implementations illustrated in the figures. Further, certain portions of the implementations may be implemented as a "module" that performs one or more functions. This module may include hardware, such as a processor, an application-specific integrated circuit (ASIC), or a field-programmable gate array (FPGA), or a combination of hardware and software.

Having thus described several aspects and embodiments of the technology set forth in the disclosure, it is to be appreciated that various alterations, modifications, and improvements will readily occur to those skilled in the art. For example, those of ordinary skill in the art will readily envision a variety of other means and/or structures for performing the function and/or obtaining the results and/or one or more of the advantages described herein, and each of such variations and/or modifications is deemed to be within the scope of the embodiments described herein. It is, therefore, to be understood that the foregoing embodiments are presented by way of example only and that. In addition, any combination of two or more features, systems, articles, materials, kits, and/or methods described herein, if such features, systems, articles, materials, kits, and/or methods are not mutually inconsistent, is included within the scope of the present disclosure.

The above-described embodiments can be implemented in any of numerous ways. One or more aspects and embodiments of the present disclosure involving the performance of processes or methods may utilize program instructions executable by a device (e.g., a computer, a processor, or other device) to perform, or control performance of, the processes or methods. In this respect, various inventive concepts may be embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other tangible computer storage medium) encoded with one or more programs that, when executed on one or more computers or other processors, perform methods that implement one or more of the various embodiments described above. The computer readable medium or media can be transportable, such that the program or programs stored thereon can be loaded onto one or more different computers or other processors to implement various ones of the aspects described above. In some embodiments, computer readable media may be non-transitory media.

The terms "program" or "software" are used herein in a generic sense to refer to any type of computer code or set of computer-executable instructions that can be employed to program a computer or other processor to implement various aspects as described above. Additionally, it should be appreciated that according to one aspect, one or more computer programs that when executed perform methods of the present disclosure need not reside on a single computer or processor, but may be distributed in a modular fashion among a number of different computers or processors to implement various aspects of the present disclosure.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

When implemented in software, the software code can be executed on any suitable processor or collection of processors, whether provided in a single computer or distributed among multiple computers.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone, a tablet, or any other suitable portable or fixed electronic device.

Also, a computer may have one or more input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible formats.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Table 7: Exemplary NCBI Accession Numbers for genes listed in Tables 1, 2, and 3

| | |
|---|---|
| HLA-C | NM_002117; NM_001243042 |
| TAPBP | XM_017011227; XM_047419272; NM_001410875; XM_047419271; XM 011514828; NM 003190; NM 172208; NM 172209 |
| HLA-B | NM_005514 |
| B2M | NM_004048; XM_005254549 |
| TAP2 | NM_001290043; NM_000544; NM_018833 |
| HLA-A | XM_041680767; NM_001242758; XM_041680768; NM_002116 |
| TAP1 | NM_000593; NM_001292022 |
| NLRC5 | NM_001330552; NM_001384961; NM_001384969; NM_001384973; NM_001384972; NR_169518; NM_001384951; NM_001384959; NM_032206; NR_169513; XR_001752000; XM_006721300; XM_017023770; XM_047434760; XM_047434765; NM_001384950; NM_001384958; NM_001384964; NR_169520; NM_001384954; NM_001384966; NR_169514; NR_169517; XM_047434761; XM_047434763; NM_001384967; NM_001384971; XM_047434766; NM_001384965; NM_001384970; NR_169512; NR_169519; XM_047434762; XM_047434764; NM_001384953; NM_001384956; NM_001384957; NM_001384962; NM_001384963; NR_169515; NR_169516; NM_001384952; NM_001384955; NM_001384960; NM_001384968 |
| HLA-DQB1 | NM_001243962; NM_001243961; NM_002123 |
| HLA-DMA | NM_006120 |
| HLA-DMB | NM_002118 |
| HLA-DRA | NM_019111 |
| CIITA | XM_047434115; NM_001379332; XR_007064880; XM_006720880; XM_011522491; XM_047434119; NM_001379334; XM_047434118; XM_047434120; XM_047434123; NM_001379333; XM_011522486; NM_000246; NM_001286402; XM_047434122; XM_047434126; XR 001751904; XR 007064879; XM 047434114; XM 047434117; XM_047434125; NM_001286403; NM_001379331; XM_011522485; XM_047434127; XM_047434128; NR_104444; XM_011522484; XM 011522490; XM 047434116; XM 047434124; NM_001379330 |
| BLA-DQA1 | NM_002122; XM_006715079 |

(continued)

| | |
|---|---|
| HLA-DPB 1 | NM_002121 |
| HLA-DRB1 | XM_024452553; NM_001359194; XM_047444767; XM_047444769; NM_002124; XM_047444770; NM_001359193; XM 047443024; XM 047444768 |
| HLA-DPA1 | NM_001405020; NM_001242525; NM_033554; XM_047418717; NM_001242524 |
| TNFRSF4 | XM_011542074; NM_003327; XR_007063145; XM_011542077; NM_001410709; XM 011542075; XM 011542076 |
| CD27 | NM_001242; XM_011521042; XM_017020234; XM_047429900 |
| CD80 | NM_005191 |
| CD40LG | NM_000074 |
| TNFRSF9 | NM_001561; XM_047419672; XM_006710618 |
| CD40 | NM_001302753; NM_001322422; NM_152854; NM_001322421; XM_011529109; XM_005260619; XM_017028135; XM_017028136; NR 126502 |
| CD28 | NM_001243078; NM_001410981; NM_001243077; XM 011512194 |
| ICOSLG | NM_001395918; XM_011529514; NM_001283050; NM_001283051; NM_001283052; XM_047440732; NM_015259; XM_047440731; NM_001365759; XM 011529516; XM 047440729; XM 047440730 |
| CD83 | NM_001040280; NM_001251901; NM_004233 |
| TNFSF9 | NM_003811 |
| CD70 | NM_001252; NM_001330332 |
| TNFSF4 | XM_047429908; NM_003326; XM_047429896; NM_001297562; XM 047429902; XM 017002228; XM 011509964 |
| ICOS | XR_007073112; XM_047444022; NM_012092 |
| CD86 | NM_001206924; NM_006889; NM_176892; NM_001206925; NM_175862 |
| ZAP70 | NM_001378594; NM_207519; XR_007081582; NM_001079; XM 047445775; XM 047445774; XM 047445776; XR 007081583 |
| GZMB | NM_001346011; NM_004131; NR 144343 |
| GZMK | NM_002104 |
| IFNG | NM_000619 |
| FASLG | NM_001302746; NM_000639 |
| EOMES | NM_001278182; XM_005265510; NM_005442; NM_001278183 |
| TBX21 | NM_013351 |
| GZMA | NM_006144 |
| CD8A | NM_001382698; NM_001145873; NM_001768; NR_168478; NR_168479; NM_171827; NR 168480; NR 168481; NR 027353 |
| GNLY | XM_005264085; NM_001302758; XM_047442947; NM_006433; XM 005264084; NM_012483 |
| PRF1 | NM_005041; NM_001083116 |
| CD8B | NM_172102; NM_172100; NM_001178100; NM_004931; NM_172101; NM_172213; NM_172099; XM 011533164 |
| NKG7 | XM_006723228; XM_005258955; NM_001363693; NM_005601 |
| FGFBP2 | NM_031950 |
| CD244 | NM_001166663; XM_047422535; XM_011509622; NM_016382; NM_001166664; XM_011509623; XM_011509621 |
| KLRK1 | NM_007360.4 |

(continued)

| KIR2DL4 | NM_001080770; NM_001080772; NM_002255; NM_001258383 |
|---|---|
| CD226 | NM_006566; XM_047437274; NM_001303619; XM_047437275; XM_047437276; XM_006722374; XM_005266642; XM_047437277; NM_001303618 |
| KLRF 1 | NM_001291822; XM_017019415; XM_047428956; NM_001366534; NR_120305; NM_001291823; NM_016523; NR_159359; NR_159360; NR 159361 |
| KLRC2 | NM_002260 |
| NCR1 | NM_004829; NM_001145457; XM_011527530; XM_047439727; NM_001242357; XM_011527529; NM_001242356; NM_001145458 |
| GZMH | NM_001270781; NM_001270780; NM_033423 |
| SH2D1B | NM_053282 |
| NCR3 | NM_001145467; XM_011514459; XM_006715049; NM_001145466; NM_147130 |
| CD160 | NM_007053; XM_005272929; XM_011509104; NR_103845 |
| TRBC2 | NG 001333.2 |
| CD3E | NM_000733 |
| CD3G | XM_005271724; XM_006718941; NM_000073 |
| ITK | NM_005546 |
| TRBC1 | NG 001333.2 |
| TRAT1 | NM_016388; NM_001317747 |
| CD5 | NM_014207; NM_001346456 |
| TRAC | NG_001332.3 |
| CD3D | NM_001040651; NM_000732 |
| CD22 | NM_001185100; NM_001185099; NM_024916; NM_001185101; NM_001771; NM_001278417 |
| TNFRSF13C | NM_052945 |
| STAP1 | NM_012108; NM_001317769 |
| CD79B | NM_001039933; NM_021602; NM_000626; NM_001329050 |
| PAX5 | NM_001280547; NM_001280553; NM_016734; NM_001280548; NR_103999; NM_001280551; NM_001280555; NM_001280554; NM_001280552; NM_001280556; NM_001280549; NM_001280550; NR 104000 |
| CR2 | NM_001877; NM_001006658 |
| TNFRSF13B | NM_012452 |
| CD79A | NM_021601; NM_001783 |
| TNFRSF17 | NM_001192 |
| FCRL5 | XM_011510032; XM_047431422; XM_011510030; XM_011510033; XM_011510031; NM_031281; NM_001195388 |
| MS4A1 | NM_021950; NM_152866; NM_152867 |
| CD19 | NM_001178098; NM_001385732; NM_001770; XR_950871; NR_169755; XM 011545981 |
| BLK | XM_047422081; NM_001330465; XM_011543829; XM_011543824; XM_011543827; XM_047422083; XM_047422084; XM_011543828; XM_047422082; NM_001715; XM_011543825 |
| CMKLR1 | NM_001142343; XM_047428313; NM_001142345; NM_001142344; NM_004072 |
| SOCS3 | NM_001378933; NM_003955; NM_001378932 |

(continued)

| IRF5 | XM_047420336; NM_001242452; XM_006715974; NM_001364314; NM_032643; XM_011516160; XM_011516158; XM_047420340; NM_001347928; XM_047420337; NM_001098629; XM_011516159; XM_047420338; NM_001098627; NM_001098630; XM_047420339 |
|---|---|
| NOS2 | NM_153292; NM_000625 |
| IL1B | NM_000576; XM_047444175 |
| IL12B | NM_002187 |
| IL23A | NM_016584 |
| TNF | NM_000594 |
| IL12A | NM_000882; NM_001354583; NM_001354582; NM_001397992 |
| IL21 | NM_021803; NM_001207006 |
| IL12RB2 | NR_047584; XM_011541384; XM_047419669; XM_047419670; XM_005270827; XM_006710617; NM_001374259; XM_011541383; XM_047419667; NM_001258215; NM_001258216; XM_047419665; XM_047419666; XM_047419668; NM_001258214; NM_001319233; XM_005270828; XM_017001203; NM_001559; NR_047583 |
| IL2 | NM_000586 |
| STAT4 | XM_047445601; XM_047445609; XM_047445602; XM_047445604; XM_047445607; NM_001243835; XM_047445603; XM_047445608; XM 047445600 |
| CCL3 | NR_168496; NR_168495; NM_002983; NR_168494 |
| IFNB 1 | NM_002176 |
| IFNA2 | NM_000605 |
| TNFSF10 | NR_033994; NM_001190943; NM_003810; NM_001190942 |
| PDCD1 | NM_005018; XM_006712573 |
| BTLA | NM_001085357; NM_181780; XM_011512447; XM_017005748; XM_047447496 |
| HAVCR2 | NM_032782 |
| CD274 | XM_047423262; NM_001314029; NM_001267706; NR_052005; NM_014143 |
| VSIR | NM_022153 |
| LAG3 | NM_002286; XM_047428839; XM_011520956 |
| TIGIT | XM_047447672; XM_047447671; NM_173799 |
| PDCD1LG2 | XM_005251600; NM_025239 |
| CTLA4 | NM_005214; NM_001037631 |
| IKZF2 | XM_011510804; XM_011510815; NM_001371277; XM_011510803; XM_011510808; XM_011510816; NM_001371275; XM_005246386; XM_011510807; XM_011510810; XM_047443723; XM_011510811; NM_001371276; XM_011510809; XM_047443724; XM_047443722; NM_001079526; NM_001371274; NM_001387220; NM_016260; XM 011510817; XM_011510818; XM 011510805; XM 011510812; XM_011510819; XM_047443721; XM_047443725; XM_047443727; XM_011510802; XM_047443726 |
| TNFRSF18 | NM_148901; NM_004195; XM_017002722; NM_148902 |
| IL10 | NM_000572; NR_168467; NR_168466; NM_001382624 |
| FOXP3 | NM_001114377; NM_014009 |
| CCR8 | NM_005201 |

(continued)

| | |
|---|---|
| IKZF4 | XM_017019810; XM_047429342; XM_047429345; XM_047429347; NM_001351089; NM_022465; XM_047429341; XM_047429346; XM_047429351; XM_047429349; NM_001351090; NM_001351091; XM_011538664; XM_011538669; XM_047429350; XM_047429352; XM_047429353; XM_017019806; XM_047429348; XM_005269089; XM_047429344; NM_001351092; XM_017019812 |
| CD177 | XM_017027021; XM_017027022; NM_020406 |
| FFAR2 | XM_047438699; NM_005306; NM_001370087; XM_017026711; XM 047438700 |
| PGLYRP1 | NM_005091 |
| CXCR1 | NM_000634 |
| MPO | NM_000250 |
| CXCR2 | XM_047444190; XM_047444188; NM_001168298; NM_001557; XM_005246530; XM_047444189; XM_017003991; XM_047444191; XM 047444187 |
| ELANE | NM_001972 |
| CTSG | NM_001911; XM_011536499 |
| PRTN3 | XM_011528136; NM_002777 |
| FCGR3B | NM_001271036; NM_001271037; NM_001244753; NM_000570; NM_001271035 |
| CCR3 | NM_001164680; NM_001837; NM_178328; NM_178329; XM_017005685; XM 006712960 |
| KITLG | NM_003994; NM_000899 |
| CCL11 | NM_002986 |
| CXCL2 | NM_002089 |
| CXCL8 | NM_000584; NM_001354840 |
| CXCL1 | NM_001511; NR_046035 |
| CXCL5 | NM_002994 |
| ARG1 | NM_001369020; NM_000045; NM_001244438; NR 160934 |
| IL6 | NM_001318095; NM_000600; NM_001371096; XM_005249745 |
| CYBB | XM_047441855; NM_000397 |
| PTGS2 | NM_000963 |
| IDO1 | NM_002164 |
| IL411 | NM_001385639; NM_172374; NM_152899; NR_047577; NM_001258018; NM_001258017 |
| MRC1 | NM_002438; NM_001009567 |
| SIGLEC1 | NM_001367089; NM_023068 |
| MSR1 | NM_138716; NM_002445; XM_024447161; NM_138715; NM_001363744 |
| CD163 | XM_047429895; XM_024449278; NM_203416; NM_001370145; NM_001370146; NM_004244; NR 163255 |
| CSF1R | NM_001375320; NM_005211; NR_164679; NM_001349736; NM_001288705; NM_001375321; NR 109969 |
| CD68 | NM_001251; NM_001040059 |
| IL13 | NM_001354991; NM_001354992; NM_002188; NM_001354993 |
| CCR4 | XM_017005687; NM_005508 |
| IL4 | NM_000589; NM_001354990; NM_172348 |
| IL5 | XM_047417148; XM_005271988; XM_011543373; NM_000879 |
| TGFB2 | NM_003238; NR_138149; NR_138148; NM 001135599 |

(continued)

| | |
|---|---|
| MIF | NM_002415 |
| TGFB3 | NM_001329938; NM_003239; NM_001329939 |
| TGFB1 | NM_000660; XM_011527242 |
| IL22 | NM_020525 |
| COL6A3 | NM_057164; NM_057167; NM_057166; NM_004369; NM_057165 |
| PDGFRB | NM_001355016; NM_002609; NM_001355017; NR_149150 |
| COL6A1 | NM_001848 |
| MFAP5 | NM_001297709; NR_123733; NR_123734; NM_001297711; NM_003480; NM_001297710; NM_001297712 |
| COL5A1 | NM_000093; XM_017014266; NM_001278074 |
| FAP | XM_011510797; NM_004460; XM_011510796; XM_017003585; XR_001738668; XR_922891; NM_001291807 |
| PDGFRA | XM_047415767; NM_001347828; NM_001347829; XM_005265743; XM_017008281; NM_001347827; XM_047415766; NM_001347830; NM_006206; XM_006714041 |
| FGF2 | NM_001361665; NM_002006 |
| ACTA2 | NM_001406467; NM_001141945; NM_001406463; NM_001406464; NM_001406471; NM_001406466; NM_001406462; NM_001320855; NM_001613; NM_001406468, NM_001406469 |
| COL6A2 | NM_001849; NM_058175; NM_058174 |
| FBLN1 | NM_006486; NM_006485; NM_001996; NM_006487 |
| CD248 | NM_020404 |
| COL1A1 | XM_005257058; XM_005257059; XM_011524341; NM_000088 |
| MMP2 | NM_001302509; NM_001127891; NM_001302508; NM_001302510; NM_004530 |
| COL1A2 | NM_000089 |
| MMP3 | NM_002422 |
| LUM | NM_002345 |
| CXCL12 | NM_000609; NM_001277990; NM_199168; NM_001178134; NM_001033886 |
| LRP1 | NM_002332 |
| LAMC2 | NM_005562; NM_018891; XM_047420361; XM_047420358; XM 017001273 |
| TNC | XM_005251975; XM_006717096; XM_011518628; XM_017014681; XM_047423311; XM_047423321; XM_047423323; XM_047423328; XM_011518626; XM_047423312; XM_047423313; XM_047423317; XM_047423318; XM_005251973; XM_006717098; XM_047423322; XM_047423324; XM_047423327; XM_006717097; XM_005251972; XM_011518629; XM_047423309; XM_047423314; XM_047423325; NM_001410991; XM_017014680; XM_047423315; XM_047423329; XM_011518625; XM_017014679; XM_047423310; XM_047423320; XM 047423330; XM 047423331; XM 005251974; XM 006717101; XM_047423316; XM_047423319; XM_047423326; XM_047423332; XM_017014678; XM_024447530; NM_002160 |
| COL11A1 | XM_017000337; XM_017000335; XM_017000336; NR_134980; NM_080629; XR_007085257; XM_017000334; NM_001854; NM_001190709; NM_080630 |
| VTN | NM_000638 |
| LAMB3 | XM_005273124; XM_047420351; NM_001127641; XM_017001272; NM_000228; NM_001017402 |

(continued)

| FN1 | NM_001306129; NM_001365519; NM_212474; NM_001306132; NM_001365517; NM_001365522; NM_001306131; NM_001365521; NM_212476; NM_212478; NM_212475; NM_001365523; NM_002026; NM_001365524; NM_001365520; NM_212482; NM_001365518; NM_054034; NM_001306130 |
|---|---|
| LAMA3 | XM_011525981; XM_017025743; XM_047437504; NM_001127717; NM_198129; XM_011525980; XM_017025744; XM_047437506; XM_011525982; XM_047437505; NM_001302996; NR_130106; NM_001127718 |
| LGALS9 | XM_011524796; NM_001330163; NR_024043; XM_006721893; XM_006721895; NM_002308; XM_006721892; NM_009587 |
| COL4A1 | NM_001845; NM_001303110 |
| ELN | XM_011515869; XM_011515873; XM_017011814; XM_047419961; XM_047419973; XM_047419978; XM_005250187; XM_011515871; XM_011515872; XM_047419958; XM_047419962; XM_047419963; XM_047419965; NM_001278914; XM_005250188; XM_011515874; XM_047419957; XM_047419964; XM_047419966; XM_047419974; XM_047419979; NM_000501; NM_001278912; NM_001278939; XM_011515877; XM_047419960; XM_047419967; XM_047419971; XM_047419977; NM_001081753; XM_011515876; XM_047419955; XM_047419970; XM_047419975; NM_001081754; NM_001278917; XM_017011813; XM_047419954; XM_047419956; XM_047419968; XM_047419969; XM_047419972; XM_047419980; NM_001278915; NM_001278918; XM_011515868; XM_011515870; XM_047419981; NM_001081755; NM_001278916; XM_011515875; XM_047419959; XM_047419976; NM_001081752; NM_001278913 |
| LGALS7 | NM_002307.4 |
| COL3A1 | NM_000090; NM_001376916 |
| VEGFC | NM_005429 |
| VEGFA | NM_003376; NM_001033756; NM_001171624; NM_001025368; NM_001025370; NM_001171623; NM_001171622; NM_001025369; NM_001171627; NM_001204384; NM_001287044 |
| PDGFC | XM_047415970; XM_017008455; NM_016205; XM_047415971; XM_047415969; XM_047415972; NR_036641 |
| KDR | NM_002253 |
| CDH5 | XM_047433469; XM_047433470; NM_001114117; NM_001795; XM_047433471; XM_011522801 |
| VEGFB | NM_003377; NM_001243733 |
| PGF | NM_001293643; NM_002632; NM_001207012; XM_047431476 |
| TEK | NM_000459; NM_001290077; NM_001290078; NM_001375476 |
| ANGPT2 | NM_001118888; NM_001386335; NM_001386337; NM_001118887; NM_001147; NM_001386336 |
| FLT1 | NM_001160030; NM_001159920; XM_011535014; XM_017020485; NM_001160031; NM_002019 |
| VWF | NM_000552; XM_047429501 |
| ANGPT1 | NM_001314051; NM_001146; NM_001199859; XM_047421699; NM_139290 |
| NOS3 | NM_001160110; NM_000603; NM_001160109; NM_001160111 |
| VCAM1 | NM_080682; NM_001078; NM_001199834 |
| MMRN1 | NM_001410735; NM_001371403; XM_047449832; XM_047449831; NM_007351 |
| CLEC14A | NM_175060 |
| ENG | NM_000118; NM_001406715; NM_001114753; NM_001278138 |
| MMRN2 | NM_024756 |

(continued)

| | |
|---|---|
| CCND1 | NM_053056 |
| CCNB1 | NM_031966; NM_001354845 |
| CETN3 | NM_004365; NM_001297765; NM_001297768 |
| CDK2 | NM_001290230; XM_011537732; NM_052827; NM_001798 |
| E2F1 | XM_047439961; NM_005225 |
| AURKA | XM_047440428; XM_047440427; NM_001323304; NM_001323303; NM_198435; NM_198437; NM_198433; NM_198434; NM_198436; XM_017028034; XM_017028035; NM_003600; NM_001323305 |
| BUB1 | NM_004336; NM_001278617; XM_047445616; NM_001278616 |
| AURKB | NM_001313950; NM_001313953; XM_017025311; XM_047437050; NM_001313952; NM_004217; NM_001313954; NR_132730; NR_132731; NM_001284526; XM_047437051; XM_011524072; NM_001256834; NM_001313951; NM_001313955 |
| PLK1 | NM_005030 |
| MCM6 | NM_005915 |
| ESCO2 | NM_001017420; XR_949378; XR_007060703; XM_011544422; XM_011544421 |
| MYBL2 | NM_002466; NM_001278610 |
| MKI67 | NM_002417; NM_001145966; XM_006717864; XM_011539818 |
| MCM2 | NM_004526; XM_024453531; NR 073375 |
| CCNE1 | NM_001238; XM_011527440; NM_001322259; NM_001322261; XM_047439606; NM_001322262; NM_057182 |
| CDH2 | XM_011525788; NM_001308176; XM_017025514; NM_001792 |
| ZEB1 | XM_047425691; NM_001174096; NM_001323650; NM_001323655; NM_001323660; NM_001323677; XM_047425683; XM_047425688; NM_001174095; NM_001323638; NM_001323645; NM_001323647; NM_001323663; NM_001323666; NM_001323672; NM_030751; XM_047425684; XM_047425686; NM_001323662; XM_047425685; XM_047425682; XM_047425689; XM_047425693; XM_047425695; NM_001128128; NM_001174094; NM_001323641; NM_001323644; NM_001323659; NM_001323674; NM_001323675; NM_001323676; XM_047425687; NM_001323642; NM_001323648; NM_001323649; NM_001323652; NM_001323654; NM_001323671; XM 047425690; |
| NM_001323656; NM_001323661; NM_001323664; NM_001323673; | NM_001323656; NM_001323661; NM_001323664; NM_001323673; NM_001174093; NM_001323646; NM_001323653; NM_001323657 |
| ZEB2 | NM_001171653; NM_014795; NR_033258 |
| TWIST1 | NR_149001; NM_000474 |
| SNAI1 | NM_005985 |
| SNAI2 | NM_003068 |
| TWIST2 | NM_057179; XR 007069137; NM_001271893 |
| CXCL13 | NM_001371558; NM_006419 |
| CCL19 | NM_006274 |
| LAMP3 | XM_011512688; XM_047447967; XM_005247360; NM_014398 |
| SELL | NR 029467; NM_000655 |
| CXCR4 | NM_001348059; NM_001348060; XM_047445802; NM_001348056; NM_003467; NM_001008540 |
| CCL21 | NM_002989 |

(continued)

| | |
|---|---|
| CCR7 | NM_001301716; NM_001301717; NM_001838; NM_001301718; NM_001301714 |
| BCL6 | NM_001130845;XM_011513062;NM_001706;XM_047448655; NM_001134738; NM_138931; XM 005247694 |
| FDCSP | NM_152997 |
| SERPINE2 | NM_006216; NR_073116; XM_005246641; XM_017004330; NM_001136528; XM_017004332; NM_001136530 |
| PRNP | NM_001080122; NM_001271561; NM_183079; NM_000311; NM_001080121; NM_001080123 |
| PDPN | XM_047434471; NM_001006625; NM_198389; NM_001385053; NM_001006624; XM_006710295; NM_006474; NM_013317; XM 024451404 |
| LTBR | NM_002342; NM_001270987; XM_006718983; XM_005253688 |
| BST1 | XM_011513879; NM_004334; XM_017008565; XM_005248186; XM 005248185; XM 017008566; XM 011513881; XM 011513878 |
| CLU | NM_001831; NR_045494; NR_038335 |
| CIS | NM_001734; NM_001346850; NM_201442 |
| C4A | NM_007293.3; NM_001252204.2 |
| SH2D1A | NM_001114937;NM_002351 |
| MAF | XM_024450279; NM_001031804; XM_017023233; XR_002957802; XR_002957804; XR_001751902; XM_017023235; XM_017023234; NM_005360; XR 002957803 |
| CD84 | NM_003874; XM_011510095; NM_001184882; NM_001330742; NM_001184881; NM_001184879; XR_921991 |
| CXCR5 | NM_032966; NM_001716 |
| CMA1 | NM_001836; NM_001308083 |
| SIGLEC8 | NM_014442; XM_011526734; NM_001363548 |
| TPSAB1 | NM_003294 |
| RNASE3 | NM_002935 |
| PRG3 | NM_006093 |
| RNASE2 | NM_002934 |
| GATA1 | NM_002049 |
| EPX | NM_000502 |
| IL5RA | NM_175726; NM_175724; NM_175727; XM_011533677; NM_001243099; XM_011533678; NM_175725; NM_175728, NM_000564 |
| PRG2 | NM_001302926; NM_002728; NM_001243245; NM_001302927 |
| CPA3 | NM_001870 |
| MS4A2 | XM_011544850; NM_001142303; NM_000139; XM_005273846; XM_017017362; NM_001256916 |
| PRSS33 | NM_001385462; NM_001385463; NM_001385464; NM_152891; NR 169625 |
| CL10 | XM_005250756.4; NM_001324095.2; NM_006438.5; XM_005250756.3 |
| CX3CL1 | NM_001304392; NM_002996 |
| CX3CR1 | NM_001171174; NM_001337; XM_047447538; NM_001171171; NM_001171172 |
| CXCL 11 | NM_001302123; NM_005409 |
| CXCR3 | XM_017029435; XM_017029436; XM_047442010; NM_001504; NM_001142797; XM 005262256; XM 005262257 |

(continued)

| CCL5 | NM_001278736; NM_002985 |
|---|---|
| CXCL9 | NM_002416 |
| CCL4 | NM_002984.4 |
| CCL8 | NM_005623 |
| CSF1 | NM_000757; NM_172210; XM_017000369; XM_047446752; NM_172211; NM_172212 |
| CCR2 | NM_001123041; NM_001123396 |
| XCL1 | NM_002995 |
| XCR1 | NM_001024644; NM_005283; NR_170111; NM_001381860 |
| CCL2 | NM_002982 |
| CCL7 | NM_006273 |
| ADAMTS4 | NM_001320336; NM_005099; XM_047434904 |
| ADAMTS5 | XM_047440680; NM_007038 |
| CA9 | XM_047423849; NM_001216; XM_047423850 |
| LOX | NM_001317073; NM_001178102; NM_002317 |
| MMP1 | NM_001145938; NM_002421 |
| MMP11 | NM_005940; NR_133013 |
| MMP12 | NM_002426 |
| MMP7 | NM_002423 |
| MMP9 | NM_004994 |
| PLOD2 | XM_017006625; NM_000935; XM_047448320; NM_182943; XM_047448319 |
| CCR10 | NM_016602 |
| CCL28 | NM_001301874;NM_001301873; NM_019846; XR_007058611; XR_007058613; XR_925633; NM_148672; NM_001301875; XR 007058610; XR 007058612; XR 427660; XR 241706 |
| CCL17 | XM_047434448; XM_011523256; NM_002987; XM_017023530 |
| CCL22 | XM_047434450; XM_047434449; NM_002990 |
| CCL1 | NM_002981 |
| CSF3R | NM_000760; XM_005270493; NM_156039; XM_011540749; NM_156038; NM_172313; XM 047446753 |
| CSF2RA | XM_047441847; XM_047441851; XM_047442715; NM_001379164; NM_001379166; XM_047441849; XM_047442710; XM_047442713; NM_001161530; NM_001379165; NM_006140; NM_172247; NM_172248; XM_011545627; XM_047441850; NM_001161531; NM_001161532; NM_001379168; NM_172245; XM_047441846; NM_001161529; NM_001379158; NM_001379161; NM_001379162; NM_172246; XM_011545620; XM _011546167; XM_047442711; XM_047442712; XM_047442716; NM_001379155; NM_001379163; NM_172249; XM_047441845; XM_047441848; NM_001379160; NM_001379167; NR_027760; XM_011545628; XM_011546174; XM_047441852; XM_047442718; NM_001379153; XM_011546175; XM_047441853; XM_047442714; XM_047442717; NM_001379154; NM_001379156; NM_001379159; NM_001379169 |
| IL6R | XM_047419649; XM_047419654; NM_001382771; XM_005245139; NM_001206866; NM_001382770; NM_181359; XM_047419650; XM_047419656; NM_001382773; XM_047419657; NM_000565; NM_001382769; NM_001382774; NM_001382772; XM_017001199; XM 047419648; XM 047419655 |
| CCL26 | NM_006072; NM_001371936; NM_001371938 |
| CCL15 | NM_004167; NM_032965; NM_032964 |

(continued)

| CSF2 | NM_000758 |
|---|---|
| CSF3 | NR_168489; NR_168491; NM_000759; NM_001178147; NM_172219; NM_172220; NR_168490; NR_033662 |

EXAMPLES

*Example 1: Basal-like Breast Cancer*

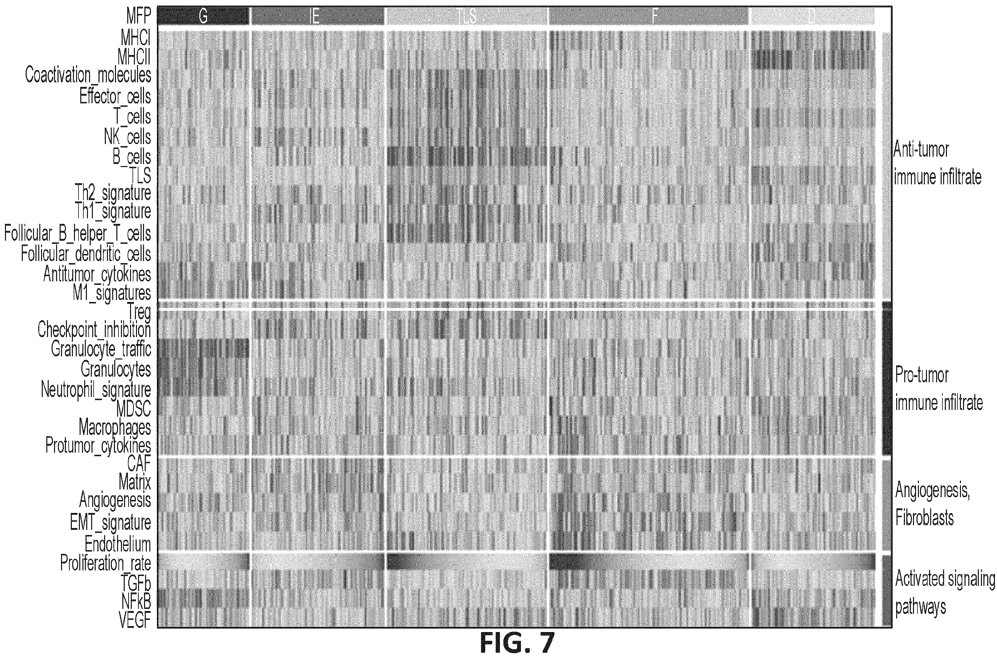

To describe TME molecular functional types of basal-like breast cancer (BLBC), a meta-cohort was collected from public datasets. The mega-cohort comprised RNA expression data from the following datasets: TCGA, Metabric, FUSCCTNBC, GSE103091, GSE106977, GSE21653, GSE25066, GSE41998, GSE47994, GSE81538, GSE96058). Basal-like breast cancer samples were isolated based on the expression profiles of 50 genes (PAM50). Gene expression signatures (e.g., using gene groups and gene group genes described in Table 1 or gene groups shown in FIG. 7), and PROGENy signatures for TGFb, NFkB, VEGF (e.g., as described by Schubert, M., Klinger, B., Klünemann, M. et al. Perturbation-response genes reveal signaling footprints in cancer gene expression. Nat Commun 9, 20 (2018), available at www.nature.com/articles/s41467-017-02391-6) were used to evaluate different biological processes in each of the samples. To overcome batch effect from different datasets, rank estimation and median scaling transformation were used. Using unsupervised clustering, five stable tumor microenvironment (TME) types (FIG. 7) were identified. Four of the molecular types have been described in other cancers, and were identified to be present in BLBC: Immune Enriched (IE), B cells enriched or TLS (TLS), Desert (D), and Fibrotic (F) types. In addition, a novel "Granulocyte enriched" type (Type G) was identified in BLBC.

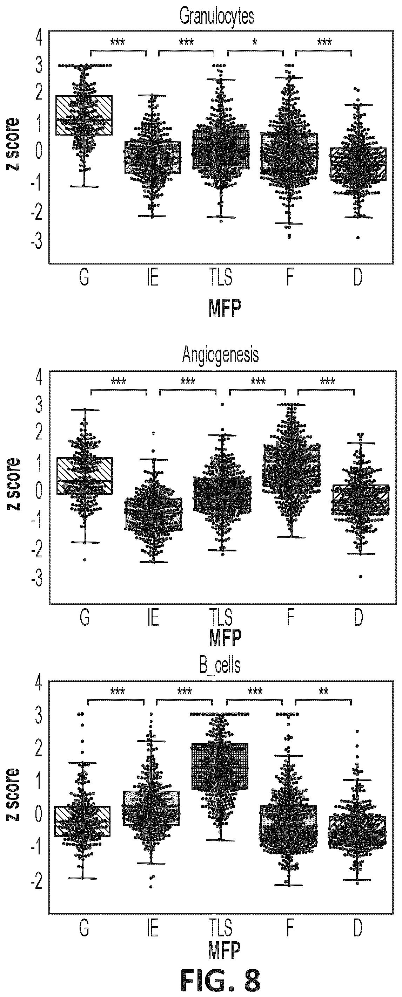

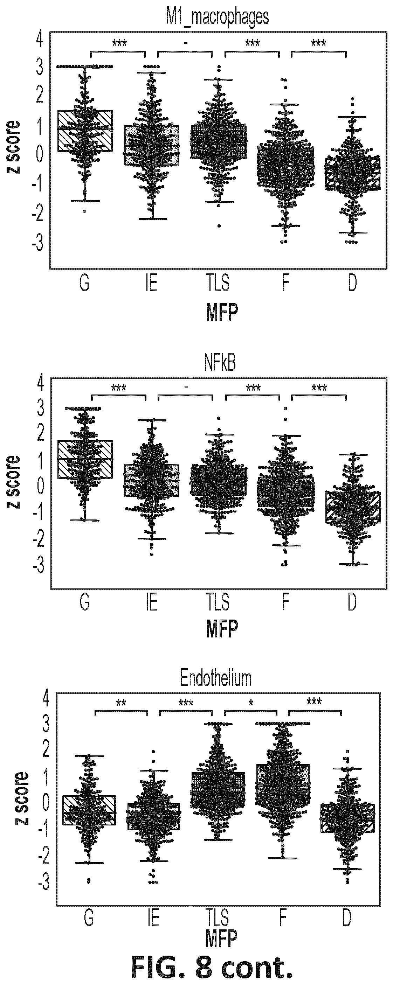

The detected biological processes were confirmed in a comparison between selected biological process signatures across BLBC TME types is shown (FIG. 8). It was observed that BLBC Type G has the highest Granulocytes, M1 macrophages, and NF-kB signals, and Type G shares a high Angiogenesis signal with F type BLBC samples. The TLS type represents the highest B cells signature. TLS type and F type also comprise the highest Endothelium signature out of the five BLBC TME types. The identified BLBC TME types are described as follows:

**Granulocyte-Enriched (G):** The G type is characterized by a high percentage of M1 macrophages, granulocytes, and cytokines regulating granulocyte traffic. The NFkB signaling pathway is upregulated relative to other BLBC TME types. A high tumor proliferation rate signal was observed.

**Immune-Enriched, Non-fibrotic (IE):** The IE type is characterized by abundant immune-active infiltrate-containing cytotoxic effector cells. An immune-inflamed phenotype was observed. Percentage of malignant cells present in subject samples was low. This BLBC TME type is associated with good prognosis.

**Fibrotic (F):** The F type is highly fibrotic with dense collagen formation. F type TME samples comprise minimal leukocyte/lymphocyte infiltration (non-inflamed) with intense angiogenesis. Cancer-associated fibroblasts (CAF) are abundant. This BLBC TME type is associated with poor prognosis.

**Immune Desert (D):** The samples of D type BLBC contain the highest percentage of malignant cells, while leukocyte/lymphocyte infiltration is minimal or completely absent. An immune non-inflamed, immune desert phenotype was observed. This BLBC TME type is associated with a high tumor proliferation rate and poor prognosis.

**B-Cell-Enriched, Tertiary Lymphoid Structure (TLS)-like:** TLS-like BLBC TME type samples are characterized by high levels of immune infiltrate, high vascularity, and a significant number of B cells. A medium prevalence of stromal and fibrotic elements was observed. This BLBC TME type is associated with good prognosis.

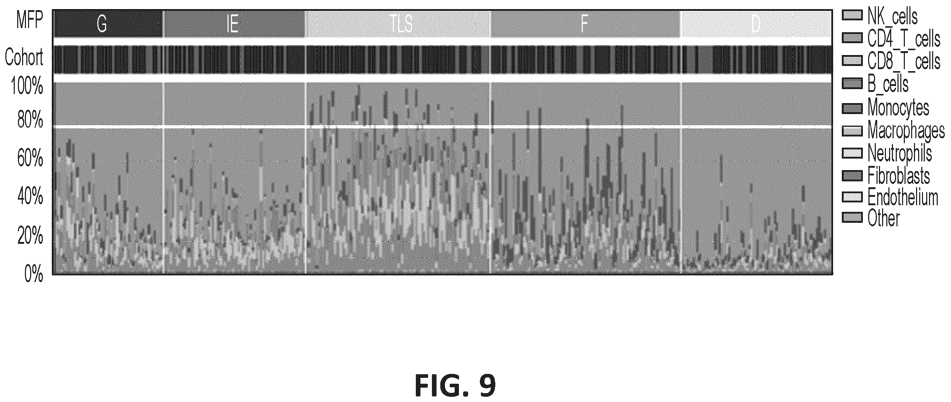

For RNA-seq samples, high B cell content was also investigated by a cell deconvolution algorithm, Kassandra (e.g., as described in International PCT Publication WO2021/183917). This algorithm allows reconstructing cell composition from bulk RNA-seq data and estimating the percentage of different cell types (fibroblasts, B cells, T cells, macrophages, etc.). E-type samples proved the highest B cells percentage (FIG. 9).

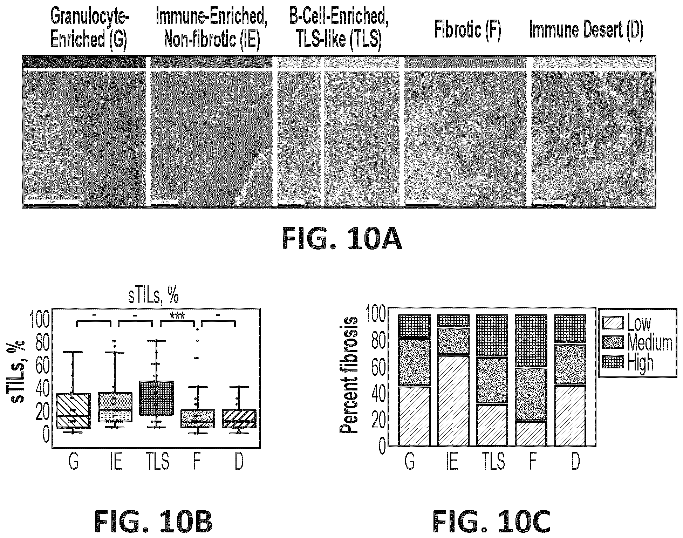

Further evaluation by quantitative histopathological analysis was performed. Data indicate that the gene expression patterns of the identified BLBC TME types correlated with the histological properties of tumors from basal breast cancer patients. Using TCGA slide images, immune infiltration and fibrotic compartments were compared in samples belonging to different TME subtypes (FIGs. 10A-10C). Samples with IE and TLS BLBC TME types had a high percentage of stromal tumor infiltrating lymphocytes, while the F type BLBC TME exhibited the highest fibroblast composition. Samples with D type BLBC TME were characterized by low percentage of stromal tumor-infiltrating lymphocytes (sTILs) and extensive cellular fibrosis, while samples with G type BLBC TME exhibited low cellular collagenized stroma and intermediate percentage of sTILs.

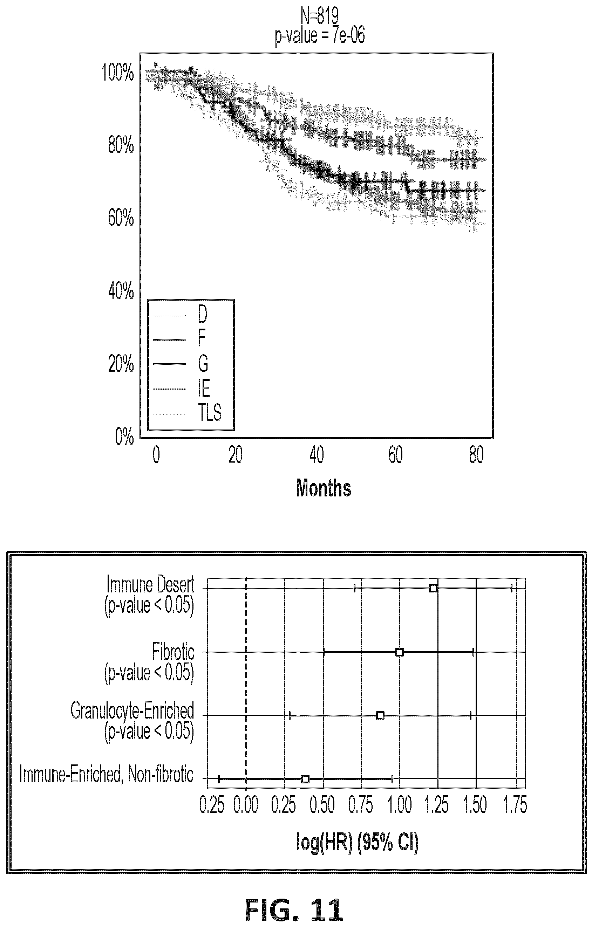

Immune enriched samples have been observed to have better postoperative outcomes and better response to immunochemotherapy. Additionally, TLS-positive samples were observed to be associated with a better disease-free survival rate. Primary breast tumors demonstrated better postoperative outcomes. Analysis of overall survival (OS) reveled a better prognosis for TLS and IE BLBC TME types compared with stromal-enriched (e.g., D type) and G type BLBC TME types (FIG. 11).

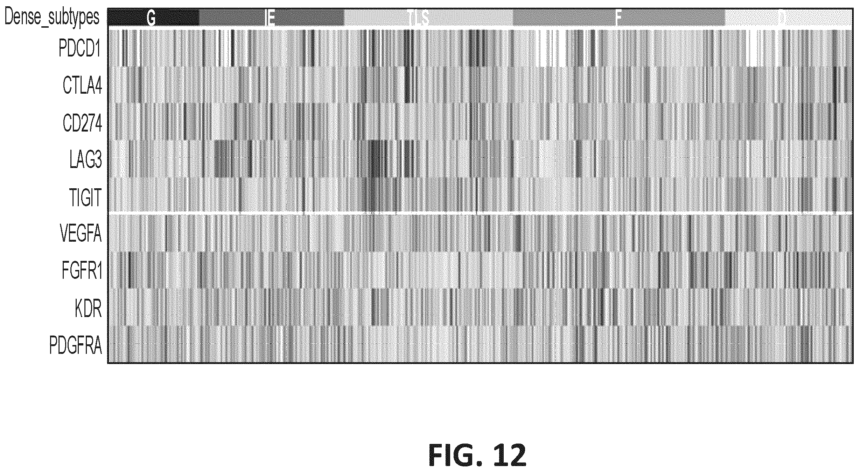

Basal-like tumors have been observed to lack the expression of estrogen receptors, progesterone receptors, and human epidermal growth factor receptors (EGFRs). Therefore, this type of breast cancer has very limited treatment options, leading to the need for use of targeted therapies. The G, IE and TLS BLBC TME type samples were observed to comprise high expression of immune checkpoint genes such as *CD274, PDCD1, CTLA4,* indicating that anti-PD1 or anti-CTLA4 therapy can be recommended to patients with those BLBC TME types. Likewise, anti-VEGF therapy may be recommended for F type BLBC , and TKI inhibitors may be recommended for D type BLBC, due to higher expression of VEGFA and FGFR1 (FIG. 12).

*Example 2: Luminal and Normal-like Breast Cancer*

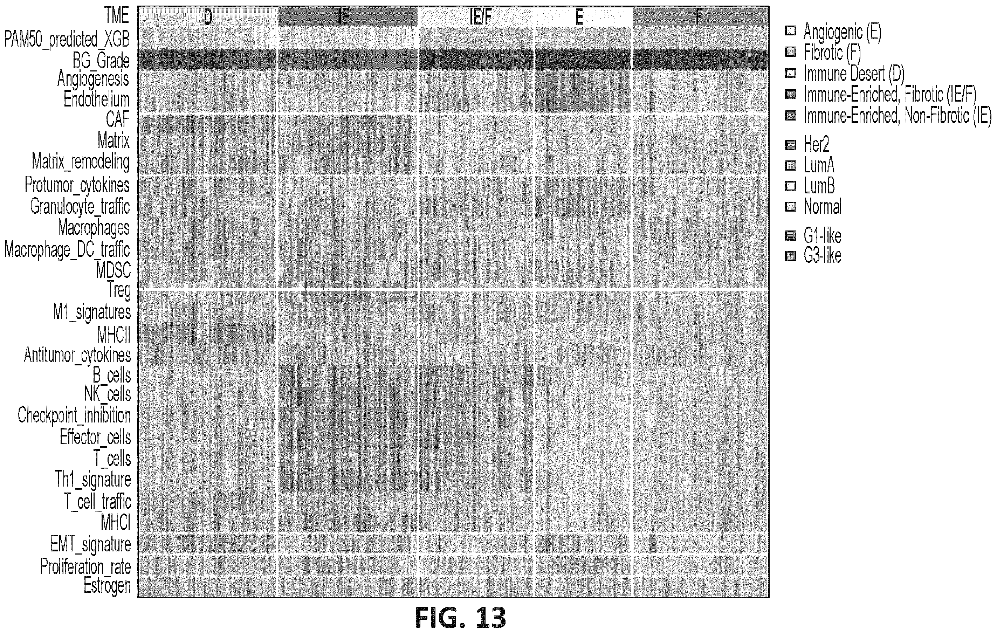

In order to identify breast cancer TME types in luminal and normal-like breast cancer (LNLBC) (e.g., as defined by PAM50), a meta-cohort was compiled using publicly available data. The datasets used to make the meta-cohort included GEO datasets (e.g., GSE102484, GSE20181, GSE20685, GSE25066, GSE59515, GSE93204, and GSE96058, which is part of the SCAN-B dataset), a Metabric dataset, and TCGA dataset. The meta-cohort was classified into intrinsic breast cancer types based on the expression of 50 genes (PAM50). Samples classified as luminal A, luminal B and normal-like were retained for further analysis (5952 samples). For each sample, 24 gene group signatures (e.g., using gene groups and gene group genes described in Table 2 or gene groups shown in FIG. 13) and one PROGENy pathway signature (e.g. Estrogen PROGENy signature) were calculated. Rank and median scale transformation were applied to samples to overcome batch effect. Unsupervised Leiden clustering identified five LNLBC TME types (FIG. 13): Immune Desert (D), Fibrotic (F), Immune-Enriched, Non-fibrotic (IE), Immune-Enriched, Fibrotic (IE/F), and Angiogenic (E) cluster. The Angiogenic (E) type TME is characterized by the high angiogenesis, endothelium, and EMT signatures, and in some embodiments, is referred to as a Highly Vascularized (HV) TME type.

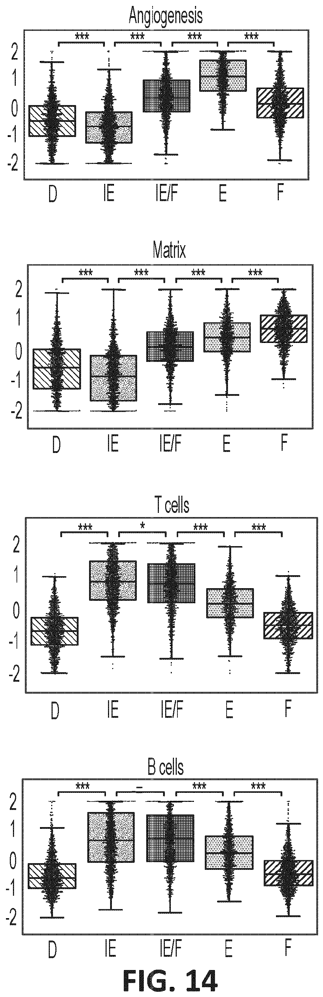

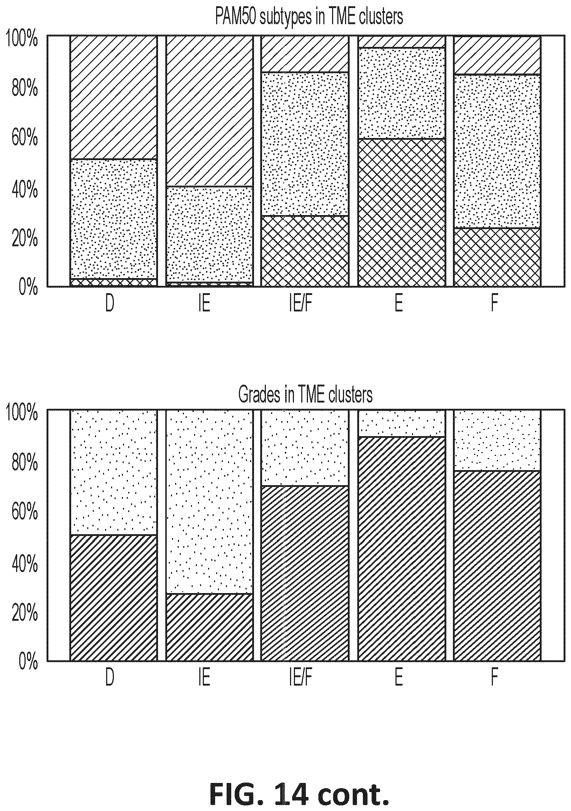

The detected biological processes were confirmed in a comparison between selected biological process signatures are compared across LNLBC TME types (FIG. 14). Type E and Type F samples were observed to have the highest Angiogenesis and stromal (Matrix signature) signals. Type IE and IE/F samples were observed to have the highest T cells signal and B cells signal. The identified LNLBC TME types are described as follows.

**Immune-Enriched, Fibrotic (IE/F).** The IE/F LNLBC TME type is characterized by increased vascularization and a high level of immune infiltrate relative to other LNLBC TME types. An immune-inflamed phenotype was observed. The percentage of malignant cells is low relative to other LNLBC TME types. This LNLBC TME type is associated with low estrogen expression and a low tumor proliferation rate.

**Immune-Enriched, Non-fibrotic (IE).** The IE LNLBC TME type is characterized by abundant immune-active infiltrate-containing cytotoxic effector cells and regulatory T cells. An immune-inflamed phenotype was observed. This LNLBC TME type is associated with a poor prognosis for patients on hormone therapy.

**Fibrotic (F).** The F type LNLBC TME is highly fibrotic with dense collagen formation. Samples were characterized by minimal leukocyte/lymphocyte infiltration, and had a non-inflamed phenotype. Cancer-associated fibroblasts (CAF) are abundant. Signs of epithelial-mesenchymal transition (EMT) are present in F type LNLBC TME samples.

**Immune Desert (D).** D type LNLBC TME contains the highest percentage of malignant cells relative to other LNLBC TME types. Leukocyte/lymphocyte infiltration was observed to be minimal or completely absent. An immune non-inflamed, immune desert phenotype was observed. Type D LNLBC TME samples were characterized by high estrogen expression. This LNLBC TME type is associated with a high tumor proliferation rate.

**Angiogenic (E).** This LNLBC TME type is characterized by intense angiogenesis and medium levels of immune infiltrate relative to other LNLBC TME types. Cancer-associated fibroblasts (CAF) are abundant. Signs of epithelial-mesenchymal transition (EMT) are present in E type LNLBC samples. High levels of pro-tumor cytokines were observed. LNLBC TME type E was associated with low estrogen expression and a low tumor proliferation rate. This LNLBC TME type is commonly associated with a good prognosis for patients on hormone therapy.

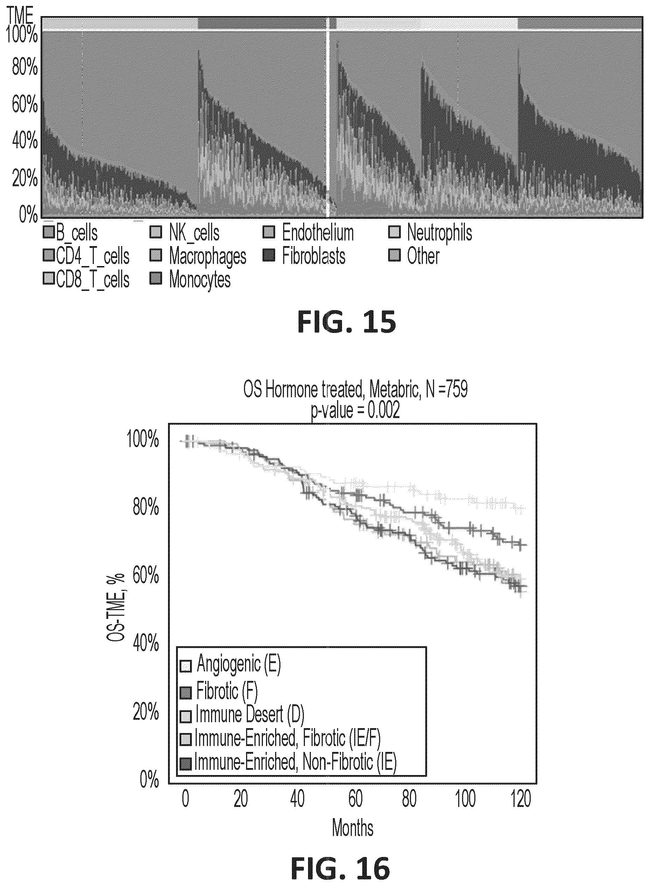

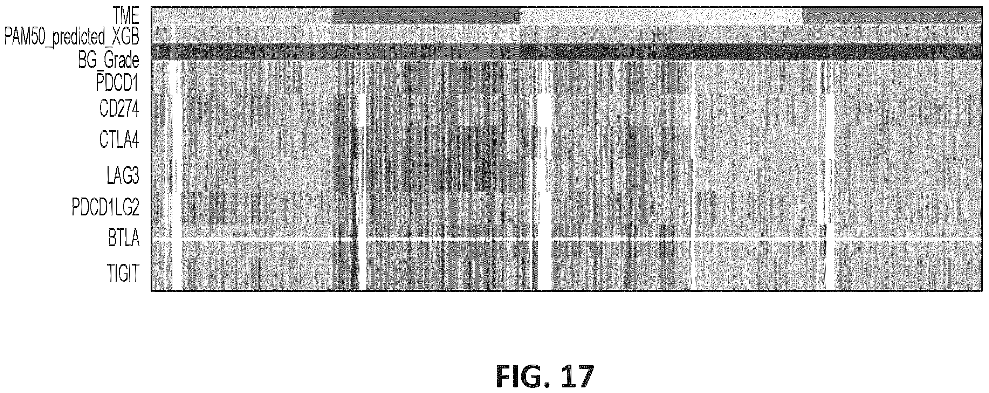

Additional validation of identified clusters was performed by a cell deconvolution algorithm, Kassandra (e.g., as described in International PCT Publication WO2021/183917), for TCGA samples (FIG. 15). Analysis of overall survival (OS) in Metabric dataset indicated a better prognosis for Angiogenic (E) LNLBC TME type compared to Immune-Enriched, Non-fibrotic (IE) and Immune Desert (D) types on hormone therapy (FIG. 16). IE and IE/F subtypes were observed to have the highest expression of immune checkpoint genes such as *CD274, PDCD1, CTLA4* among the LNLBC TME types, indicating that anti-PD1 or anti-CTLA4 therapy can be recommended to patients with those LNLBC TME types (FIG. 17).

*Example 3: HER2-enriched breast cancer*

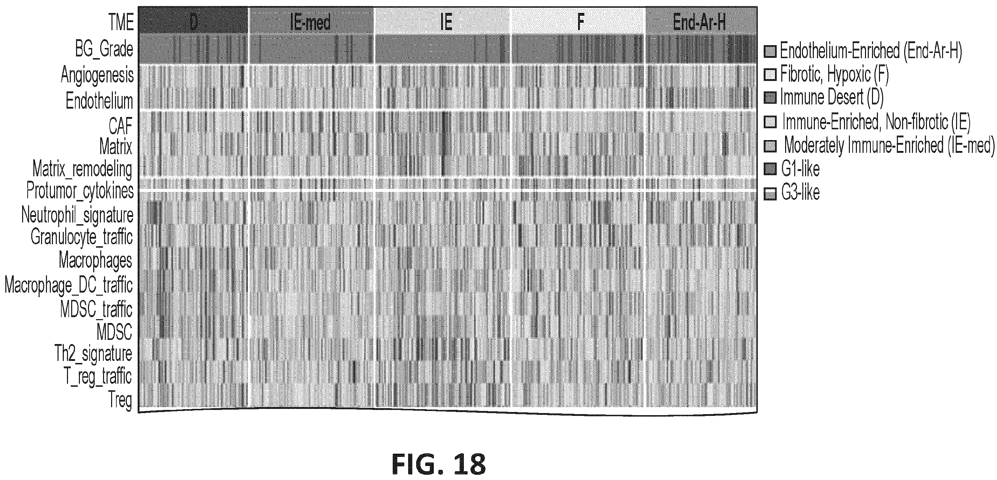

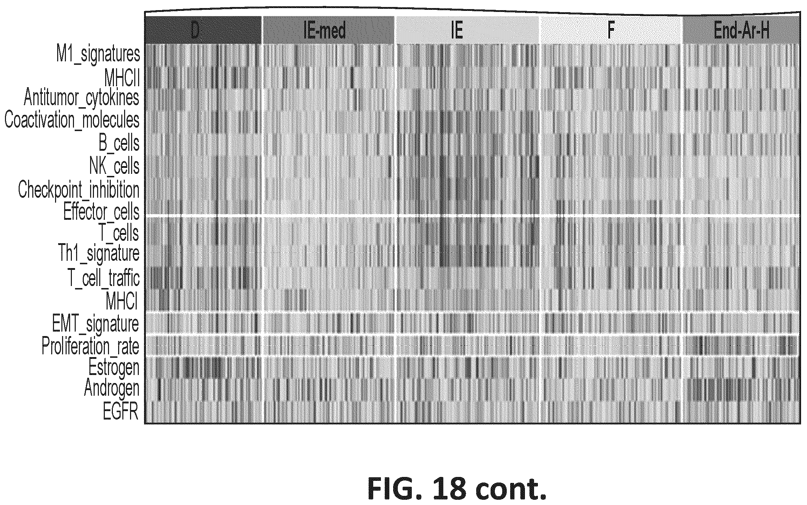

In order to identify TME types in HER2-enriched breast cancer, RNA expression data was obtained from luminal and normal-like breast cancer types. Publicly available data, which included GEO datasets (GSE102484, GSE20685, GSE96058 (part of the SCAN-B dataset), GSE59515, GSE76360, GSE55348, GSE58984), a Metabric dataset, and a TCGA dataset were included. The meta-cohort was classified into intrinsic breast cancer types based on the expression of 50 genes (PAM50). Samples classified as HER2-enriched were selected (924 samples). For each sample, 29 expression signatures and 3 PROGENy pathway signatures (e.g., using gene groups and gene group genes described in Table 3 or gene groups shown in Figure FIG. 18 were calculated. Rank and median scale transformation were applied to samples to overcome batch effect. Unsupervised Leiden clustering identified five TME types (FIG. 18). Three of the types have been described in other cancer types: Immune Desert (D), Fibrotic (F) and Immune-Enriched, Non-fibrotic (IE). Additionally, novel Moderately Immune-Enriched (IE-med) and Endothelium enriched (End-Ar-H) types were identified in HER2-enriched breast cancer (H2EBC). The IE-med type TME is characterized by a moderate level of tumor-infiltrating immune cells, which is less than in IE type breast cancer TME, but higher than in other breast cancer TME types. The End-Ar-H was observed to have the highest number of endothelial cells relative to other H2EBC TME types, and was associated with higher activity of the Androgen PROGENy pathway compared to other H2EBC TME types.

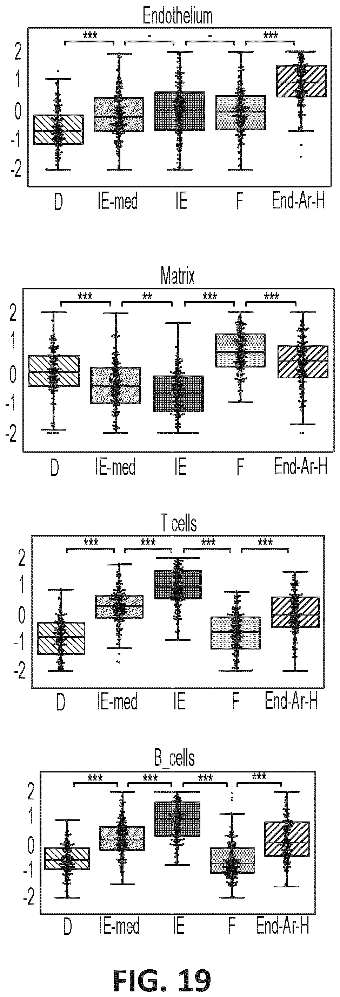

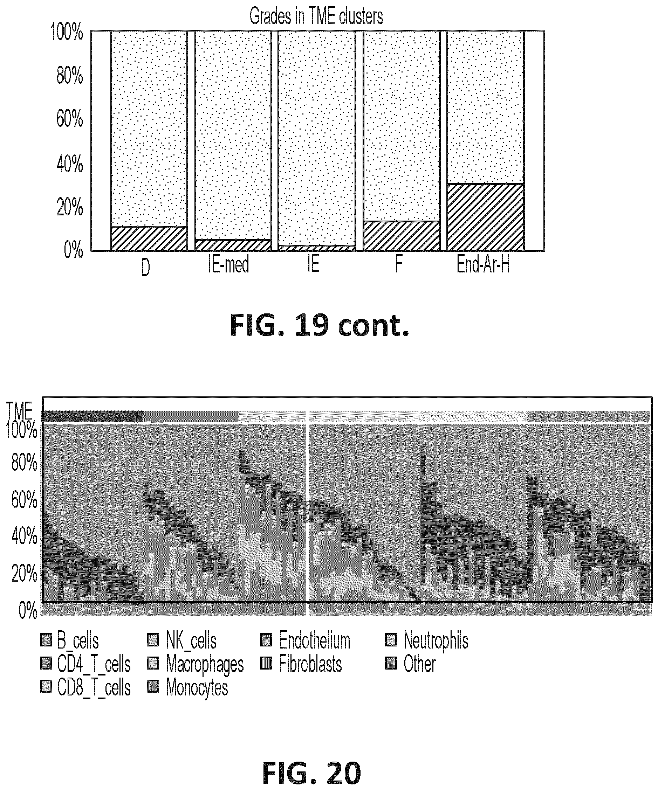

The identified H2EBC TME types were also confirmed by comparing selected biological process signatures TME types (FIG. 19). Type IE was observed to have the highest T cells signal and B cells signal relative to other H2EBC TME types. Type End-Ar-H and F type H2EBC were observed to have the highest stromal (e.g., Matrix signature) signal. The End-Ar-H TME type also had the highest Endothelium signature signal. The identified H2EBC TME types are described as follows.

**Immune Desert (D).** The D type H2EBC TME contains the highest percentage of malignant cells relative to other H2EBC TME types. Leukocyte/lymphocyte infiltration was observed to be minimal or completely absent. An immune non-inflamed, immune desert phenotype was observed. Type D H2EBC TME is characterized by high estrogen pathway activity compared to other H2EBC TME types.

**Moderately Immune-Enriched (IE-med).** The IE-med type H2EBC TME is characterized by a moderate number of tumor-infiltrating immune cells, including B cells, cytotoxic effector cells, and regulatory T cells, relative to other H2EBC TME types. The level of immune cell abundance is lower than in the IE type H2EBC TME. A low level of vascularization was observed. This TME type is associated with a poor prognosis on chemotherapy.

**Immune-Enriched, Non-fibrotic (IE).** The IE type H2EBC TME is characterized by abundant immune-active infiltrate containing cytotoxic effector cells and regulatory T cells. An immune-inflamed phenotype was observed, with signs of vascularization. The percentage of malignant cells is low relative to other H2EBC TME types. This TME type is associated with a good prognosis on chemotherapy.

**Fibrotic, Hypoxic (F).** The F type H2EBC TME is highly vascularized relative to other H2EBC TME types. Samples of F type TME are characterized by dense collagen formations and epithelial-mesenchymal transition (EMT). An immune non-inflamed phenotype was observed. Cancer-associated fibroblasts (CAF) are abundant in this H2EBC TME type.

**Endothelium-rich (End-Ar-H).** The End-Ar-H type H2EBC TME is characterized by the highest number of endothelial cells relative to other H2EBC TME types. This type is associated with angiogenesis and epithelial-mesenchymal transition (EMT). An immune non-inflamed phenotype was observed, with indications of pro-tumor cytokines. The End-Ar-H TME type is characterized by high androgen pathway activity and a low tumor proliferation rate relative to other H2EBC TME types. In some embodiments, the End-Ar-H H2EBC TME type is characterized by higher vascularization relative to other H2EBC TME types.

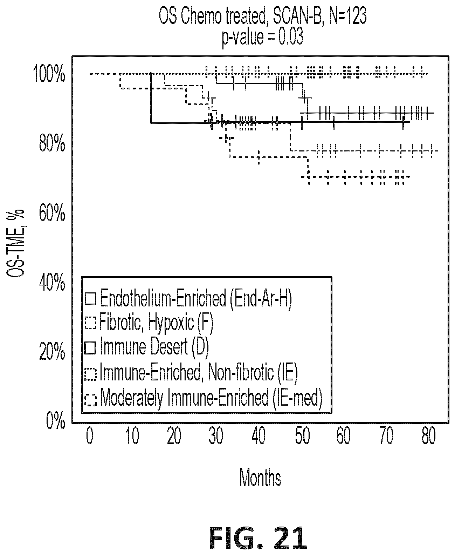

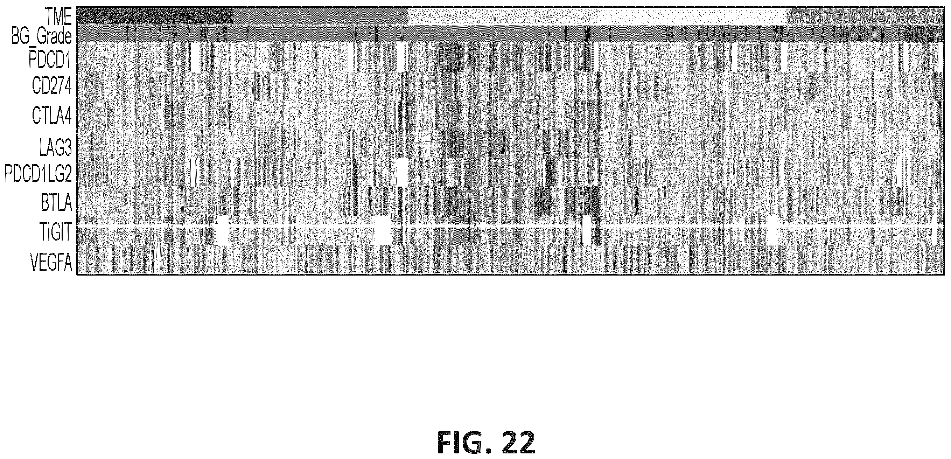

Additional validation of identified H2EBC TME types was performed by a cell deconvolution algorithm, Kassandra (e.g., as described in International PCT Publication WO2021/183917), for TCGA samples (FIG. 20). Analysis of overall survival (OS) in GSE96058 dataset indicated a better prognosis for Immune-Enriched, Non-fibrotic (IE) H2EBC TME type compared to other TME types on chemotherapy. Moderately immune-enriched (IE-med) TME type was associated with the worst prognosis. (FIG. 21). The highest expression of immune checkpoint genes was observed in IE and IE-med samples across all TME types. This indicates that anti-PD1 or anti-CTLA4 therapy can be recommended to patients with those TME types. Likewise, anti-VEGF therapy can be recommended for H2EBC F type, due to higher expression of *VEGFA* (FIG. 22).

EQUIVALENTS

Having thus described several aspects and embodiments of the technology set forth in the disclosure, it is to be appreciated that various alterations, modifications, and improvements will readily occur to those skilled in the art. For example, those of ordinary skill in the art will readily envision a variety of other means and/or structures for performing the function and/or obtaining the results and/or one or more of the advantages described herein, and each of such variations and/or modifications is deemed to be within the scope of the embodiments described herein. Those skilled in the art will recognize or be able to ascertain using no more than routine experimentation many equivalents to the specific embodiments described herein. It is, therefore, to be understood that the foregoing embodiments are presented by way of example only. In addition, any combination of two or more features, systems, articles, materials, kits, and/or methods described herein, if such features, systems, articles, materials, kits, and/or methods are not mutually inconsistent, is included within the scope of the present disclosure.

The above-described embodiments can be implemented in any of numerous ways. One or more aspects and embodiments of the present disclosure involving the performance of processes or methods may utilize program instructions executable by a device (e.g., a computer, a processor, or other device) to perform, or control performance of, the processes or methods. In this respect, various inventive concepts may be embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other tangible computer storage medium) encoded with one or more programs that, when executed on one or more computers or other processors, perform methods that implement one or more of the various embodiments described above. The computer readable medium or media can be transportable, such that the program or programs stored thereon can be loaded onto one or more different computers or other processors to implement various ones of the aspects described above. In some embodiments, computer readable media may be non-transitory media.

The terms "program" or "software" are used herein in a generic sense to refer to any type of computer code or set of computer-executable instructions that can be employed to program a computer or other processor to implement various aspects as described above. Additionally, it should be appreciated that according to one aspect, one or more computer programs that when executed perform methods of the present disclosure need not reside on a single computer or processor, but may be distributed in a modular fashion among a number of different computers or processors to implement various aspects of the present disclosure.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

When implemented in software, the software code can be executed on any suitable processor or collection of processors, whether provided in a single computer or distributed among multiple computers.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone, a tablet, or any other suitable portable or fixed electronic device.

Also, a computer may have one or more input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible formats.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B," when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

**Claims**

1. A method for determining a basal-like breast cancer (BLBC) tumor microenvironment (TME) type of a subject having basal-like breast cancer, the method comprising:
   using at least one computer hardware processor to perform:

   obtaining RNA expression data for the subject, the RNA expression data indicating RNA expression levels for at least some genes in each group of at least some of a plurality of gene groups listed in the table below in a biological sample comprising tumor tissue of the subject:

| MHC I | HLA-C, TAPBP, HLA-B, B2M, TAP2, HLA-A, TAP1, NLRC5 |
|---|---|
| MHC II | HLA-DQB1, HLA-DMA, HLA-DMB, HLA-DRA, CIITA, HLA-DQA1, HLA-DPB1, HLA-DRB1, HLA-OPA 1 |
| Coactivation molecules | TNFRSF4, CD27, CD80, CD40LG, TNFRSF9, CD40, CD28, ICOSLG, CD83, TNFSF9, CD70, TNFSF4, ICOS, CD86 |
| Effector cells | ZAP70, GZMB, GZMK, IFNG, FASLG, EOMES, TBX21, GZMA, CD8A, GNLY, PRF1, CD8B |
| NK cells | NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160 |
| T cells | TRBC2, CD3E, CD3G, ITK, CD28, TRBC1, TRAT1, TBX21, CD5, TRAC, CD3D |
| B cells | CD22, TNFRSF13C, STAP1, CD79B, PAX5, CR2, TNFRSF13B, CD79A, TNFRSF17, FCRL5, MS4A 1, CD19, BLK |
| M1 signatures | CMKLR1, SOCS3, IRF5, NOS2, IL1B, IL12B, IL23A, TNF, IL12A |
| Th1 signature | IL21, TBX21, IL12RB2, CD40LG, IFNG, IL2, STAT4 |
| Antitumor cytokines | CCL3, IL21, IFNB1, IFNA2, TNF, TNFSF10 |
| Checkpoint inhibition | PDCD1, BTLA, HAVCR2, CD274, VSIR, LAG3, TIGIT, PDCD1LG2, CTLA4 |
| Treg | IKZF2, TNFRSF18, IL10, FOXP3, CCR8, IKZF4, CTLA4 |
| Neutrophil signature | CD177, FFAR2, PGLYRP1, CXCR1, MPO, CXCR2, ELANE, CTSG, PRTN3, FCGR3B |
| Granulocyte traffic | CCR3, KITLG, CCL11, CXCL2, CXCR1, CXCR2, CXCL8, CXCL1, CXCL5 |
| MDSC | ARG1, IL6, CYBB, IL10, PTGS2, IDO1, IL4I1 |
| Macrophages | MRC1, SIGLEC1, MSR1, CD163, CSF1R, CD68, IL4I1, IL10 |
| Th2 signature | IL13, CCR4, IL10, IL4, IL5 |
| Protumor cytokines | TGFB2, MIF, IL6, TGFB3, TGFB1, IL22, IL10 |
| CAF | COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, LRP1 |
| Matrix | LAMC2, TNC, COL11A1, VTN, LAMB3, COL1A1, FN1, LAMA3, LGALS9, COL1A2, COL4A1, COL5A1, ELN, LGALS7, COL3A1 |

(continued)

| | |
|---|---|
| Angiogenesis Endothelium | *VEGFC, VEGFA, PDGFC, KDR, CDH5, VEGFB, PGF, TEK, ANGPT2, CXCR2, FLT1, CXCL8, VWF, ANGPT1, CXCL5 KDR, CDH5, NOS3, VCAM1, VWF, FLT1, MMRN1, CLEC14A, ENG, MMRN2* |
| Proliferation rate | *CCND1, CCNB1, CETN3, CDK2, E2F1, AURKA, BUB1, AURKB, PLK1, MCM6, ESCO2, MYBL2, MKI67, MCM2, CCNE1* |
| EMT signature | *CDH2, ZEB1, ZEB2, TWIST1, SNAI1, SNAI2, TWIST2* |
| TLS | *CXCL13, CCL19, LAMP3, SELL, CXCR4, CCL21, CCR7, CD86, BCL6* |
| Follicular dendritic cells | *FDCSP, SERPINE2, PRNP, PDPN, LTBR, BST1, CLU, C1S, C4A* |
| Follicular B helper T cells | *SH2D1A, IL6, MAF, CD40LG, IL21, ICOS, IL4, CD84, CXCR5, BCL6* |
| Granulocytes | *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, PRSS33* |

wherein the RNA expression data indicate RNA expression levels for at least three genes from each of at least: the granulocytes gene group, the angiogenesis gene group, the B-cells gene group, the M1 signatures gene group, and the endothelium gene group; and

generating a BLBC TME signature for the subject using the RNA expression data, the BLBC TME signature comprising gene group scores for respective gene groups in the at least some of the plurality of gene groups, the generating comprising:

determining gene groups scores using the RNA expression levels and a gene set enrichment analysis technique; and

identifying, using the BLBC TME signature and from among a plurality of BLBC TME types, a BLBC TME type for the subject, wherein the plurality of BLBC TME types comprises: Immune Enriched (IE) type, TLS (TLS) type, Desert (D) type, Fibrotic (F) type, and Granulocyte enriched (G) type and wherein:

(i) BLBC Type G comprises the highest Granulocytes and M1 signatures gene group scores of the plurality of BLBC TME types,
(ii) types G and F have higher Angiogenesis gene group scores than the other BLBC TME types of the plurality of BLBC TME types,
(iii) the TLS type has the highest B cells gene group score of the plurality of BLBC TME types, and
(iv) types TLS and F have the highest Endothelium expression level gene group scores of the plurality of BLBC TME types.

2. The method of claim 1, wherein: (i) obtaining the RNA expression data for the subject comprises obtaining sequencing data previously obtained by sequencing a biological sample obtained from the subject, wherein the biological sample comprises tumor tissue of the subject, and/or (ii) the method further comprises normalizing the RNA expression data to transcripts per million (TPM) units prior to generating the BLBC TME signature.

3. The method of claim 2, wherein the sequencing data comprises:

(a) at least 1 million reads, at least 5 million reads, at least 10 million reads, at least 20 million reads, at least 50 million reads, or at least 100 million reads; and/or
(b) whole exome sequencing (WES) data, bulk RNA sequencing (RNA-seq) data, single cell RNA sequencing (scRNA-seq) data, and/or next generation sequencing (NGS) data; or
(c) microarray data.

4. The method of any one of claims 1 to 3, wherein obtaining the RNA expression data for the subject comprises sequencing a biological sample obtained from the subject, wherein the biological sample comprises tumor tissue of the subject.

5. The method of any one of claims 1 to 4, wherein the RNA expression levels comprise RNA expression levels for at least three genes from each of the following gene groups, or RNA expression levels for each of the genes from each of the following gene groups:

(a) MHC I group: *HLA-C, TAPBP, HLA-B, B2M, TAP2, HLA-A, TAP1,* and *NLRC5*;
(b) MHC II group: *HLA-DQB1, HLA-DMA, HLA-DMB, HLA-DRA, CIITA, HLA-DQA1, HLA-DPB1, HLA-DRB1,* and *HLA-DPA1;*
(c) Coactivation molecules group: *TNFRSF4, CD27, CD80, CD40LG, TNFRSF9, CD40, CD28, ICOSLG, CD83, TNFSF9, CD70, TNFSF4, ICOS,* and *CD86;*
(d) Effector cells group: *ZAP70, GZMB, GZMK, IFNG, FASLG, EOMES, TBX21, GZMA, CD8A, GNLY, PRF1,* and *CD8B;*
(e) NK cells group: *NKG7, FGFBP2, CD244, KLRK1, KIR2DL4, CD226, KLRF1, GNLY, GZMB, KLRC2, NCR1, GZMH, IFNG, SH2D1B, NCR3, EOMES,* and *CD160;*
(f) T cells group: *TRBC2, CD3E, CD3G, ITK, CD28, TREC1, TRAT1, TBX21, CD5, TRAC,* and *CD3D;*
(g) B cells group: *CD22, TNFRSF13C, STAP1, CD79B, PAX5, CR2, TNFRSF13B, CD79A, TNFRSF17, FCRL5, MS4A1, CD19,* and *BLK;*
(h) M1 signature group: *CMKLR1, SOCS3, IRF5, NOS2, IL1B, IL12B, IL23A, TNF,* and *IL12A*;
(i) Th1 signature group: *IL21, TBX21, IL12RB2, CD40LG, IFNG, IL2,* and *STAT4;*
(j) Antitumor cytokines group: *CCL3, IL21, IFNB1, IFNA2, TNF,* and *TNFSF10*;
(k) Checkpoint inhibition group: *PDCD1, BTLA, HAVCR2, CD274, VSIR, LAG3, TIGIT, PDCD1LG2, and CTLA4;*
(l) Treg group: *IKZF2, TNFRSF18, IL10, FOXP3, CCR8, IKZF4,* and *CTLA4;*
(m) Neutrophil signature group: *CD177, FFAR2, PGLYRP1, CXCR1, MPO, CXCR2, ELANE, CTSG, PRTN3,* and *FCGR3B;*
(n) Granulocyte traffic group: *CCR3, KITLG, CCL11, CXCL2, CXCR1, CXCR2, CXCL8, CXCL1,* and *CXCL5;*
(o) MDSC group: *ARG1, IL6, CYBB, IL10, PTGS2, IDO1,* and *IL4I1*;
(p) Th2 signature group: *IL13, CCR4, IL10, IL4, and IL5;*
(q) Protumor cytokines group: *TGFB2, MIF, IL6, TGFB3, TGFB1, IL22,* and *IL10*;
(r) Cancer-associated fibroblast (CAF) group: *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* and *LRP1*;
(s) Matrix group: *LAMC2, TNC, COL11A1, VTN, LAMB3, COL1A1, FN1, LAMA3, LGALS9, COL1A2, COL4A1, COL5A1, ELN, LGALS7,* and *COL3A1;*
(t) Angiogenesis group: *VEGFC, VEGFA, PDGFC, KDR, CDH5, VEGFB, PGF, TEK, ANGPT2, CXCR2, FLT1, CXCL8, VWF, ANGPT1,* and *CXCL5*;
(u) Endothelium group: *KDR, CDH5, NOS3, VCAM1, VWF, FLT1, MMRN1, CLEC14A, ENG,* and *MMRN2;*
(v) Proliferation rate group: *CCND1, CCNB1, CETN3, CDK2, E2F1, AURKA, BUB1, AURKB, PLK1, MCM6, ESCO2, MYBL2, MKI67, MCM2,* and *CCNE1*;
(w) EMT signature group: *CDH2, ZEB1, ZEB2, TWIST1, SNAI1, SNAI2,* and *TWIST2*;
(x) Tertiary Lymphoid Structure (TLS) group: *CXCL13, CCL19, LAMP3, SELL, CXCR4, CCL21, CCR7, CD86,* and *BCL6;*
(y) Follicular dendritic cells group: *FDCSP, SERPINE2, PRNP, PDPN, LTBR, BST1, CLU, C1S,* and *C4A;*
(z) Follicular B helper T cells group: *SH2D1A, IL6, MAF, CD40LG, IL21, ICOS, IL4, CD84, CXCR5,* and *BCL6;* and
(aa) Granulocytes group: *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* and *PRSS33.*

6. The method of any one of claims 1 to 5, wherein determining the gene group scores comprises:

(i) determining a respective gene group score for each of at least six of the following gene groups including the granulocytes gene group, the angiogenesis gene group, the B-cells gene group, the M1 signatures gene group, and the endothelium gene group, using, for a particular gene group, RNA expression levels for at least three genes in the particular gene group to determine the gene group score for the particular group, the gene groups including:

(a) MHC I group: *HLA-C, TAPBP, HLA-B, B2M, TAP2, HLA-A, TAP1,* and *NLRC5*;
(b) MHC II group: *HLA-DQB1, HLA-DMA, HLA-DMB, HLA-DRA, CIITA, HLA-DQA1, HLA-DPB1, HLA-DRB1,* and *HLA-DPA1;*
(c) Coactivation molecules group: *TNFRSF4, CD27, CD80, CD40LG, TNFRSF9, CD40, CD28, ICOSLG, CD83, TNFSF9, CD70, TNFSF4, ICOS,* and *CD86;*

(d) Effector cells group: *ZAP70, GZMB, GZMK, IFNG, FASLG, EOMES, TBX21, GZMA, CD8A, GNLY, PRF1,* and *CD8B;*

(e) NK cells group: *NKG7, FGFBP2, CD244, KLRK1, KIR2DL4, CD226, KLRF1, GNLY, GZMB, KLRC2, NCR1, GZMH, IFNG, SH2D1B, NCR3, EOMES,* and *CD160;*

(f) T cells group: *TRBC2, CD3E, CD3G, ITK, CD28, TRBC1, TRAT1, TBX21, CD5, TRAC,* and *CD3D;*

(g) B cells group: *CD22, TNFRSF13C, STAP1, CD79B, PAX5, CR2, TNFRSF13B, CD79A, TNFRSF17, FCRL5, MS4A1, CD19,* and *BLK;*

(h) M1 signature group: *CMKLR1, SOCS3, IRF5, NOS2, IL1B, IL12B, IL23A, TNF,* and *IL12A;*

(i) Th1 signature group: *IL21, TBX21, IL12RB2, CD40LG, IFNG, IL2,* and *STAT4;*

(j) Antitumor cytokines group: *CCL3, IL21, IFNB1, IFNA2, TNF,* and *TNFSF10;*

(k) Checkpoint inhibition group: *PDCD1, BTLA, HAVCR2, CD274, VSIR, LAG3, TIGIT, PDCD1LG2, and CTLA4;*

(l) Treg group: *IKZF2, TNFRSF18, IL10, FOXP3, CCR8, IKZF4,* and *CTLA4;*

(m) Neutrophil signature group: *CD177, FFAR2, PGLYRP1, CXCR1, MPO, CXCR2, ELANE, CTSG, PRTN3,* and *FCGR3B;*

(n) Granulocyte traffic group: *CCR3, KITLG, CCL11, CXCL2, CXCR1, CXCR2, CXCL8, CXCL1,* and *CXCL5;*

(o) MDSC group: *ARG1, IL6, CYBB, IL10, PTGS2, IDO1,* and *IL4I1;*

(p) Th2 signature group: *IL13, CCR4, IL10, IL4, and IL5;*

(q) Protumor cytokines group: *TGFB2, MIF, IL6, TGFB3, TGFB1, IL22,* and *IL10;*

(r) Cancer-associated fibroblast (CAF) group: *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* and *LRP1;*

(s) Matrix group: *LAMC2, TNC, COL11A1, VTN, LAMB3, COL1A1, FN1, LAMA3, LGALS9, COL1A2, COL4A1, COL5A1, ELN, LGALS7,* and *COL3A1;*

(t) Angiogenesis group: *VEGFC, VEGFA, PDGFC, KDR, CDH5, VEGFB, PGF, TEK, ANGPT2, CXCR2, FLT1, CXCL8, VWF, ANGPT1,* and *CXCL5;*

(u) Endothelium group: *KDR, CDH5, NOS3, VCAM1, VWF, FLT1, MMRN1, CLEC14A, ENG,* and *MMRN2;*

(v) Proliferation rate group: *CCND1, CCNB1, CETN3, CDK2, E2F1, AURKA, BUB1, AURKB, PLK1, MCM6, ESCO2, MYBL2, MKI67, MCM2,* and *CCNE1;*

(w) EMT signature group: *CDH2, ZEB1, ZEB2, TWIST1, SNAI1, SNAI2,* and *TWIST2;*

(x) Tertiary Lymphoid Structure (TLS) group: *CXCL13, CCL19, LAMP3, SELL, CXCR4, CCL21, CCR7, CD86,* and *BCL6;*

(y) Follicular dendritic cells group: *FDCSP, SERPINE2, PRNP, PDPN, LTBR, BST1, CLU, C1S,* and *C4A;*

(z) Follicular B helper T cells group: *SH2D1A, IL6, MAF, CD40LG, IL21, ICOS, IL4, CD84, CXCR5,* and *BCL6;* and

(aa) Granulocytes group: *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* and *PRSS33.*

**7.** The method of any preceding claim, wherein determining the gene group scores comprises:

(i) determining a respective gene group score for each of the following gene groups, using, for a particular gene group, RNA expression levels for each of the genes in each gene group to determine the gene group score for each particular group, the gene groups including:

(a) MHC I group: *HLA-C, TAPBP, HLA-B, B2M, TAP2, HLA-A, TAP1,* and *NLRC5;*

(b) MHC II group: *HLA-DQB1, HLA-DMA, HLA-DMB, HLA-DRA, CIITA, HLA-DQA1, HLA-DPB1, HLA-DRB1,* and *HLA-DPA1;*

(c) Coactivation molecules group: *TNFRSF4, CD27, CD80, CD40LG, TNFRSF9, CD40, CD28, ICOSLG, CD83, TNFSF9, CD70, TNFSF4, ICOS,* and *CD86;*

(d) Effector cells group: *ZAP70, GZMB, GZMK, IFNG, FASLG, EOMES, TBX21, GZMA, CD8A, GNLY, PRF1,* and *CD8B;*

(e) NK cells group: *NKG7, FGFBP2, CD244, KLRK1, KIR2DL4, CD226, KLRF1, GNLY, GZMB, KLRC2, NCR1, GZMH, IFNG, SH2D1B, NCR3, EOMES,* and *CD160;*

(f) T cells group: *TRBC2, CD3E, CD3G, ITK, CD28, TRBC1, TRAT1, TBX21, CD5, TRAC,* and *CD3D;*

(g) B cells group: *CD22, TNFRSF13C, STAP1, CD79B, PAX5, CR2, TNFRSF13B, CD79A, TNFRSF17, FCRL5, MS4A1, CD19,* and *BLK;*

(h) M1 signature group: *CMKLR1, SOCS3, IRF5, NOS2, IL1B, IL12B, IL23A, TNF,* and *IL12A;*

(i) Th1 signature group: *IL21, TBX21, IL12RB2, CD40LG, IFNG, IL2,* and *STAT4;*

(j) Antitumor cytokines group: *CCL3, IL21, IFNB1, IFNA2, TNF,* and *TNFSF10;*

(k) Checkpoint inhibition group: *PDCD1, BTLA, HAVCR2, CD274, VSIR, LAG3, TIGIT, PDCD1LG2, and CTLA4;*

(l) Treg group: *IKZF2, TNFRSF18, IL10, FOXP3, CCR8, IKZF4,* and *CTLA4;*

(m) Neutrophil signature group: *CD177, FFAR2, PGLYRP1, CXCR1, MPO, CXCR2, ELANE, CTSG, PRTN3,* and *FCGR3B;*

(n) Granulocyte traffic group: *CCR3, KITLG, CCL11, CXCL2, CXCR1, CXCR2, CXCL8, CXCL1,* and *CXCL5;*

(o) MDSC group: *ARG1, IL6, CYBB, IL10, PTGS2, IDO1,* and *IL4I1;*

(p) Th2 signature group: *IL13, CCR4, IL10, IL4, and IL5;*

(q) Protumor cytokines group: *TGFB2, MIF, IL6, TGFB3, TGFB1, IL22,* and *IL10;*

(r) Cancer-associated fibroblast (CAF) group: *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* and *LRP1;*

(s) Matrix group: *LAMC2, TNC, COL11A1, VTN, LAMB3, COL1A1, FN1, LAMA3, LGALS9, COL1A2, COL4A1, COL5A1, ELN, LGALS7,* and *COL3A1;*

(t) Angiogenesis group: *VEGFC, VEGFA, PDGFC, KDR, CDH5, VEGFB, PGF, TEK, ANGPT2, CXCR2, FLT1, CXCL8, VWF, ANGPT1,* and *CXCL5;*

(u) Endothelium group: *KDR, CDH5, NOS3, VCAM1, VWF, FLT1, MMRN1, CLEC14A, ENG,* and *MMRN2;*

(v) Proliferation rate group: *CCND1, CCNB1, CETN3, CDK2, E2F1, AURKA, BUB1, AURKB, PLK1, MCM6, ESCO2, MYBL2, MKI67, MCM2,* and *CCNE1;*

(w) EMT signature group: *CDH2, ZEB1, ZEB2, TWIST1, SNAI1, SNAI2,* and *TWIST2;*

(x) Tertiary Lymphoid Structure (TLS) group: *CXCL13, CCL19, LAMP3, SELL, CXCR4, CCL21, CCR7, CD86,* and *BCL6;*

(y) Follicular dendritic cells group: *FDCSP, SERPINE2, PRNP, PDPN, LTBR, BST1, CLU, C1S,* and *C4A;*

(z) Follicular B helper T cells group: *SH2D1A, IL6, MAF, CD40LG, IL21, ICOS, IL4, CD84, CXCR5,* and *BCL6;* and

(aa) Granulocytes group: *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* and *PRSS33;* and/or

(ii) determining a first score of a first gene group using a single-sample Gene Set Enrichment Analysis (ssGSEA) technique from RNA expression levels for at least some of the genes in one or the following gene groups:

(a) MHC I group: *HLA-C, TAPBP, HLA-B, B2M, TAP2, HLA-A, TAP1,* and *NLRC5;*

(b) MHC II group: *HLA-DQB1, HLA-DMA, HLA-DMB, HLA-DRA, CIITA, HLA-DQA1, HLA-DPB1, HLA-DRB1,* and *HLA-DPA1;*

(c) Coactivation molecules group: *TNFRSF4, CD27, CD80, CD40LG, TNFRSF9, CD40, CD28, ICOSLG, CD83, TNFSF9, CD70, TNFSF4, ICOS,* and *CD86;*

(d) Effector cells group: *ZAP70, GZMB, GZMK, IFNG, FASLG, EOMES, TBX21, GZMA, CD8A, GNLY, PRF1,* and *CD8B;*

(e) NK cells group: *NKG7, FGFBP2, CD244, KLRK1, KIR2DL4, CD226, KLRF1, GNLY, GZMB, KLRC2, NCR1, GZMH, IFNG, SH2D1B, NCR3, EOMES,* and *CD160;*

(f) T cells group: *TRBC2, CD3E, CD3G, ITK, CD28, TRBC1, TRAT1, TBX21, CD5, TRAC,* and *CD3D;*

(g) B cells group: *CD22, TNFRSF13C, STAP1, CD79B, PAX5, CR2, TNFRSF13B, CD79A, TNFRSF17, FCRL5, MS4A1, CD19,* and *BLK;*

(h) M1 signature group: *CMKLR1, SOCS3, IRF5, NOS2, IL1B, IL12B, IL23A, TNF,* and *IL12A;*

(i) Th1 signature group: *IL21, TBX21, IL12RB2, CD40LG, IFNG, IL2,* and *STAT4;*

(j) Antitumor cytokines group: *CCL3, IL21, IFNB1, IFNA2, TNF,* and *TNFSF10;*

(k) Checkpoint inhibition group: *PDCD1, BTLA, HAVCR2, CD274, VSIR, LAG3, TIGIT, PDCD1LG2, and CTLA4;*

(l) Treg group: *IKZF2, TNFRSF18, IL10, FOXP3, CCR8, IKZF4,* and *CTLA4;*

(m) Neutrophil signature group: *CD177, FFAR2, PGLYRP1, CXCR1, MPO, CXCR2, ELANE, CTSG, PRTN3,* and *FCGR3B;*

(n) Granulocyte traffic group: *CCR3, KITLG, CCL11, CXCL2, CXCR1, CXCR2, CXCL8, CXCL1,* and *CXCL5;*

(o) MDSC group: *ARG1, IL6, CYBB, IL10, PTGS2, IDO1,* and *IL4I1;*

(p) Th2 signature group: *IL13, CCR4, IL10, IL4, and IL5;*

(q) Protumor cytokines group: *TGFB2, MIF, IL6, TGFB3, TGFB1, IL22,* and *IL10;*

(r) Cancer-associated fibroblast (CAF) group: *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* and *LRP1;*

(s) Matrix group: *LAMC2, TNC, COL11A1, VTN, LAMB3, COL1A1, FN1, LAMA3, LGALS9, COL1A2, COL4A1, COL5A1, ELN, LGALS7,* and *COL3A1;*

(t) Angiogenesis group: *VEGFC, VEGFA, PDGFC, KDR, CDH5, VEGFB, PGF, TEK, ANGPT2, CXCR2, FLT1, CXCL8, VWF, ANGPT1,* and *CXCL5;*

(u) Endothelium group: *KDR, CDH5, NOS3, VCAM1, VWF, FLT1, MMRN1, CLEC14A, ENG,* and *MMRN2;*

(v) Proliferation rate group: *CCND1, CCNB1, CETN3, CDK2, E2F1, AURKA, BUB1, AURKB, PLK1, MCM6, ESCO2, MYBL2, MKI67, MCM2,* and *CCNE1;*

(w) EMT signature group: *CDH2, ZEB1, ZEB2, TWIST1, SNAI1, SNAI2,* and *TWIST2;*

(x) Tertiary Lymphoid Structure (TLS) group: *CXCL13, CCL19, LAMP3, SELL, CXCR4, CCL21, CCR7, CD86,* and *BCL6;*

(y) Follicular dendritic cells group: *FDCSP, SERPINE2, PRNP, PDPN, LTBR, BST1, CLU, C1S,* and *C4A;*

(z) Follicular B helper T cells group: *SH2D1A, IL6, MAF, CD40LG, IL21, ICOS, IL4, CD84, CXCR5,* and *BCL6;* and

(aa) Granulocytes group: *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* and *PRSS33;* and/or

(iv) using a single-sample GSEA (ssGSEA) technique to determine the gene group scores from RNA expression levels for each of the genes in each of the following gene groups:

(a) MHC I group: *HLA-C, TAPBP, HLA-B, B2M, TAP2, HLA-A, TAP1,* and *NLRC5;*

(b) MHC II group: *HLA-DQB1, HLA-DMA, HLA-DMB, HLA-DRA, CIITA, HLA-DQA1, HLA-DPB1, HLA-DRB1,* and *HLA-DPA1;*

(c) Coactivation molecules group: *TNFRSF4, CD27, CD80, CD40LG, TNFRSF9, CD40, CD28, ICOSLG, CD83, TNFSF9, CD70, TNFSF4, ICOS,* and *CD86;*

(d) Effector cells group: *ZAP70, GZMB, GZMK, IFNG, FASLG, EOMES, TBX21, GZMA, CD8A, GNLY, PRF1,* and *CD8B;*

(e) NK cells group: *NKG7, FGFBP2, CD244, KLRK1, KIR2DL4, CD226, KLRF1, GNLY, GZMB, KLRC2, NCR1, GZMH, IFNG, SH2D1B, NCR3, EOMES,* and *CD160;*

(f) T cells group: *TRBC2, CD3E, CD3G, ITK, CD28, TRBC1, TRAT1, TBX21, CD5, TRAC,* and *CD3D;*

(g) B cells group: *CD22, TNFRSF13C, STAP1, CD79B, PAX5, CR2, TNFRSF13B, CD79A, TNFRSF17, FCRL5, MS4A1, CD19,* and *BLK;*

(h) M1 signature group: *CMKLR1, SOCS3, IRF5, NOS2, IL1B, IL12B, IL23A, TNF,* and *IL12A;*

(i) Th1 signature group: *IL21, TBX21, IL12RB2, CD40LG, IFNG, IL2,* and *STAT4;*

(j) Antitumor cytokines group: *CCL3, IL21, IFNB1, IFNA2, TNF,* and *TNFSF10;*

(k) Checkpoint inhibition group: *PDCD1, BTLA, HAVCR2, CD274, VSIR, LAG3, TIGIT, PDCD1LG2,* and *CTLA4;*

(l) Treg group: *IKZF2, TNFRSF18, IL10, FOXP3, CCR8, IKZF4,* and *CTLA4;*

(m) Neutrophil signature group: *CD177, FFAR2, PGLYRP1, CXCR1, MPO, CXCR2, ELANE, CTSG, PRTN3,* and *FCGR3B;*

(n) Granulocyte traffic group: *CCR3, KITLG, CCL11, CXCL2, CXCR1, CXCR2, CXCL8, CXCL1,* and *CXCL5;*

(o) MDSC group: *ARG1, IL6, CYBB, IL10, PTGS2, IDO1,* and *IL4I1;*

(p) Th2 signature group: *IL13, CCR4, IL10, IL4,* and *IL5;*

(q) Protumor cytokines group: *TGFB2, MIF, IL6, TGFB3, TGFB1, IL22,* and *IL10;*

(r) Cancer-associated fibroblast (CAF) group: *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* and *LRP1;*

(s) Matrix group: *LAMC2, TNC, COL11A1, VTN, LAMB3, COL1A1, FN1, LAMA3, LGALS9, COL1A2, COL4A1, COL5A1, ELN, LGALS7,* and *COL3A1;*

(t) Angiogenesis group: *VEGFC, VEGFA, PDGFC, KDR, CDH5, VEGFB, PGF, TEK, ANGPT2, CXCR2, FLT1, CXCL8, VWF, ANGPT1,* and *CXCL5;*

(u) Endothelium group: *KDR, CDH5, NOS3, VCAM1, VWF, FLT1, MMRN1, CLEC14A, ENG,* and *MMRN2;*

(v) Proliferation rate group: *CCND1, CCNB1, CETN3, CDK2, E2F1, AURKA, BUB1, AURKB, PLK1, MCM6, ESCO2, MYBL2, MKI67, MCM2,* and *CCNE1;*

(w) EMT signature group: *CDH2, ZEB1, ZEB2, TWIST1, SNAI1, SNAI2,* and *TWIST2;*

(x) Tertiary Lymphoid Structure (TLS) group: *CXCL13, CCL19, LAMP3, SELL, CXCR4, CCL21, CCR7, CD86,* and *BCL6;*
(y) Follicular dendritic cells group: *FDCSP, SERPINE2, PRNP, PDPN, LTBR, BST1, CLU, C1S,* and *C4A;*
(z) Follicular B helper T cells group: *SH2D1A, IL6, MAF, CD40LG, IL21, ICOS, IL4, CD84, CXCR5,* and *BCL6;* and
(aa) Granulocytes group: *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* and *PRSS33.*

8. The method of any one of claims 1 to 7, wherein generating the BLBC TME signature further comprises normalizing the gene group scores, wherein the normalizing comprises applying median scaling to the gene group scores.

9. The method of any one of claims 1 to 8,

wherein the plurality of BLBC TME types is associated with a respective plurality of BLBC TME signature clusters, wherein identifying, using the BLBC TME signature and from among a plurality of BLBC TME types, the BLBC TME type for the subject comprises:
associating the BLBC TME signature of the subject with a particular one of the plurality of BLBC TME signature clusters; and
identifying the BLBC TME type for the subject as the BLBC TME type corresponding to the particular one of the plurality of BLBC TME signature clusters to which the BLBC TME signature of the subject is associated.

10. The method of claim 9, wherein the method further comprises generating the plurality of BLBC TME signature clusters, the generating comprising:

obtaining multiple sets of RNA expression data by sequencing biological samples from multiple respective subjects, each of the multiple sets of RNA expression data indicating RNA expression levels for at least some genes in each of the at least some of the plurality of gene groups listed in the table below:

| MHC I | *HLA-C, TAPBP, HLA-B, B2M, TAP2, HLA-A, TAP1, NLRC5* |
|---|---|
| MHC II | *HLA-DQB1, HLA-DMA, HLA-DMB, HLA-DRA, CIITA, HLA-DQA1, HLA-DPB1, HLA-DRB1, HLA-OPA 1* |
| Coactivation molecules | *TNFRSF4, CD27, CD80, CD40LG, TNFRSF9, CD40, CD28, ICOSLG, CD83, TNFSF9, CD70, TNFSF4, ICOS, CD86* |
| Effector cells | *ZAP70, GZMB, GZMK, IFNG, FASLG, EOMES, TBX21, GZMA, CD8A, GNLY, PRF1, CD8B* |
| NK cells | *NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160* |
| T cells | *TRBC2, CD3E, CD3G, ITK, CD28, TRBC1, TRAT1, TBX21, CD5, TRAC, CD3D* |
| B cells | *CD22, TNFRSF13C, STAP1, CD79B, PAX5, CR2, TNFRSF13B, CD79A, TNFRSF17, FCRL5, MS4A1, CD19, BLK* |
| M1 signatures | *CMKLR1, SOCS3, IRF5, NOS2, IL1B, IL12B, IL23A, TNF, IL12A* |
| Th1 signature | *IL21, TBX21, IL12RB2, CD40LG, IFNG, IL2, STAT4* |
| Antitumor cytokines | *CCL3, IL21, IFNB1, IFNA2, TNF, TNFSF10* |
| Checkpoint inhibition | *PDCD1, BTLA, HAVCR2, CD274, VSIR, LAG3, TIGIT, PDCD1LG2, CTLA4* |
| Treg | *IKZF2, TNFRSF18, IL10, FOXP3, CCR8, IKZF4, CTLA4* |
| Neutrophil signature | *CD177, FFAR2, PGLYRP1, CXCR1, MPO, CXCR2, ELANE, CTSG, PRTN3, FCGR3B* |
| Granulocyte traffic | *CCR3, KITLG, CCL11, CXCL2, CXCR1, CXCR2, CXCL8, CXCL1, CXCL5* |

(continued)

| | |
|---|---|
| MDSC | ARG1, IL6, CYBB, IL10, PTGS2, IDO1, IL4I1 |
| Macrophages | MRC1, SIGLEC1, MSR1, CD163, CSF1R, CD68, IL4I1, IL10 |
| Th2 signature | IL13, CCR4, IL10, IL4, IL5 |
| Protumor cytokines | TGFB2, MIF, IL6, TGFB3, TGFB1, IL22, IL10 |
| CAF | COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, LRP1 |
| Matrix | LAMC2, TNC, COL11A1, VTN, LAMB3, COL1A1, FN1, LAMA3, LGALS9, COL1A2, COL4A1, COL5A1, ELN, LGALS7, COL3A1 |
| Angiogenesis | VEGFC, VEGFA, PDGFC, KDR, CDH5, VEGFB, PGF, TEK, ANGPT2, CXCR2, FLT1, CXCL8, VWF, ANGPT1, CXCL5 |
| Endothelium | KDR, CDH5, NOS3, VCAM1, VWF, FLT1, MMRN1, CLEC14A, ENG, MMRN2 |
| Proliferation rate | CCND1, CCNB1, CETN3, CDK2, E2F1, AURKA, BUB1, AURKB, PLK1, MCM6, ESCO2, MYBL2, MKI67, MCM2, CCNE1 |
| EMT signature | CDH2, ZEB1, ZEB2, TWIST1, SNAI1, SNAI2, TWIST2 |
| TLS | CXCL13, CCL19, LAMP3, SELL, CXCR4, CCL21, CCR7, CD86, BCL6 |
| Follicular dendritic cells | FDCSP, SERPINE2, PRNP, PDPN, LTBR, BST1, CLU, C1S, C4A |
| Follicular B helper T cells | SH2D1A, IL6, MAF, CD40LG, IL21, ICOS, IL4, CD84, CXCR5, BCL6 |
| Granulocytes | CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, PRSS33 |

wherein the at least some of the plurality of gene groups comprise the granulocytes gene group, the angiogenesis gene group, the B-cells gene group, the M1 signatures gene group, and the endothelium gene group; generating multiple BLBC TME signatures from the multiple sets of RNA expression data, each of the multiple BLBC TME signatures comprising gene group expression scores for respective gene groups in the plurality of gene groups, the generating comprising, for each particular one of the multiple BLBC TME signatures:

determining the BLBC TME signature by determining the gene group expression scores using the RNA expression levels in the particular set of RNA expression data for which the particular one BLBC TME signature is being generated; and
clustering the multiple BLBC signatures to obtain the plurality of BLBC TME signature clusters.

11. The method of any of claims 8 to 10, further comprising:
updating the plurality of BLBC TME signature clusters using the BLBC TME signature of the subject, wherein the BLBC TME signature of the subject is one of a threshold number BLBC TME signatures for a threshold number of subjects, wherein when the threshold number of BLBC TME signatures is generated the BLBC TME signature clusters are updated,
wherein the threshold number of BLBC TME signatures is at least 50, at least 75, at least 100, at least 200, at least 500, at least 1000, or at least 5000 BLBC TME signatures.

12. The method of claim 11, wherein the updating is performed using a clustering algorithm selected from the group consisting of a dense clustering algorithm, spectral clustering algorithm, k-means clustering algorithm, hierarchical clustering algorithm, and an agglomerative clustering algorithm.

13. The method of any one of claims 8 to 12, further comprising:
determining an BLBC TME type of a second subject, wherein the BLBC TME type of the second subject is identified using the updated BLBC TME signature clusters, wherein the identifying comprises:

determining an BLBC TME signature of the second subject from RNA expression data obtained by sequencing a biological sample obtained from the second subject;

associating the BLBC TME signature of the second subject with a particular one of the plurality of the updated BLBC TME signature clusters; and

identifying the BLBC TME type for the second subject as the BLBC TME type corresponding to the particular one of the plurality of updated BLBC TME signature clusters to which the BLBC TME signature of the second subject is associated.

14. A system, comprising:

at least one computer hardware processor; and

at least one non-transitory computer readable medium storing processor-executable instructions that, when executed by the at least one computer hardware processor, cause the at least one computer hardware processor to perform the method of any one of claims 1 to 13.

15. At least one non-transitory computer readable medium storing processor-executable instructions that, when executed by at least one computer hardware processor, cause the at least one computer hardware processor to perform the method of any one of claims 1 to 13.

**Patentansprüche**

1. Verfahren zur Bestimmung des Typs der Tumor-Mikroumgebung (MTE) von basalem Brustkrebs ("basal-like breast cancer", BLBC) bei einem Individuum mit basalem Brustkrebs, wobei das Verfahren Folgendes umfasst:

das Verwenden zumindest eines Computerhardware-Prozessors, um Folgendes auszuführen:

das Erhalten von RNA-Expressionsdaten für das Individuum, wobei die RNA-Expressionsdaten die RNA-Expressionsniveaus für zumindest manche Gene in jeder Gruppe von zumindest manchen aus einer Vielzahl von Gengruppen, die in der nachstehenden Tabelle aufgelistet sind, in einer biologischen Probe angeben, die Tumorgewebe des Individuums umfasst:

| MHC I | HLA-C, TAPBP, HLA-B, B2M, TAP2, HLA-A, TAP1, NLRCS |
|---|---|
| MHC II | HLA-DQB1, HLA-DMA, HLA-DMB, HLA-DRA, CIITA, HLA-DQA1, HLA-DPB1, HLA-DRB1, HLA-DPA1 |
| Koaktivierungsmoleküle | TNFRSF4, CD27, CD80, CD40LG, TNFRSF9, CD40, CD28, ICOSLG, CD83, TNFSF9, CD70, TNFSF4, ICOS, CD86 |
| Effektorzellen | ZAP70, GZMB, GZMK, IFNG, FASLG, EOMES, TBX21, GZMA, CD8A, GNLY, PRF1, CD8B |
| NK-Zellen | NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160 |
| T-Zellen | TRBC2, CD3E, CD3G, ITK, CD28, TRBC1, TRAT1, TBX21, CD5, TRAC, CD3D |
| B-Zellen | CD22, TNFRSF13C, STAP1, CD79B, PAXS, CR2, TNFRSF13B, CD79A, TNFRSF17, FCRL5, MS4A1, CD19, BLK |
| M1-Signaturen | CMKLR1, SOCS3, IRF5, NOS2, IL1B, 1L12B, IL23A, TNF, IL12A |
| Th1-Signatur | IL21, TBX21, IL12RB2, CD40LG, IFNG, IL2, STAT4 |
| Antitumor-Cytokine | CCL3, IL21, IFNB1, IFNA2, TNF, TNFSF10 |
| Checkpoint-Inhibition | PDCD1, BTLA, HAVCR2, CD274, VSIR, LAG3, TIGIT, PDCD1LG2, CTLA4 |
| Treg | IKZF2, TNFRSF18, IL10, FOXP3, CCR8, IKZF4, CTLA4 |
| Neutrophilen-Signatur | CD177, FFAR2, PGLYRP1, CXCR1, MPO, CXCR2, ELANE, CTSG, PRTN3, FCGR3B |

(continued)

| Granulozyten-Wanderung | CCR3, KITLG, CCL11, CXCL2, CXCR1, CXCR2, CXCL8, CXCL1, CXCL5 |
|---|---|
| MDSC | ARG1, IL6, CYBB, IL10, PTGS2, IDO1, IL411 |
| Makrophagen | MRC1, SIGLEC1, MSR1, CD163, CSF1R, CD68, IL4I1, IL10 |
| Th2-Signatur | IL13, CCR4, IL10, IL4, ILS |
| Protumor-Cytokine | TGFB2, MIF, IL6, TGFB3, TGFB1, IL22, IL10 |
| CAF | COL6A3, PDGFRB, COL6A1, MFAPS, COL5A1, FAP, PDGFRA, FGF2, ACTA2, COL6A2, FBLN1, CD248, COL1A1, MMP2, COL1A2, MMP3, LUM, CXCL12, LRP1 |
| Matrix | LAMC2, TNC, COL11A1, VTN, LAMB3, COL1A1, FN1, LAMA3, LGALS9, COL1A2, COL4A1, COLSA1, ELN, LGALS7, COL3A1 |
| Angiogenese | VEGFC, VEGFA, PDGFC, KDR, CDH5, VEGFB, PGF, TEK, ANGPT2, CXCR2, FLT1, CXCL8, VWF, ANGPT1, CXCL5 |
| Endothel | KDR, CDHS, NOS3, VCAM1, VWF, FLT1, MMRN1, CLEC14A, ENG, MMRN2 |
| Proliferationsrate | CCND1, CCNB1, CETN3, CDK2, E2F1, AURKA, BUB1, AURKB, PLK1, MCM6, ESCO2, MYBL2, MKI67, MCM2, CCNE1 |
| EMT-Signatur | CDH2, ZEB1, ZEB2, TWIST1, SNAI1, SNAI2, TWIST2 |
| TLS | CXCL13, CCL19, LAMP3, SELL, CXCR4, CCL21, CCR7, CD86, BCL6 |
| follikuläre dendritische Zellen | FDCSP, SERPINE2, PRNP, PDPN, LTBR, BST1, CLU, C1S, C4A |
| follikuläre B-Helfer-T-Zellen | SH2D1A, IL6, MAF, CD40LG, IL21, ICOS, IL4, CD84, CKCR5, BCL6 |
| Granulozyten | CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATAT1, EPX, IL13, MPO, IL5RA, CTSG, CXCL5, PRG2, CD177, CPA3, CCL11, PGLYRP1, MS4A2, IL4, PRSS33 |

wobei die RNA-Expressionsdaten die RNA-Expressionsniveaus für zumindest drei Gene aus jeder zumindest der Folgenden angegeben: die Granylozyten-Gengruppe, die Angiogenese-Gengruppe, die B-Zellen-Gengruppe, die M1-Signatur-Gengruppe und die Endothel-Gengruppe; und

das Erzeugen einer BLBC-TME-Signatur für das Individuum unter Verwendung der RNA-Expressionsdaten, wobei die BLBC-TME-Signatur Gengruppen-Werte für jeweilige Gengruppen in den zumindest manchen der Vielzahl von Gengruppen umfassen, wobei das Erzeugen Folgendes umfasst:

das Bestimmen von Gengruppen-Werten unter Verwendung der RNA-Expressionsniveaus und einer Gensatz-Anreicherungs-Analysemethode; und
das Identifizieren eines BLBC-TME-Typs für das Individuum aus einer Vielzahl von BLBC-TME-Typen unter Verwendung der BLBC-TME-Signatur, wobei die Vielzahl von BLBC-TME-Typen die folgenden umfasst: einen immunangereicherten Typ (IE), einen TLS-(TLS-) Typ, einen Desert- (D-) Typ, einen fibrotischen (F-) Typ und einen Granulozytenangereicherten (G-) Typ, und wobei:

(i) der BLBC-Typ G die höchsten Granulozyten- und M1-Signatur-Gengruppen-Werte der Vielzahl von BLBC-TME-Typen aufweist,
(ii) die Typen G und F höhere Angiogenese-Gengruppen-Werte aufweisen als die anderen BLBC-TME-Typen der Vielzahl von BLBC-TME-Typen,
(iii) der TLS-Typ den höchsten B-Zellen-Gengruppen-Wert der Vielzahl von BLBC-TME-Typen aufweist und
(iv) die Typen TLS und F die höchsten Endothel-Expressionsniveau-Gengruppen-Werte der Vielzahl von BLBC-TME-Typen aufweisen.

2. Verfahren nach Anspruch 1, wobei: (1) das Erhalten der RNA-Expressionsdaten für das Individuum das Erhalten von Sequenzierungsdaten umfasst, die zuvor durch Sequenzieren einer von dem Individuum erhaltenen biologischen Probe erhalten wurden, wobei die biologische Probe Tumorgewebe vom Individuum umfasst, und/oder (ii) das Verfahren weiters das Normalisieren der RNA-Expressionsdaten auf Transkripte pro Million (TPM) Einheiten vor der Erzeugung der BLBC-TME-Signatur umfasst.

3. Verfahren nach Anspruch 2, wobei die Sequenzierungsdaten Folgendes umfassen:

(a) zumindest 1 Million Ablesungen, zumindest 5 Millionen Ablesungen, zumindest 10 Millionen Ablesungen, zumindest 20 Millionen Ablesungen, zumindest 50 Millionen Ablesungen oder zumindest 100 Millionen Ablesungen; und/oder

(b) Ganz-Exom-Sequenzierungs- (WES-) Daten, Bulk-RNA-Sequenzierungs- (RNA-seq-) Daten, Einzelzellen-RNA-Sequenzierungs- (scRNA-seq-) Daten und/oder Next-Generation-Sequenzierungs- (NGS-) Daten; oder

(c) Microarray-Daten.

4. Verfahren nach einem der Ansprüche 1 bis 3, wobei das Erhalten der RNA-Expressionsdaten für das Individuum das Sequenzieren einer von dem Individuum erhaltenen biologischen Probe umfasst, wobei die biologische Probe Tumorgewebe von dem Individuum umfasst.

5. Verfahren nach einem der Ansprüche 1 bis 4, wobei die RNA-Expressionsniveaus RNA-Expressionsniveaus für zumindest drei Gene aus jeder der folgenden Gengruppen oder RNA-Expressionsniveaus für jedes der Gene aus jeder der folgenden Gengruppen umfassen:

(a) MHC-I-Gruppe: HLA-C, TAPBP, HLA-B, B2M, TAP2, HLA-A, TAP1 und NLRC5;

(b) MHC-II-Gruppe: HLA-DQB1, HLA-DMA, HLA-DMB, HLA-DRA, CIITA, HLA-DQA1, HLA-DPB1, HLA-DRB1 und HLA-DPA1;

(c) Koaktivierungsmolekül-Gruppe: TNFRSF4, CD27, CD80, CD40LG, TNFRSP9, CD40, CD28, ICOSLG, CD83, TNFSF9, CD70, TNFSF4, ICOS und CD86;

(d) Effektorzellen-Gruppe: ZAP70, GZMB, GZMK, IFNG, FASLG, EOMES, TBX21, GZMA, CD8A, GNLY, PRF1 und CD8B;

(e) NK-Zellen-Gruppe: NKG7, FGFBP2, CD244, KLRK1, KIR2DL4, CD226, KLRF1, GNLY, GZMB, KLRC2, NCR1, GZMH, IFNG, SH2D1B, NCR3, EOMES und CD160;

(f) T-Zellen-Gruppe: TRBC2, CD3E, CD3G, ITK, CD28, TRBC1, TRAT1, TBX21, CD5, TRAC und CD3D;

(g) B-Zellen-Gruppe: CD22, TNFRSF13C, STAP1, CD79B, PAX5, CR2, TNFRSF13B, CD79A, TNFRSF17, FCRL5, MS4A1, CD19 und BLK;

(h) M1-Signatur-Gruppe: CMKLR1, SOCS3, IRF5, NOS2, IL1B, IL12B, IL23A, TNF und IL12A;

(i) Th1-Signatur-Gruppe: IL21, TBX21, IL12RB2, CD40LG, IFNG, IL2 und STAT4;

(j) Antitumor-Cytokin-Gruppe: CCL3, IL21, IFNB1, IFNA2, TNF und TNFSF10;

(k) Checkpoint-Inhibitions-Gruppe: PDCD1, BTLA, HAVCR2, CD274, VSIR, LAG3, TIGIT, PDCD1LG2 und CTLA4,

(l) Treg-Gruppe: IKZF2, TNFRSF18, IL10, FOXP3, CCR8, IKZF4 und CTLA4;

(m) Neutrophilen-Signatur-Gruppe: CD177, FFAR2, PGLYRP1, CXCR1, MPO, CXCR2, ELANE, CTSG, PRTN3 und FCGR3B;

(n) Granulozyten-Wanderungs-Gruppe: CCR3, KITLG, CCL11, CXCL2, CXCR1, CXCR2, CXCL8, CXCL1 und CXCL5;

(o) MDSC-Gruppe: ARG1, IL6, CYBB, IL10, PTGS2, IDO1 und IL411;

(p) Th2-Signatur-Gruppe: IL13, CCR4, IL10, IL4 und IL5;

(q) Protumor-Cytokin-Gruppe: TGFB2, MIF, IL6, TGFB3, TGFB1, IL22 und IL10;

(r) Gruppe der krebsassoziierten Fibroblasten (CAF): COL6A3, PDGFRB, COL6A1, MPFAP5, COL5A1, FAP, PDGFRA, FGF2, ACTA2, COL6A2, FBLN1, CD248, COL1A1, MMP2, COL1A2, MMP3, LUM, CXCL12 und LRP1;

(s) Matrix-Gruppe: LAMC2, TNC, COL11A1, VTN, LAMB3, COL1A1, FN1, LAMA3, LGALS9, COL1A2, COL4A1, COL5A1, ELN, LGALS7 und COL3A1,

(t) Angiogenese-Gruppe: VEGFC, VEGFA, PDGFC, KDR, CDH5, VEGFB, PGF, TEK, ANGPT2, CXCR2, FLT1, CXCL8, VWF, ANGPT1 und CXCL5;

(u) Endothel-Gruppe: KDR, CDH5, NOS3, VCAM1, VWF, FLT1, MMRN1, CLEC14A, ENG und MMRN2;

(v) Proliferationsraten-Gruppe: CCND1, CCNB1, CETN3, CDK2, E2F1, AURKA, BUB1, AURKB, PLK1, MCM6, ESCO2, MYBL2, MKI67, MCM2 und CCNE1;

(w) EMT-Signatur-Gruppe: CDH2, ZEB1, ZEB2, TWIST1, SNAI1, SNAI2 und TWIST2;

(x) Gruppe der tertiären Lymphstruktur (TLS): CXCL13, CCL19, LAMP3, SELL, CXCR4, CCL21, CCR7, CD86 und BCL6;

(y) Gruppe der follikulären dendritischen Zellen: FDCSP, SERPINE2, PRNP, PDPN, LTBR, BST1, CLU, C1S und C4A;

(z) Gruppe der follikulären Helfer-T-Zellen: SH2D1A, IL6, MAF, CD40LG, IL21, ICOS, IL4, CD84, CXCR5 und BCL6; und

(aa) Granulozyten-Gruppe: CCR3, FFAR2, CXCL2, CMA1, SIGLECS8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4 und PRSS33.

6. Verfahren nach einem der Ansprüche 1 bis 5, wobei das Bestimmen der Gengruppen-Werte Folgendes umfasst:

(i) das Bestimmen eines jeweiligen Gengruppen-Werts für jede von zumindest sechs der folgenden Gengruppen, einschließlich der Granulozyten-Gengruppe, der Angiogenese-Gengruppe, der B-Zellen-Gengruppe, der M1-Signatur-Gengruppe und der Endothel-Gengruppe, unter Verwendung von, für eine bestimmte Gengruppe, RNA-Expressionsniveaus für zumindest drei Gene in der bestimmten Gengruppe, um den Gengruppen-Wert für die bestimmte Gruppe zu bestimmen, wobei die Gengruppen die folgenden umfassen:

(a) MHC-I-Gruppe: HLA-C, TAPBP, HLA-B, B2M, TAP2, HLA-A, TAP1 und NLRC5;

(b) MHC-II-Gruppe: HLA-DQB1, HLA-DMA, HLA-DMB, HLA-DRA, CIITA, HLA-DQA1, HLA-DPB1, HLA-DRB1 und HLA-DPA1;

(c) Koaktivierungsmolekül-Gruppe: TNFRSF4, CD27, CD80, CD40LG, TNFRSP9, CD40, CD28, ICOSLG, CD83, TNFSF9, CD70, TNFSF4, ICOS und CD86;

(d) Effektorzellen-Gruppe: ZAP70, GZMB, GZMK, IFNG, FASLG, EOMES, TBX21, GZMA, CD8A, GNLY, PRF1 und CD8B;

(e) NK-Zellen-Gruppe: NKG7, FGFBP2, CD244, KLRK1, KIR2DL4, CD226, KLRF1, GNLY, GZMB, KLRC2, NCR1, GZMH, IFNG, SH2D1B, NCR3, EOMES und CD160;

(f) T-Zellen-Gruppe: TRBC2, CD3E, CD3G, ITK, CD28, TRBC1, TRAT1, TBX21, CD5, TRAC und CD3D;

(g) B-Zellen-Gruppe: CD22, TNFRSF13C, STAP1, CD79B, PAX5, CR2, TNFRSF13B, CD79A, TNFRSF17, FCRL5, MS4A1, CD19 und BLK;

(h) M1-Signatur-Gruppe: CMKLR1, SOCS3, IRF5, NOS2, IL1B, IL12B, IL23A, TNF und IL12A;

(i) Th1-Signatur-Gruppe: IL21, TBX21, IL12RB2, CD40LG, IFNG, IL2 und STAT4;

(j) Antitumor-Cytokin-Gruppe: CCL3, IL21, IFNB1, IFNA2, TNF und TNFSF10;

(k) Checkpoint-Inhibitions-Gruppe: PDCD1, BTLA, HAVCR2, CD274, VSIR, LAG3, TIGIT, PDCD1LG2 und CTLA4;

(l) Treg-Gruppe: IKZF2, TNFRSF18, IL10, FOXP3, CCR8, IKZF4 und CTLA4;

(m) Neutrophilen-Signatur-Gruppe: CD177, FFAR2, PGLYRP1, CXCR1, MPO, CXCR2, ELANE, CTSG, PRTN3 und FCGR3B;

(n) Granulozyten-Wanderungs-Gruppe: CCR3, KITLG, CCL11, CXCL2, CXCR1, CXCR2, CXCL8, CXCL1 und CXCL5;

(o) MDSC-Gruppe: ARG1, IL6, CYBB, IL10, PTGS2, IDO1 und IL411;

(p) Th2-Signatur-Gruppe: IL13, CCR4, IL10, IL4 und IL5;

(q) Protumor-Cytokin-Gruppe: TGFB2, MIF, IL6, TGFB3, TGFB1, IL22 und IL 10;

(r) Gruppe der krebsassoziierten Fibroblasten (CAF): COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12 und LRP1;

(s) Matrix-Gruppe: LAMC2, TNC, COL11A1, VTN, LAMB3, COL1A1, FN1, LAMA3, LGALS9, COL1A2, COL4A1, COL5A1, ELN, LGALS7 und COL3A1;

(t) Angiogenese-Gruppe: VEGFC, VEGFA, PDGFC, KDR, CDH5, VEGFB, PGF, TEK, ANGPT2, CXCR2, FLT1, CXCL8, VWF, ANGPT1 und CXCL5;

(u) Endothel-Gruppe: KDR, CDH5, NOS3, VCAM1, VWF, FLT1, MMRN1, CLEC14A, ENG und MMRN2;

(v) Proliferationsraten-Gruppe: CCND1, CCNB1, CETN3, CDK2, E2F1, AURKA, BUB1, AURKB, PLK1, MCM6, ESCO2, MYBL2, MKI67, MCM2 und CCNE1;

(w) EMT-Signatur-Gruppe: CDH2, ZEB1, ZEB2, TWIST1, SNAI1, SNAI2 und TWIST2;

(x) Gruppe der tertiären Lymphstruktur (TLS): CXCL13, CCL19, LAMP3, SELL, CXCR4, CCL21, CCR7, CD86 und BCL6;

(y) Gruppe der follikulären dendritischen Zellen: FDCSP, SERPINE2, PRNP, PDPN, LTBR, BST1, CLU, C1S und C4A;

(z) Gruppe der follikulären Helfer-T-Zellen: SH2D1A, IL6, MAF, CD40LG, IL21, ICOS, IL4, CD84, CXCRS5 und BCL6; und

(aa) Granulozyten-Gruppe: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4 und PRSS33.

**7.** Verfahren nach einem der vorangegangenen Ansprüche, wobei das Bestimmen der Gengruppen-Werte Folgendes umfasst:

(i) das Bestimmen eines jeweiligen Gengruppen-Werts für jede der folgenden Gengruppen unter Verwendung von, für eine bestimmte Gengruppe, RNA-Expressionsniveaus für jedes der Gene in der jeweiligen Gengruppe, um den Gengruppen-Wert für jede bestimmte Gruppe zu bestimmen, wobei die Gengruppen die folgenden umfassen:

(a) MHC-I-Gruppe: HLA-C, TAPBP, HLA-B, B2M, TAP2, HLA-A, TAP1 und NLRC5;
(b) MHC-II-Gruppe: HLA-DQB1, HLA-DMA, HLA-DMB, HLA-DRA, CIITA, HLA-DQA1, HLA-DPB1, HLA-DRB1 und HLA-DPA1;
(c) Koaktivierungsmolekül-Gruppe: TNFRSF4, CD27, CD80, CD40LG, TNFRSP9, CD40, CD28, ICOSLG, CD83, TNFSF9, CD70, TNFSF4, ICOS und CD86;
(d) Effektorzellen-Gruppe: ZAP70, GZMB, GZMK, IFNG, FASLG, EOMES, TBX21, GZMA, CD8A, GNLY, PRF1 und CD8B;
(e) NK-Zellen-Gruppe: NKG7, FGFBP2, CD244, KLRK1, KIR2DL4, CD226, KLRF1, GNLY, GZMB, KLRC2, NCR1, GZMH, IFNG, SH2D1B, NCR3, EOMES und CD160;
(f) T-Zellen-Gruppe: TRBC2, CD3E, CD3G, ITK, CD28, TRBC1, TRAT1, TBX21, CD5, TRAC und CD3D;
(g) B-Zellen-Gruppe: CD22, TNFRSF13C, STAP1, CD79B, PAX5, CR2, TNFRSF13B, CD79A, TNFRSF17, FCRL5, MS4A1, CD19 und BLK;
(h) M1-Signatur-Gruppe: CMKLR1, SOCS3, IRF5, NOS2, IL1B, IL12B, IL23A, TNF und IL12A;
(i) Th1-Signatur-Gruppe: IL21, TBX21, IL12RB2, CD40LG, IFNG, IL2 und STAT4;
(j) Antitumor-Cytokin-Gruppe: CCL3, IL21, IFNB1, IFNA2, TNF und TNFSF10;
(k) Checkpoint-Inhibitions-Gruppe: PDCD1, BTLA, HAVCR2, CD274, VSIR, LAG3, TIGIT, PDCD1LG2 und CTLA4;
(l) Treg-Gruppe: IKZF2, TNFRSF18, IL10, FOXP3, CCR8, IKZF4 und CTLA4;
(m) Neutrophilen-Signatur-Gruppe: CD177, FFAR2, PGLYRP1, CXCR1, MPO, CXCR2, ELANE, CTSG, PRTN3 und FCGR3B;
(n) Granulozyten-Wanderungs-Gruppe: CCR3, KITLG, CCL11, CXCL2, CXCR1, CXCR2, CXCL8, CXCL1 und CXCL5;
(o) MDSC-Gruppe: ARG1, IL6, CYBB, IL10, PTGS2, IDO1 und IL4I1;
(p) Th2-Signatur-Gruppe: IL13, CCR4, IL10, IL4 und IL5;
(q) Protumor-Cytokin-Gruppe: TGFB2, MIF, IL6, TGFB3, TGFB1, IL22 und IL 10;
(r) Gruppe der krebsassoziierten Fibroblasten (CAF): COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12 und LRP1;
(s) Matrix-Gruppe: LAMC2, TNC, COL11A1, VTN, LAMB3, COL1A1, FN1, LAMA3, LGALS9, COL1A2, COL4A1, COL5A1, ELN, LGALS7 und COL3A1;
(t) Angiogenese-Gruppe: VEGFC, VEGFA, PDGFC, KDR, CDH5, VEGFB, PGF, TEK, ANGPT2, CXCR2, FLT1, CXCL8, VWF, ANGPT1 und CXCL5;
(u) Endothel-Gruppe: KDR, CDH5, NOS3, VCAM1, VWF, FLT1, MMRN1, CLEC14A, ENG und MMRN2;
(v) Proliferationsraten-Gruppe: CCND1, CCNB1, CETN3, CDK2, E2F1, AURKA, BUB1, AURKB, PLK1, MCM6, ESCO2, MYBL2, MKI67, MCM2 und CCNE1;
(w) EMT-Signatur-Gruppe: CDH2, ZEB1, ZEB2, TWIST1, SNAI1, SNAI2 und TWIST2;
(x) Gruppe der tertiären Lymphstruktur (TLS): CXCL13, CCL19, LAMP3, SELL, CXCR4, CCL21, CCR7, CD86 und BCL6;
(y) Gruppe der follikulären dendritischen Zellen: FDCSP, SERPINE2, PRNP, PDPN, LTBR, BST1, CLU, C1S und C4A;
(z) Gruppe der follikulären Helfer-T-Zellen: SH2D1A, IL6, MAF, CD40LG, IL21, ICOS, IL4, CD84, CXCRS5 und BCL6; und
(aa) Granulozyten-Gruppe: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4 und PRSS33; und/oder

(ii) das Bestimmen eines ersten Werts einer ersten Gengruppe unter Verwendung einer Einzelproben-Gengruppen-Anreicherungsanalyse-Methode (ssGSEA) aus RNA-Expressionsniveaus für zumindest manche der Gene in einer der folgenden Gengruppen:

(a) MHC-I-Gruppe: HLA-C, TAPBP, HLA-B, B2M, TAP2, HLA-A, TAP1 und NLRC5;
(b) MHC-II-Gruppe: HLA-DQB1, HLA-DMA, HLA-DMB, HLA-DRA, CIITA, HLA-DQA1, HLA-DPB1, HLA-DRB1 und HLA-DPA1;
(c) Koaktivierungsmolekül-Gruppe: TNFRSF4, CD27, CD80, CD40LG, TNFRSP9, CD40, CD28, ICOSLG, CD83, TNFSF9, CD70, TNFSF4, ICOS und CD86;
(d) Effektorzellen-Gruppe: ZAP70, GZMB, GZMK, IFNG, FASLG, EOMES, TBX21, GZMA, CD8A, GNLY, PRF1 und CD8B;
(e) NK-Zellen-Gruppe: NKG7, FGFBP2, CD244, KLRK1, KIR2DL4, CD226, KLRF1, GNLY, GZMB, KLRC2, NCR1, GZMH, IFNG, SH2D1B, NCR3, EOMES und CD160;
(f) T-Zellen-Gruppe: TRBC2, CD3E, CD3G, ITK, CD28, TRBC1, TRAT1, TBX21, CD5, TRAC und CD3D;
(g) B-Zellen-Gruppe: CD22, TNFRSF13C, STAP1, CD79B, PAX5, CR2, TNFRSF13B, CD79A, TNFRSF17, FCRL5, MS4A1, CD19 und BLK;
(h) M1-Signatur-Gruppe: CMKLR1, SOCS3, IRF5, NOS2, IL1B, IL12B, IL23A, TNF und IL12A;
(i) Th1-Signatur-Gruppe: IL21, TBX21, IL12RB2, CD40LG, IFNG, IL2 und STAT4;
(j) Antitumor-Cytokin-Gruppe: CCL3, IL21, IFNB1, IFNA2, TNF und TNFSF10;
(k) Checkpoint-Inhibitions-Gruppe: PDCD1, BTLA, HAVCR2, CD274, VSIR, LAG3, TIGIT, PDCD1LG2 und CTLA4;
(l) Treg-Gruppe: IKZF2, TNFRSF18, IL10, FOXP3, CCR8, IKZF4 und CTLA4;
(m) Neutrophilen-Signatur-Gruppe: CD177, FFAR2, PGLYRP1, CXCR1, MPO, CXCR2, ELANE, CTSG, PRTN3 und FCGR3B;
(n) Granulozyten-Wanderungs-Gruppe: CCR3, KITLG, CCL11, CXCL2, CXCR1, CXCR2, CXCL8, CXCL1 und CXCL5;
(o) MDSC-Gruppe: ARG1, IL6, CYBB, IL10, PTGS2, IDO1 und IL4I1;
(p) Th2-Signatur-Gruppe: IL13, CCR4, IL10, IL4 und IL5;
(q) Protumor-Cytokin-Gruppe: TGFB2, MIF, IL6, TGFB3, TGFB1, IL22 und IL 10;
(r) Gruppe der krebsassoziierten Fibroblasten (CAF): COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12 und LRP1;
(s) Matrix-Gruppe: LAMC2, TNC, COL11A1, VTN, LAMB3, COL1A1, FN1, LAMA3, LGALS9, COL1A2, COL4A1, COL5A1, ELN, LGALS7 und COL3A1;
(t) Angiogenese-Gruppe: VEGFC, VEGFA, PDGFC, KDR, CDH5, VEGFB, PGF, TEK, ANGPT2, CXCR2, FLT1, CXCL8, VWF, ANGPT1 und CXCL5;
(u) Endothel-Gruppe: KDR, CDH5, NOS3, VCAM1, VWF, FLT1, MMRN1, CLEC14A, ENG und MMRN2;
(v) Proliferationsraten-Gruppe: CCND1, CCNB1, CETN3, CDK2, E2F1, AURKA, BUB1, AURKB, PLK1, MCM6, ESCO2, MYBL2, MKI67, MCM2 und CCNE1;
(w) EMT-Signatur-Gruppe: CDH2, ZEB1, ZEB2, TWIST1, SNAI1, SNAI2 und TWIST2;
(x) Gruppe der tertiären Lymphstruktur (TLS): CXCL13, CCL19, LAMP3, SELL, CXCR4, CCL21, CCR7, CD86 und BCL6;
(y) Gruppe der follikulären dendritischen Zellen: FDCSP, SERPINE2, PRNP, PDPN, LTBR, BST1, CLU, C1S und C4A;
(z) Gruppe der follikulären Helfer-T-Zellen: SH2D1A, IL6, MAF, CD40LG, IL21, ICOS, IL4, CD84, CXCRS5 und BCL6; und
(aa) Granulozyten-Gruppe: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4 und PRSS33; und/oder

(iv) das Verwenden einer Einzelproben-GSEA-Methode (ssGSEA), um die Gengruppen-Werte aus RNA-Expressionsniveaus für jedes der Gene in jeder der folgenden Gengruppen zu bestimmen:

(a) MHC-I-Gruppe: HLA-C, TAPBP, HLA-B, B2M, TAP2, HLA-A, TAP1 und NLRC5;
(b) MHC-II-Gruppe: HLA-DQB1, HLA-DMA, HLA-DMB, HLA-DRA, CIITA, HLA-DQA1, HLA-DPB1, HLA-DRB1 und HLA-DPA1;

(c) Koaktivierungsmolekül-Gruppe: TNFRSF4, CD27, CD80, CD40LG, TNFRSP9, CD40, CD28, ICOSLG, CD83, TNFSF9, CD70, TNFSF4, ICOS und CD86;

(d) Effektorzellen-Gruppe: ZAP70, GZMB, GZMK, IFNG, FASLG, EOMES, TBX21, GZMA, CD8A, GNLY, PRF1 und CD8B;

(e) NK-Zellen-Gruppe: NKG7, FGFBP2, CD244, KLRK1, KIR2DL4, CD226, KLRF1, GNLY, GZMB, KLRC2, NCR1, GZMH, IFNG, SH2D1B, NCR3, EOMES und CD160;

(f) T-Zellen-Gruppe: TRBC2, CD3E, CD3G, ITK, CD28, TRBC1, TRAT1, TBX21, CD5, TRAC und CD3D;

(g) B-Zellen-Gruppe: CD22, TNFRSF13C, STAP1, CD79B, PAX5, CR2, TNFRSF13B, CD79A, TNFRSF17, FCRL5, MS4A1, CD19 und BLK;

(h) M1-Signatur-Gruppe: CMKLR1, SOCS3, IRF5, NOS2, IL1B, IL12B, IL23A, TNF und IL12A;

(i) Th1-Signatur-Gruppe: IL21, TBX21, IL12RB2, CD40LG, IFNG, IL2 und STAT4;

(j) Antitumor-Cytokin-Gruppe: CCL3, IL21, IFNB1, IFNA2, TNF und TNFSF10;

(k) Checkpoint-Inhibitions-Gruppe: PDCD1, BTLA, HAVCR2, CD274, VSIR, LAG3, TIGIT, PDCD1LG2 und CTLA4;

(l) Treg-Gruppe: IKZF2, TNFRSF18, IL10, FOXP3, CCR8, IKZF4 und CTLA4;

(m) Neutrophilen-Signatur-Gruppe: CD177, FFAR2, PGLYRP1, CXCR1, MPO, CXCR2, ELANE, CTSG, PRTN3 und FCGR3B;

(n) Granulozyten-Wanderungs-Gruppe: CCR3, KITLG, CCL11, CXCL2, CXCR1, CXCR2, CXCL8, CXCL1 und CXCL5;

(o) MDSC-Gruppe: ARG1, IL6, CYBB, IL10, PTGS2, IDO1 und IL4I1;

(p) Th2-Signatur-Gruppe: IL13, CCR4, IL10, IL4 und IL5;

(q) Protumor-Cytokin-Gruppe: TGFB2, MIF, IL6, TGFB3, TGFB1, IL22 und IL 10;

(r) Gruppe der krebsassoziierten Fibroblasten (CAF): COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12 und LRP1;

(s) Matrix-Gruppe: LAMC2, TNC, COL11A1, VTN, LAMB3, COL1A1, FN1, LAMA3, LGALS9, COL1A2, COL4A1, COL5A1, ELN, LGALS7 und COL3A1;

(t) Angiogenese-Gruppe: VEGFC, VEGFA, PDGFC, KDR, CDH5, VEGFB, PGF, TEK, ANGPT2, CXCR2, FLT1, CXCL8, VWF, ANGPT1 und CXCL5;

(u) Endothel-Gruppe: KDR, CDH5, NOS3, VCAM1, VWF, FLT1, MMRN1, CLEC14A, ENG und MMRN2;

(v) Proliferationsraten-Gruppe: CCND1, CCNB1, CETN3, CDK2, E2F1, AURKA, BUB1, AURKB, PLK1, MCM6, ESCO2, MYBL2, MKI67, MCM2 und CCNE1;

(w) EMT-Signatur-Gruppe: CDH2, ZEB1, ZEB2, TWIST1, SNAI1, SNAI2 und TWIST2;

(x) Gruppe der tertiären Lymphstruktur (TLS): CXCL13, CCL19, LAMP3, SELL, CXCR4, CCL21, CCR7, CD86 und BCL6;

(y) Gruppe der follikulären dendritischen Zellen: FDCSP, SERPINE2, PRNP, PDPN, LTBR, BST1, CLU, C1S und C4A;

(z) Gruppe der follikulären Helfer-T-Zellen: SH2D1A, IL6, MAF, CD40LG, IL21, ICOS, IL4, CD84, CXCRS5 und BCL6; und

(aa) Granulozyten-Gruppe: CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4 und PRSS33.

**8.** Verfahren nach einem der Ansprüche 1 bis 7, wobei das Erzeugen der BLBC-TME-Signatur weiters das Normalisieren der Gengruppen-Werte umfasst, wobei das Normalisieren das Anwenden von Median-Skalierung auf die Gengruppen-Werte umfasst.

**9.** Verfahren nach einem der Ansprüche 1 bis 8,

wobei die Vielzahl von BLBC-TME-Typen einer entsprechenden Vielzahl von BLBC-TMA-Signatur-Clustern zugeordnet ist,

wobei das Identifizieren des BLBC-TME-Typs für das Individuums unter Verwendung der BLBC-TME-Signatur und aus der Vielzahl von BLBC-TME-Typen Folgendes umfasst:

das Zuordnen der BLBC-TME-Signatur des Individuums zu einem bestimmten aus der Vielzahl von BLBC-TME-Signatur-Clustern; und
das Identifizieren des BLBC-TME-Typs für das Individuum als jenen BLBC-TME-Typ, der dem bestimmten aus der Vielzahl von BLBC-TME-Signatur-Clustern entspricht, dem die BLBC-TME-Signatur des Individuums zugeordnet wird.

10. Verfahren nach Anspruch 9, wobei das Verfahren weiters das Erzeugen der Vielzahl von BLBC-TME-Signatur-Clustern umfasst, wobei das Erzeugen Folgendes umfasst:

das Erhalten von mehreren Sätzen von RNA-Expressionsdaten durch Sequenzieren von biologischen Proben von mehreren entsprechenden Individuen, wobei jeder der mehreren Sätze von RNA-Expressionsdaten RNA-Expressionswerte für zumindest manche Gene in jeder der zumindest manchen der Vielzahl von Gengruppen angibt, die in der nachstehenden Tabelle aufgelistet sind:

| | |
|---|---|
| MHC I | HLA-C, TAPBP, HLA-B, B2M, TAP2, HLA-A, TAP1, NLRCS |
| MHC II | HLA-DQB1, HLA-DMA, HLA-DMB, HLA-DRA, CIITA, HLA-DQA1, HLA-DPB1, HLA-DRB1, HLA-DPA1 |
| Koaktivierungsmoleküle | TNFRSF4, CD27, CD80, CD40LG, TNFRSF9, CD40, CD28, ICOSLG, CD83, TNFSF9, CD70, TNFSF4, ICOS, CD86 |
| Effektorzellen | ZAP70, GZMB, GZMK, IFNG, FASLG, EOMES, TBX21, GZMA, CD8A, GNLY, PRF1, CD8B |
| NK-Zellen | NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160 |
| T-Zellen | TRBC2, CD3E, CD3G, ITK, CD28, TRBC1, TRAT1, TBX21, CD5, TRAC, CD3D |
| B-Zellen | CD22, TNFRSF13C, STAP1, CD79B, PAXS, CR2, TNFRSF13B, CD79A, TNFRSF17, FCRL5, MS4A1, CD19, BLK |
| M1-Signaturen | CMKLR1, SOCS3, IRF5, NOS2, IL1B, 1L12B, IL23A, TNF, IL12A |
| Th1-Signatur | IL21, TBX21, IL12RB2, CD40LG, IFNG, IL2, STAT4 |
| Antitumor-Cytokine | CCL3, IL21, IFNB1, IFNA2, TNF, TNFSF10 |
| Checkpoint-Inhibition | PDCD1, BTLA, HAVCR2, CD274, VSIR, LAG3, TIGIT, PDCD1LG2, CTLA4 |
| Treg | IKZF2, TNFRSF18, IL10, FOXP3, CCR8, IKZF4, CTLA4 |
| Neutrophilen-Signatur | CD177, FFAR2, PGLYRP1, CXCR1, MPO, CXCR2, ELANE, CTSG, PRTN3, FCGR3B |
| Granulozyten-Wanderung | CCR3, KITLG, CCL11, CXCL2, CXCR1, CXCR2, CXCL8, CXCL1, CXCL5 |
| MDSC | ARG1, IL6, CYBB, IL10, PTGS2, IDO1, IL411 |
| Makrophagen | MRC1, SIGLEC1, MSR1, CD163, CSF1R, CD68, IL4I1, IL10 |
| Th2-Signatur | IL13, CCR4, IL10, IL4, ILS |
| Protumor-Cytokine | TGFB2, MIF, IL6, TGFB3, TGFB1, IL22, IL10 |
| CAF | COL6A3, PDGFRB, COL6A1, MFAPS, COL5A1, FAP, PDGFRA, FGF2, ACTA2, COL6A2, FBLN1, CD248, COL1A1, MMP2, COL1A2, MMP3, LUM, CXCL12, LRP1 |
| Matrix | LAMC2, TNC, COL11A1, VTN, LAMB3, COL1A1, FN1, LAMA3, LGALS9, COL1A2, COL4A1, COLSA1, ELN, LGALS7, COL3A1 |
| Angiogenese | VEGFC, VEGFA, PDGFC, KDR, CDH5, VEGFB, PGF, TEK, ANGPT2, CXCR2, FLT1, CXCL8, VWF, ANGPT1, CXCL5 |
| Endothel | KDR, CDHS, NOS3, VCAM1, VWF, FLT1, MMRN1, CLEC14A, ENG, MMRN2 |
| Proliferationsrate | CCND1, CCNB1, CETN3, CDK2, E2F1, AURKA, BUB1, AURKB, PLK1, MCM6, ESCO2, MYBL2, MKI67, MCM2, CCNE1 |

(continued)

| EMT-Signatur | CDH2, ZEB1, ZEB2, TWIST1, SNAI1, SNAI2, TWIST2 |
|---|---|
| TLS | CXCL13, CCL19, LAMP3, SELL, CXCR4, CCL21, CCR7, CD86, BCL6 |
| follikuläre dendritische Zellen | FDCSP, SERPINE2, PRNP, PDPN, LTBR, BST1, CLU, C1S, C4A |
| follikuläre B-Helfer-T-Zellen | SH2D1A, IL6, MAF, CD40LG, IL21, ICOS, IL4, CD84, CKCR5, BCL6 |
| Granulozyten | CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATAT1, EPX, IL13, MPO, IL5RA, CTSG, CXCL5, PRG2, CD177, CPA3, CCL11, PGLYRP1, MS4A2, IL4, PRSS33 |

wobei zumindest manche der Vielzahl von Gengruppen die Granylozyten-Gengruppe, die Angiogenese-Gengruppe, die B-Zellen-Gengruppe, die M1-Signatur-Gengruppe und die Endothel-Gengruppe umfassen;
das Erzeugen mehrerer BLBC-TME-Signaturen aus den mehreren Sätzen von RNA-Expressionsdaten, wobei jede der mehreren BLBC-TME-Signaturen Gengruppen-Werte für jeweilige Gengruppen in der Vielzahl von Gengruppen umfasst, wobei das Erzeugen für jede bestimmte der mehreren BLBC-TME-Signaturen Folgendes umfasst:

das Bestimmen der BLBC-TME-Signatur durch Bestimmen der Gengruppen-Expressionswerte unter Verwendung der RNA-Expressionsniveaus in dem jeweiligen Satz von RNA-Expressionsdaten, für den die jeweilige BLBC-TME-Signatur erzeugt wird; und
das Clustern der mehreren BLBC-Signaturen, um die Vielzahl von BLBC-TME-Signatur-Clustern zu erhalten.

11. Verfahren nach einem der Ansprüche 8 bis 10, das weiters Folgendes umfasst:

das Aktualisieren der Vielzahl von BLBC-TME-Signatur-Clustern unter Verwendung der BLBC-TME-Signatur des Individuums, wobei die BLBC-TME-Signatur des Individuums eine aus einer Schwellen-Anzahl von BLBC-TME-Signaturen für eine Schwellen-Anzahl von Individuen ist, wobei die BLBC-TME-Signatur-Cluster aktualisiert werden, wenn die Schwellen-Anzahl an BLBC-TME-Signaturen erzeugt ist,
wobei die Schwellen-Anzahl an BLBC-TME-Signaturen zumindest 50, zumindest 75, zumindest 100, zumindest 200, zumindest 500, zumindest 1000 oder zumindest 5000 BLBC-TME-Signaturen sind.

12. Verfahren nach Anspruch 11, wobei das Aktualisieren unter Verwendung eines Clustering-Algorithmus erfolgt, der aus der aus einem Dichte-Clustering-Algorithmus, einem spektralen Clustering-Algorithmus, einem k-Means-Clustering-Algorithmus, einem hierarchischen Clustering-Algorithmus und einem agglomerativen Clustering-Algorithmus bestehenden Gruppe ausgewählt ist.

13. Verfahren nach einem der Ansprüche 8 bis 12, das weiters Folgendes umfasst:
das Bestimmen eines BLBC-TME-Typs eines zweiten Individuums, wobei der BLBC-TME-Typ des zweiten Individuums unter Verwendung der aktualisierten BLBC-TME-Signatur-Cluster identifiziert wird, wobei das Identifizieren Folgendes umfasst:

das Bestimmen einer BLBC-TME-Signatur des zweiten Individuums aus RNA-Expressionsdaten, die durch Sequenzieren einer biologischen Probe erhalten wurden, die von dem zweiten Individuum erhalten wurde;
das Zuordnen der BLBC-TME-Signatur des zweiten Individuums zu einem bestimmten der Vielzahl der aktualisierten BLBC-TME-Signatur-Cluster; und
das Identifizieren des BLBC-TME-Typs für das zweite Individuum als jenen BLBC-TME-Typ, der dem bestimmten der Vielzahl von aktualisierten BLBC-TME-Signatur-Clustern entspricht, dem die BLBC-TME-Signatur des zweiten Individuums zugeordnet ist.

14. System, das Folgendes umfasst:

zumindest einen Computerhardware-Prozessor; und
zumindest ein nichtflüchtiges computerlesbares Medium, auf dem durch einen Prozessor ausführbare Anweisungen gespeichert sind, die bei Ausführung durch den zumindest einen Computerhardware-Prozessor bewirken, dass der zumindest eine Computerhardware-Prozessor ein Verfahren nach einem der Ansprüche 1 bis 13 ausführt.

15. Zumindest ein nichtflüchtiges computerlesbares Medium, auf dem durch einen Prozessor ausführbare Anweisungen gespeichert sind, die bewirken, dass der zumindest eine Computerhardware-Prozessor ein Verfahren nach einem der Ansprüche 1 bis 13 ausführt.

**Revendications**

1. Procédé pour déterminer un type de microenvironnement tumoral (TME) du cancer du sein de type basal (BLBC) d'un sujet souffrant d'un cancer du sein de type basal, le procédé comprenant :
l'utilisation d'au moins un processeur de matériel informatique pour réaliser les tâches suivantes :

obtenir des données d'expression d'ARN pour le sujet, les données d'expression d'ARN indiquant des taux d'expression d'ARN pour au moins certains gènes dans chaque groupe d'au moins certains groupes d'une pluralité de groupes de gènes répertoriés dans le tableau ci-dessous dans un échantillon biologique comprenant un tissu tumoral du sujet :

| | |
|---|---|
| CMH I | *HLA-C, TAPBP, HLA-B, B2M, TAP2, HLA-A, TAP1, NLRC5* |
| CMH II | *HLA-DQB1, HLA-DMA, HLA-DMB, HLA-DRA, CIITA, HLA-DQA1, HLA-DPB1, HLA-DRB1, HLA-DPA1* |
| Molécules de co-activation | *TNFRSF4, CD27, CD80, CD40LG, TNFRSF9, CD40, CD28, ICOSLG, CD83, TNFSF9, CD70, TNFSF4, ICOS, CD86* |
| Cellules effectrices | *ZAP70, GZMB, GZMK, IFNG, FASLG, EOMES, TBX21, GZMA, CD8A, GNLY, PRF1, CD8B* |
| Cellules NK | *NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160* |
| Cellules T | *TRBC2, CD3E, CD3G, ITK, CD28, TRBC1, TRAT1, TBX21, CD5, TRAC, CD3D* |
| Cellules B | *CD22, TNFRSF13C, STAP1, CD79B, PAX5, CR2, TNFRSF13B, CD79A, TNFRSF17, FCRL5, MS4A1, CD19, BLK* |
| Signatures M1 | *CMKLR1, SOCS3, IRF5, NOS2, IL1B, IL12B, IL23A, TNF, IL12A* |
| Signature Th1 | *IL21, TBX21, IL12RB2, CD40LG, IFNG, IL2, STAT4* |
| Cytokines antitumorales | *CCL3, IL21, IFNB1, IFNA2, TNF, TNFSF10* |
| Inhibition du point de contrôle | *PDCD1, BTLA, HAVCR2, CD274, VSIR, LAG3, TIGIT, PDCD1LG2, CTLA4* |
| Treg | *IKZF2, TNFRSF18, IL10, FOXP3, CCR8, IKZF4, CTLA4* |
| Signature des neutrophiles | *CD177, FFAR2, PGLYRP1, CXCR1, MPO, CXCR2, ELANE, CTSG, PRTN3, FCGR3B* |
| Trafic des granulocytes | *CCR3, KITLG, CCL11, CXCL2, CXCR1, CXCR2, CXCL8, CXCL1, CXCL5* |
| MDSC | *ARG, IL6, CYBB, IL10, PTGS2, IDO1, IL4I1* |
| Macrophages | *MRC1, SIGLEC1, MSR1, CD163, CSF1R, CD68, IL4I1, 1L10* |
| Signature Th2 | *IL13, CCR4, IL10, IL4, IL5* |
| Cytokines pro-tumorales | *TGFB2, MIF, IL6, TGFB3, TGFB1, IL22, IL10* |
| CAF | *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, LRP1* |

(continued)

| Matrice | LAMC2, TNC, COL11A1, VTN, LAMB3, COL1A1, FN1, LAMA3, LGALS9, COL1A2, COL4A1, COL5A1, ELN, LGALS7, COL3A1 |
|---|---|
| Angiogenèse | VEGFC, VEGFA, PDGFC, KDR, CDH5, VEGFB, PGF, TEK, ANGPT2, CXCR2, FLT1, CXCL8, VWF, ANGPT1, CXCL5 |
| Endothélium | KDR, CDH5, NOS3, VCAM1, VWF, FLT1, MMRN1, CLEC14A, ENG, MMRN2 |
| Taux de prolifération | CCND1, CCNB1, CETN3, CDK2, E2F1, AURKA, BUB1, AURKB, PLK1, MCM6, ESCO2, MYBL2, MKI67, MCM2, CCNE1 |
| Signature EMT | CDH2, ZEB1, ZEB2, TWIST1, SNAI1, SNAI2, TWIST2 |
| TLS | CXCL13, CCL19, LAMP3, SELL, CXCR4, CCL21, CCR7, CD86, BCL6 |
| Cellules dendritiques folliculaires | FDCSP, SERPINE2, PRNP, PDPN, LTBR, BST1, CLU, C1S, C4A |
| Cellules T auxiliaires des cellules B folliculaires | SH2D1A, IL6, MAF, CD40LG, IL21, ICOS, IL4, CD84, CXCR5, BCL6 |
| Granulocytes | CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, PRSS33 |

dans lequel les données d'expression d'ARN indiquent des taux d'expression d'ARN pour au moins trois gènes de chacun d'au moins : le groupe de gènes des granulocytes, le groupe de gènes de l'angiogenèse, le groupe de gènes des cellules B, le groupe de gènes des signatures M1, et le groupe de gènes de l'endothélium ; et

générer une signature de TME du BLBC pour le sujet en utilisant les données d'expression d'ARN, la signature de TME du BLBC comprenant des scores de groupes de gènes pour des groupes de gènes respectifs dans les au moins certains groupes de la pluralité de groupes de gènes, la génération comprenant :

la détermination des scores de groupes de gènes en utilisant les taux d'expression d'ARN et une technique d'analyse d'enrichissement d'ensembles de gènes ; et

identifier, en utilisant la signature de TME du BLBC et parmi une pluralité de types de TME du BLBC, un type de TME du BLBC pour le sujet, dans lequel la pluralité de types de TME du BLBC comprend : le type immuno-enrichi (IE), le type TLS (TLS), le type désert (D), le type fibrotique (F), et le type enrichi en granulocytes (G) et dans lequel :

(i) le type G du BLBC comprend les scores de groupe de gènes des granulocytes et des signatures M1 les plus élevés parmi la pluralité de types de TME du BLBC,

(ii) les types G et F possèdent des scores de groupe de gènes de l'angiogenèse plus élevés que les autres types de TME du BLBC parmi la pluralité de types de TME du BLBC,

(iii) le type TLS possède le score de groupe de gènes des cellules B le plus élevé parmi la pluralité de types de TME du BLBC, et

(iv) les types TLS et F possèdent les scores de groupe de gènes de taux d'expression dans l'endothélium les plus élevés parmi la pluralité de types de TME du BLBC.

2. Procédé selon la revendication 1, dans lequel : (i) l'obtention des taux d'expression d'ARN pour le sujet comprend l'obtention de données de séquençage précédemment obtenues par le séquençage d'un échantillon biologique obtenu chez le sujet, dans lequel l'échantillon biologique comprend un tissu tumoral du sujet, et/ou (ii) le procédé comprend en outre la normalisation des données d'expression d'ARN en unités de transcrits par million (TPM) avant de générer la signature de TME du BLBC.

3. Procédé selon la revendication 2, dans lequel les données de séquençage comprennent :

(a) au moins 1 million de lectures, au moins 5 millions de lectures, au moins 10 millions de lectures, au moins 20 millions de lectures, au moins 50 millions de lectures, ou au moins 100 millions de lectures ; et/ou

(b) des données de séquençage de l'exome entier (WES), des données de séquençage d'ARN en masse (RNA-seq), des données de séquençage d'ARN de cellule unique (scRNA-seq), et/ou des données de séquençage de nouvelle génération (NGS) ; ou

(c) des données de micro-réseau.

4. Procédé selon l'une quelconque des revendications 1 à 3, dans lequel l'obtention des données d'expression d'ARN pour le sujet comprend le séquençage d'un échantillon biologique obtenu chez le sujet, dans lequel l'échantillon biologique comprend un tissu tumoral du sujet.

5. Procédé selon l'une quelconque des revendications 1 à 4, dans lequel les taux d'expression d'ARN comprennent des taux d'expression d'ARN pour au moins trois gènes de chacun des groupes de gènes suivants, ou des taux d'expression d'ARN pour chacun des gènes de chacun des groupes de gènes suivants :

(a) groupe du CMH I : *HLA-C, TAPBP, HLA-B, B2M, TAP2, HLA-A, TAP1,* et *NLRC5 ;*

(b) groupe du CMH II : *HLA-DQB1, HLA-DMA, HLA-DMB, HLA-DRA, CIITA, HLA-DQA1, HLA-DPB1, HLA-DRB1,* et *HLA-DPA1 ;*

(c) groupe des molécules de co-activation : *TNFRSF4, CD27, CD80, CD40LG, TNFRSF9, CD40, CD28, ICOSLG, CD83, TNFSF9, CD70, TNFSF4, ICOS,* et *CD86 ;*

(d) groupe des cellules effectrices : *ZAP70, GZMB, GZMK, IFNG, FASLG, EOMES, TBX21, GZMA, CD8A, GNLY, PRF1,* et *CD8B ;*

(e) groupe des cellules NK *: NKG7, FGFBP2, CD244, KLRK1, KIR2DL4, CD226, KLRF1, GNLY, GZMB, KLRC2, NCR1, GZMH, IFNG, SH2D1B, NCR3, EOMES,* et *CD160 ;*

(f) groupe des cellules T *: TRBC2, CD3E, CD3G, ITK, CD28, TRBC1, TRAT1, TBX21, CD5, TRAC,* et *CD3D ;*

(g) groupe des cellules B : *CD22, TNFRSF13C, STAP1, CD79B, PAX5, CR2, TNFRSF13B, CD79A, TNFRSF17, FCRL5, MS4A1, CD19,* et *BLK ;*

(h) groupe des signatures M1 : *CMKLR1, SOCS3, IRF5, NOS2, IL1B, IL12B, IL23A, TNF,* et *IL12A ;*

(i) groupe de signature Th1 : *IL21, TBX21, IL12RB2, CD40LG, IFNG, IL2,* et *STAT4 ;*

(j) groupe des cytokines antitumorales : *CCL3, IL21, IFNB1, IFNA2, TNF,* et *TNFSF10 ;*

(k) groupe d'inhibition de point de contrôle : *PDCD1, BTLA, HAVCR2, CD274, VSIR, LAG3, TIGIT, PDCD1LG2,* et *CTLA4 ;*

(l) groupe des Treg : *IKZF2, TNFRSF18, IL10, FOXP3, CCR8, IKZF4,* et *CTLA4 ;*

(m) groupe de signature des neutrophiles : *CD177, FFAR2, PGLYRP1, CXCR1, MPO, CXCR2, ELANE, CTSG, PRTN3,* et *FCGR3B ;*

(n) groupe du trafic des granulocytes : *CCR3, KITLG, CCL11, CXCL2, CXCR1, CXCR2, CXCL8, CXCL1,* et *CXCL5 ;*

(o) groupe des MDSC *: ARG, IL6, CYBB, IL10, PTGS2, IDO1,* et *IL4I1 ;*

(p) groupe de signature Th2 *: IL13, CCR4, IL10, IL4,* et *IL5 ;*

(q) groupe des cytokines pro-tumorales : *TGFB2, MIF, IL6, TGFB3, TGFB1, IL22,* et *IL10 ;*

(r) groupe des fibroblastes associés au cancer (CAF) : *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* et *LRP1 ;*

(s) groupe de la matrice : *LAMC2, TNC, COL11A1, VTN, LAMB3, COL1A1, FN1, LAMA3, LGALS9, COL1A2, COL4A1, COL5A1, ELN, LGALS7,* et *COL3A1 ;*

(t) groupe de l'angiogenèse : *VEGFC, VEGFA, PDGFC, KDR, CDH5, VEGFB, PGF, TEK, ANGPT2, CXCR2, FLT1, CXCL8, VWF, ANGPT1,* et *CXCL5 ;*

(u) groupe de l'endothélium : *KDR, CDH5, NOS3, VCAM1, VWF, FLT1, MMRN1, CLEC14A, ENG,* et *MMRN2 ;*

(v) groupe du taux de prolifération : *CCND1, CCNB1, CETN3, CDK2, E2F1, AURKA, BUB1, AURKB, PLK1, MCM6, ESCO2, MYBL2, MKI67, MCM2,* et *CCNE1 ;*

(w) groupe de signature EMT : *CDH2, ZEB1, ZEB2, TWIST1, SNAI1, SNAI2,* et *TWIST2 ;*

(x) groupe des structures lymphoïdes tertiaires (TLS) : *CXCL13, CCL19, LAMP3, SELL, CXCR4, CCL21, CCR7, CD86,* et *BCL6 ;*

(y) groupe des cellules dendritiques folliculaires : *FDCSP, SERPINE2, PRNP, PDPN, LTBR, BST1, CLU, C1S,* et *C4A ;*

(z) groupe des cellules T auxiliaires des cellules B folliculaires : *SH2D1A, IL6, MAF, CD40LG, IL21, ICOS, IL4, CD84, CXCR5,* et *BCL6 ;* et

(aa) groupe des granulocytes : *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* et *PRSS33.*

**6.** Procédé selon l'une quelconque des revendications 1 à 5, dans lequel la détermination des scores de groupes de gènes comprend :

(i) la détermination d'un score de groupe de gènes respectif pour chacun d'au moins six des groupes de gènes suivants incluant le groupe de gènes des granulocytes, le groupe de gènes de l'angiogenèse, le groupe de gènes des cellules B, le groupe de gène des signatures M1, et le groupe de gènes de l'endothélium, en utilisant, pour un groupe de gènes particulier, les taux d'expression d'ARN pour au moins trois gènes dans le groupe de gènes particulier afin de déterminer le score de groupe de gènes pour le groupe particulier, les groupes de gènes comprenant :

(a) groupe du CMH I : *HLA-C, TAPBP, HLA-B, B2M, TAP2, HLA-A, TAP1,* et *NLRC5 ;*
(b) groupe du CMH II : *HLA-DQB1, HLA-DMA, HLA-DMB, HLA-DRA, CIITA, HLA-DQA1, HLA-DPB1, HLA-DRB1,* et *HLA-DPA1 ;*
(c) groupe des molécules de co-activation : *TNFRSF4, CD27, CD80, CD40LG, TNFRSF9, CD40, CD28, ICOSLG, CD83, TNFSF9, CD70, TNFSF4, ICOS,* et *CD86 ;*
(d) groupe des cellules effectrices : *ZAP70, GZMB, GZMK, IFNG, FASLG, EOMES, TBX21, GZMA, CD8A, GNLY, PRF1,* et *CD8B ;*
(e) groupe des cellules NK *: NKG7, FGFBP2, CD244, KLRK1, KIR2DL4, CD226, KLRF1, GNLY, GZMB, KLRC2, NCR1, GZMH, IFNG, SH2D1B, NCR3, EOMES,* et *CD160 ;*
(f) groupe des cellules T *: TRBC2, CD3E, CD3G, ITK, CD28, TRBC1, TRAT1, TBX21, CD5, TRAC,* et *CD3D ;*
(g) groupe des cellules B *: CD22, TNFRSF13C, STAP1, CD79B, PAX5, CR2, TNFRSF13B, CD79A, TNFRSF17, FCRL5, MS4A1, CD19,* et *BLK ;*
(h) groupe des signatures M1 : *CMKLR1, SOCS3, IRF5, NOS2, IL1B, IL12B, IL23A, TNF,* et *IL12A ;*
(i) groupe de signature Th1 : *IL21, TBX21, IL12RB2, CD40LG, IFNG, IL2,* et *STAT4 ;*
(j) groupe des cytokines antitumorales : *CCL3, IL21, IFNB1, IFNA2, TNF,* et *TNFSF10 ;*
(k) groupe d'inhibition de point de contrôle : *PDCD1, BTLA, HAVCR2, CD274, VSIR, LAG3, TIGIT, PDCD1LG2,* et *CTLA4 ;*
(l) groupe des Treg : *IKZF2, TNFRSF18, IL10, FOXP3, CCR8, IKZF4,* et *CTLA4 ;*
(m) groupe de signature des neutrophiles : *CD177, FFAR2, PGLYRP1, CXCR1, MPO, CXCR2, ELANE, CTSG, PRTN3,* et *FCGR3B ;*
(n) groupe du trafic des granulocytes : *CCR3, KITLG, CCL11, CXCL2, CXCR1, CXCR2, CXCL8, CXCL1,* et *CXCL5 ;*
(o) groupe des MDSC *: ARG, IL6, CYBB, IL10, PTGS2, IDO1,* et *IL4I1 ;*
(p) groupe de signature Th2 : *IL13, CCR4, IL10, IL4,* et *IL5 ;*
(q) groupe des cytokines pro-tumorales : *TGFB2, MIF, IL6, TGFB3, TGFB1, IL22,* et *IL10 ;*
(r) groupe des fibroblastes associés au cancer (CAF) : *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* et *LRP1 ;*
(s) groupe de la matrice : *LAMC2, TNC, COL11A1, VTN, LAMB3, COL1A1, FN1, LAMA3, LGALS9, COL1A2, COL4A1, COL5A1, ELN, LGALS7,* et *COL3A1 ;*
(t) groupe de l'angiogenèse : *VEGFC, VEGFA, PDGFC, KDR, CDH5, VEGFB, PGF, TEK, ANGPT2, CXCR2, FLT1, CXCL8, VWF, ANGPT1,* et *CXCL5 ;*
(u) groupe de l'endothélium : *KDR, CDH5, NOS3, VCAM1, VWF, FLT1, MMRN1, CLEC14A, ENG,* et *MMRN2 ;*
(v) groupe du taux de prolifération : *CCND1, CCNB1, CETN3, CDK2, E2F1, AURKA, BUB1, AURKB, PLK1, MCM6, ESCO2, MYBL2, MKI67, MCM2,* et *CCNE1 ;*
(w) groupe de signature EMT : *CDH2, ZEB1, ZEB2, TWIST1, SNAI1, SNAI2,* et *TWIST2 ;*
(x) groupe des structures lymphoïdes tertiaires (TLS) : *CXCL13, CCL19, LAMP3, SELL, CXCR4, CCL21, CCR7, CD86,* et *BCL6 ;*
(y) groupe des cellules dendritiques folliculaires : *FDCSP, SERPINE2, PRNP, PDPN, LTBR, BST1, CLU, C1S,* et *C4A ;*
(z) groupe des cellules T auxiliaires des cellules B folliculaires : *SH2D1A, IL6, MAF, CD40LG, IL21, ICOS, IL4, CD84, CXCR5,* et *BCL6 ;* et
(aa) groupe des granulocytes : *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* et *PRSS33.*

**7.** Procédé selon une quelconque revendication précédente, dans lequel la déermination des scores de groupe de gènes comprend :

(i) la détermination d'un score de groupe de gènes respectif pour chacun des groupes de gènes suivants, en utilisant, pour un groupe de gènes particulier, les taux d'expression d'ARN pour chacun des gènes dans chaque groupe de gènes afin de déterminer le score de groupe de gènes pour chaque groupe particulier, les groupes de gènes comprenant :

(a) groupe du CMH I : *HLA-C, TAPBP, HLA-B, B2M, TAP2, HLA-A, TAP1,* et *NLRC5 ;*
(b) groupe du CMH II : *HLA-DQB1, HLA-DMA, HLA-DMB, HLA-DRA, CIITA, HLA-DQA1, HLA-DPB1, HLA-DRB1,* et *HLA-DPA1 ;*
(c) groupe des molécules de co-activation : *TNFRSF4, CD27, CD80, CD40LG, TNFRSF9, CD40, CD28, ICOSLG, CD83, TNFSF9, CD70, TNFSF4, ICOS,* et *CD86 ;*
(d) groupe des cellules effectrices : *ZAP70, GZMB, GZMK, IFNG, FASLG, EOMES, TBX21, GZMA, CD8A, GNLY, PRF1,* et *CD8B ;*
(e) groupe des cellules NK *: NKG7, FGFBP2, CD244, KLRK1, KIR2DL4, CD226, KLRF1, GNLY, GZMB, KLRC2, NCR1, GZMH, IFNG, SH2D1B, NCR3, EOMES,* et *CD160 ;*
(f) groupe des cellules T *: TRBC2, CD3E, CD3G, ITK, CD28, TRBC1, TRAT1, TBX21, CD5, TRAC,* et *CD3D ;*
(g) groupe des cellules B : *CD22, TNFRSF13C, STAP1, CD79B, PAX5, CR2, TNFRSF13B, CD79A, TNFRSF17, FCRL5, MS4A1, CD19,* et *BLK ;*
(h) groupe des signatures M1 : *CMKLR1, SOCS3, IRF5, NOS2, IL1B, IL12B, IL23A, TNF,* et *IL12A ;*
(i) groupe de signature Th1 : *IL21, TBX21, IL12RB2, CD40LG, IFNG, IL2,* et *STAT4 ;*
(j) groupe des cytokines antitumorales : *CCL3, IL21, IFNB1, IFNA2, TNF,* et *TNFSF10 ;*
(k) groupe d'inhibition de point de contrôle : *PDCD1, BTLA, HAVCR2, CD274, VSIR, LAG3, TIGIT, PDCD1LG2,* et *CTLA4 ;*
(l) groupe des Treg : *IKZF2, TNFRSF18, IL10, FOXP3, CCR8, IKZF4,* et *CTLA4 ;*
(m) groupe de signature des neutrophiles : *CD177, FFAR2, PGLYRP1, CXCR1, MPO, CXCR2, ELANE, CTSG, PRTN3,* et *FCGR3B ;*
(n) groupe du trafic des granulocytes : *CCR3, KITLG, CCL11, CXCL2, CXCR1, CXCR2, CXCL8, CXCL1,* et *CXCL5 ;*
(o) groupe des MDSC *: ARG, IL6, CYBB, IL10, PTGS2, IDO1,* et *ILA4I1 ;*
(p) groupe de signature Th2 : *IL13, CCR4, IL10, IL4,* et *IL5 ;*
(q) groupe des cytokines pro-tumorales : *TGFB2, MIF, IL6, TGFB3, TGFB1, IL22,* et *IL10 ;*
(r) groupe des fibroblastes associés au cancer (CAF) : *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* et *LRP1 ;*
(s) groupe de la matrice : *LAMC2, TNC, COL11A1, VTN, LAMB3, COL1A1, FN1, LAMA3, LGALS9, COL1A2, COL4A1, COL5A1, ELN, LGALS7,* et *COL3A1 ;*
(t) groupe de l'angiogenèse : *VEGFC, VEGFA, PDGFC, KDR, CDH5, VEGFB, PGF, TEK, ANGPT2, CXCR2, FLT1, CXCL8, VWF, ANGPT1,* et *CXCL5 ;*
(u) groupe de l'endothélium : *KDR, CDH5, NOS3, VCAM1, VWF, FLT1, MMRN1, CLEC14A, ENG,* et *MMRN2 ;*
(v) groupe du taux de prolifération : *CCND1, CCNB1, CETN3, CDK2, E2F1, AURKA, BUB1, AURKB, PLK1, MCM6, ESCO2, MYBL2, MKI67, MCM2,* et *CCNE1 ;*
(w) groupe de signature EMT : *CDH2, ZEB1, ZEB2, TWIST1, SNAI1, SNAI2,* et *TWIST2 ;*
(x) groupe des structures lymphoïdes tertiaires (TLS) : *CXCL13, CCL19, LAMP3, SELL, CXCR4, CCL21, CCR7, CD86,* et *BCL6 ;*
(y) groupe des cellules dendritiques folliculaires : *FDCSP, SERPINE2, PRNP, PDPN, LTBR, BST1, CLU, C1S,* et *C4A ;*
(z) groupe des cellules T auxiliaires des cellules B folliculaires : *SH2D1A, IL6, MAF, CD40LG, IL21, ICOS, IL4, CD84, CXCR5,* et *BCL6 ;* et
(aa) groupe des granulocytes : *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* et *PRSS33 ;* et/ou

(ii) la détermination d'un premier score d'un premier groupe de gènes en utilisant une technique d'analyse d'enrichissement d'ensembles de gènes à échantillon unique (ssGSEA) à partir des taux d'expression d'ARN pour au moins certains des gènes dans un des groupes de gènes suivants :

(a) groupe du CMH I : *HLA-C, TAPBP, HLA-B, B2M, TAP2, HLA-A, TAP1,* et *NLRC5 ;*

(b) groupe du CMH II : *HLA-DQB1, HLA-DMA, HLA-DMB, HLA-DRA, CIITA, HLA-DQA1, HLA-DPB1, HLA-DRB1,* et *HLA-DPA1 ;*

(c) groupe des molécules de co-activation : *TNFRSF4, CD27, CD80, CD40LG, TNFRSF9, CD40, CD28, ICOSLG, CD83, TNFSF9, CD70, TNFSF4, ICOS,* et *CD86 ;*

(d) groupe des cellules effectrices : *ZAP70, GZMB, GZMK, IFNG, FASLG, EOMES, TBX21, GZMA, CD8A, GNLY, PRF1,* et *CD8B ;*

(e) groupe des cellules NK : *NKG7, FGFBP2, CD244, KLRK1, KIR2DL4, CD226, KLRF1, GNLY, GZMB, KLRC2, NCR1, GZMH, IFNG, SH2D1B, NCR3, EOMES,* et *CD160 ;*

(f) groupe des cellules T : *TRBC2, CD3E, CD3G, ITK, CD28, TRBC1, TRAT1, TBX21, CD5, TRAC,* et *CD3D ;*

(g) groupe des cellules B : *CD22, TNFRSF13C, STAP1, CD79B, PAX5, CR2, TNFRSF13B, CD79A, TNFRSF17, FCRL5, MS4A1, CD19,* et *BLK ;*

(h) groupe des signatures M1 : *CMKLR1, SOCS3, IRF5, NOS2, IL1B, IL12B, IL23A, TNF,* et *IL12A ;*

(i) groupe de signature Th1 : *IL21, TBX21, IL12RB2, CD40LG, IFNG, IL2,* et *STAT4 ;*

(j) groupe des cytokines antitumorales : *CCL3, IL21, IFNB1, IFNA2, TNF,* et *TNFSF10 ;*

(k) groupe d'inhibition de point de contrôle : *PDCD1, BTLA, HAVCR2, CD274, VSIR, LAG3, TIGIT, PDCD1LG2,* et *CTLA4 ;*

(l) groupe des Treg : *IKZF2, TNFRSF18, IL10, FOXP3, CCR8, IKZF4,* et *CTLA4 ;*

(m) groupe de signature des neutrophiles : *CD177, FFAR2, PGLYRP1, CXCR1, MPO, CXCR2, ELANE, CTSG, PRTN3,* et *FCGR3B ;*

(n) groupe du trafic des granulocytes : *CCR3, KITLG, CCL11, CXCL2, CXCR1, CXCR2, CXCL8, CXCL1,* et *CXCL5 ;*

(o) groupe des MDSC : *ARG, IL6, CYBB, IL10, PTGS2, IDO1,* et *ILA4I1 ;*

(p) groupe de signature Th2 : *IL13, CCR4, IL10, IL4,* et *IL5 ;*

(q) groupe des cytokines pro-tumorales : *TGFB2, MIF, IL6, TGFB3, TGFB1, IL22,* et *IL10 ;*

(r) groupe des fibroblastes associés au cancer (CAF) : *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12,* et *LRP1 ;*

(s) groupe de la matrice : *LAMC2, TNC, COL11A1, VTN, LAMB3, COL1A1, FN1, LAMA3, LGALS9, COL1A2, COL4A1, COL5A1, ELN, LGALS7,* et *COL3A1 ;*

(t) groupe de l'angiogenèse : *VEGFC, VEGFA, PDGFC, KDR, CDH5, VEGFB, PGF, TEK, ANGPT2, CXCR2, FLT1, CXCL8, VWF, ANGPT1,* et *CXCL5 ;*

(u) groupe de l'endothélium : *KDR, CDH5, NOS3, VCAM1, VWF, FLT1, MMRN1, CLEC14A, ENG,* et *MMRN2 ;*

(v) groupe du taux de prolifération : *CCND1, CCNB1, CETN3, CDK2, E2F1, AURKA, BUB1, AURKB, PLK1, MCM6, ESCO2, MYBL2, MKI67, MCM2,* et *CCNE1 ;*

(w) groupe de signature EMT : *CDH2, ZEB1, ZEB2, TWIST1, SNAI1, SNAI2,* et *TWIST2 ;*

(x) groupe des structures lymphoïdes tertiaires (TLS) : *CXCL13, CCL19, LAMP3, SELL, CXCR4, CCL21, CCR7, CD86,* et *BCL6 ;*

(y) groupe des cellules dendritiques folliculaires : *FDCSP, SERPINE2, PRNP, PDPN, LTBR, BST1, CLU, C1S,* et *C4A ;*

(z) groupe des cellules T auxiliaires des cellules B folliculaires : *SH2D1A, IL6, MAF, CD40LG, IL21, ICOS, IL4, CD84, CXCR5,* et *BCL6 ;* et

(aa) groupe des granulocytes : *CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4,* et *PRSS33 ;* et/ou

(iv) l'utilisation d'une technique GSEA à échantillon unique pour déterminer les scores de groupes de gènes à partir des taux d'expression d'ARN pour chacun des gènes dans chacun des groupes de gènes suivants :

(a) groupe du CMH I : *HLA-C, TAPBP, HLA-B, B2M, TAP2, HLA-A, TAP1,* et *NLRC5 ;*

(b) groupe du CMH II : *HLA-DQB1, HLA-DMA, HLA-DMB, HLA-DRA, CIITA, HLA-DQA1, HLA-DPB1, HLA-DRB1,* et *HLA-DPA1 ;*

(c) groupe des molécules de co-activation : *TNFRSF4, CD27, CD80, CD40LG, TNFRSF9, CD40, CD28, ICOSLG, CD83, TNFSF9, CD70, TNFSF4, ICOS,* et *CD86 ;*

(d) groupe des cellules effectrices : *ZAP70, GZMB, GZMK, IFNG, FASLG, EOMES, TBX21, GZMA, CD8A, GNLY, PRF1,* et *CD8B ;*

(e) groupe des cellules NK : *NKG7, FGFBP2, CD244, KLRK1, KIR2DL4, CD226, KLRF1, GNLY, GZMB,*

KLRC2, NCR1, GZMH, IFNG, SH2D1B, NCR3, EOMES, et CD160 ;

(f) groupe des cellules T : TRBC2, CD3E, CD3G, ITK, CD28, TRBC1, TRAT1, TBX21, CD5, TRAC, et CD3D ;

(g) groupe des cellules B : CD22, TNFRSF13C, STAP1, CD79B, PAX5, CR2, TNFRSF13B, CD79A, TNFRSF17, FCRL5, MS4A1, CD19, et BLK ;

(h) groupe des signatures M1 : CMKLR1, SOCS3, IRF5, NOS2, IL1B, IL12B, IL23A, TNF, et IL12A ;

(i) groupe de signature Th1 : IL21, TBX21, IL12RB2, CD40LG, IFNG, IL2, et STAT4 ;

(j) groupe des cytokines antitumorales : CCL3, IL21, IFNB1, IFNA2, TNF, et TNFSF10 ;

(k) groupe d'inhibition de point de contrôle : PDCD1, BTLA, HAVCR2, CD274, VSIR, LAG3, TIGIT, PDCD1LG2, et CTLA4 ;

(l) groupe des Treg : IKZF2, TNFRSF18, IL10, FOXP3, CCR8, IKZF4, et CTLA4 ;

(m) groupe de signature des neutrophiles : CD177, FFAR2, PGLYRP1, CXCR1, MPO, CXCR2, ELANE, CTSG, PRTN3, et FCGR3B ;

(n) groupe du trafic des granulocytes : CCR3, KITLG, CCL11, CXCL2, CXCR1, CXCR2, CXCL8, CXCL1, et CXCL5 ;

(o) groupe des MDSC : ARG, IL6, CYBB, IL10, PTGS2, IDO1, et ILA4I1 ;

(p) groupe de signature Th2 : IL13, CCR4, IL10, IL4, et IL5 ;

(q) groupe des cytokines pro-tumorales : TGFB2, MIF, IL6, TGFB3, TGFB1, IL22, et IL10 ;

(r) groupe des fibroblastes associés au cancer (CAF) : COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, et LRP1 ;

(s) groupe de la matrice : LAMC2, TNC, COL11A1, VTN, LAMB3, COL1A1, FN1, LAMA3, LGALS9, COL1A2, COL4A1, COL5A1, ELN, LGALS7, et COL3A1 ;

(t) groupe de l'angiogenèse : VEGFC, VEGFA, PDGFC, KDR, CDH5, VEGFB, PGF, TEK, ANGPT2, CXCR2, FLT1, CXCL8, VWF, ANGPT1, et CXCL5 ;

(u) groupe de l'endothélium : KDR, CDH5, NOS3, VCAM1, VWF, FLT1, MMRN1, CLEC14A, ENG, et MMRN2 ;

(v) groupe du taux de prolifération : CCND1, CCNB1, CETN3, CDK2, E2F1, AURKA, BUB1, AURKB, PLK1, MCM6, ESCO2, MYBL2, MKI67, MCM2, et CCNE1 ;

(w) groupe de signature EMT : CDH2, ZEB1, ZEB2, TWIST1, SNAI1, SNAI2, et TWIST2 ;

(x) groupe des structures lymphoïdes tertiaires (TLS) : CXCL13, CCL19, LAMP3, SELL, CXCR4, CCL21, CCR7, CD86, et BCL6 ;

(y) groupe des cellules dendritiques folliculaires : FDCSP, SERPINE2, PRNP, PDPN, LTBR, BST1, CLU, C1S, et C4A ;

(z) groupe des cellules T auxiliaires des cellules B folliculaires : SH2D1A, IL6, MAF, CD40LG, IL21, ICOS, IL4, CD84, CXCR5, et BCL6 ; et

(aa) groupe des granulocytes : CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, et PRSS33.

8. Procédé selon l'une quelconque des revendications 1 à 7, dans lequel la génération de la signature de TME du BLBC comprend en outre la normalisation des scores de groupes de gènes, dans lequel la normalisation comprend l'application d'une mise à l'échelle de la médiane aux scores de groupes de gènes.

9. Procédé selon l'une quelconque des revendications 1 à 8,

dans lequel la pluralité de types de TME du BLBC est associée à une pluralité respective de clusters de signature de TME du BLBC,

dans lequel l'identification, en utilisant la signature de TME du BLBC et parmi une pluralité de types de TME du BLBC, du type de TME du BLBC pour le sujet comprend :

l'association de la signature de TME du BLBC avec une signature particulière de la pluralité de clusters de signature de TME du BLBC; et

l'identification du type de TME du BLBC pour le sujet en tant que type de TME du BLBC correspondant au type particulier de la pluralité de clusters de signature de TME du BLBC auquel la signature de TME du BLBC du sujet est associée.

10. Procédé selon la revendication 9, dans lequel le procédé comprend en outre la génération de la pluralité de de clusters de signature de TME du BLBC, la génération comprenant :

l'obtention de multiples ensembles de données d'expression d'ARN par le séquençage d'échantillons biologiques à partir de multiples sujets respectifs, chacun des multiples ensembles de données d'expression d'ARN indiquant des taux d'expression d'ARN pour au moins certains gènes dans chacun des au moins certains groupes de la pluralité de groupes de gènes répertoriés dans le tableau ci-dessous :

| | |
|---|---|
| CMH I | *HLA-C, TAPBP, HLA-B, B2M, TAP2, HLA-A, TAP1, NLRC5* |
| CMH II | *HLA-DQB1, HLA-DMA, HLA-DMB, HLA-DRA, CIITA, HLA-DQA1, HLA-DPB1, HLA-DRB1, HLA-DPA1* |
| Molécules de co-activation | *TNFRSF4, CD27, CD80, CD40LG, TNFRSF9, CD40, CD28, ICOSLG, CD83, TNFSF9, CD70, TNFSF4, ICOS, CD86* |
| Cellules effectrices | *ZAP70, GZMB, GZMK, IFNG, FASLG, EOMES, TBX21, GZMA, CD8A, GNLY, PRF1, CD8B* |
| Cellules NK | *NKG7, FGFBP2, CD244, KLRK1, KIR2DL4, CD226, KLRF1, GNLY, GZMB, KLRC2, NCR1, GZMH, IFNG, SH2D1B, NCR3, EOMES, CD160* |
| Cellules T | *TRBC2, CD3E, CD3G, ITK, CD28, TRBC1, TRAT1, TBX21, CD5, TRAC, CD3D* |
| Cellules B | *CD22, TNFRSF13C, STAP1, CD79B, PAX5, CR2, TNFRSF13B, CD79A, TNFRSF17, FCRL5, MS4A1, CD19, BLK* |
| Signatures M1 | *CMKLR1, SOCS3, IRF5, NOS2, IL1B, IL12B, IL23A, TNF, IL12A* |
| Signature Th1 | *IL21, TBX21, IL12RB2, CD40LG, IFNG, IL2, STAT4* |
| Cytokines antitumorales | *CCL3, IL21, IFNB1, IFNA2, TNF, TNFSF10* |
| Inhibition du point de contrôle | *PDCD1, BTLA, HAVCR2, CD274, VSIR, LAG3, TIGIT, PDCD1LG2, CTLA4* |
| Treg | *IKZF2, TNFRSF18, IL10, FOXP3, CCR8, IKZF4, CTLA4* |
| Signature des neutrophiles | *CD177, FFAR2, PGLYRP1, CXCR1, MPO, CXCR2, ELANE, CTSG, PRTN3, FCGR3B* |
| Trafic des granulocytes | *CCR3, KITLG, CCL11, CXCL2, CXCR1, CXCR2, CXCL8, CXCL1, CXCL5* |
| MDSC | *ARG, IL6, CYBB, IL10, PTGS2, IDO1, IL4I1* |
| Macrophages | *MRC1, SIGLEC1, MSR1, CD163, CSF1R, CD68, IL4I1, 1L10* |
| Signature Th2 | *IL13, CCR4, IL10, IL4, IL5* |
| Cytokines pro-tumorales | *TGFB2, MIF, IL6, TGFB3, TGFB1, IL22, IL10* |
| CAF | *COL6A3, PDGFRB, COL6A1, MFAP5, COL5A1, FAP, PDGFRA, FGF2, ACTA2, COL6A2, FBLN1, CD248, COL1A1, MMP2, COL1A2, MMP3, LUM, CXCL12, LRP1* |
| Matrice | *LAMC2, TNC, COL11A1, VTN, LAMB3, COL1A1, FN1, LAMA3, LGALS9, COL1A2, COL4A1, COL5A1, ELN, LGALS7, COL3A1* |
| Angiogenèse | *VEGFC, VEGFA, PDGFC, KDR, CDH5, VEGFB, PGF, TEK, ANGPT2, CXCR2, FLT1, CXCL8, VWF, ANGPT1, CXCL5* |
| Endothélium | *KDR, CDH5, NOS3, VCAM1, VWF, FLT1, MMRN1, CLEC14A, ENG, MMRN2* |
| Taux de prolifération | *CCND1, CCNB1, CETN3, CDK2, E2F1, AURKA, BUB1, AURKB, PLK1, MCM6, ESCO2, MYBL2, MKI67, MCM2, CCNE1* |
| Signature EMT | *CDH2, ZEB1, ZEB2, TWIST1, SNAI1, SNAI2, TWIST2* |
| TLS | *CXCL13, CCL19, LAMP3, SELL, CXCR4, CCL21, CCR7, CD86, BCL6* |

(continued)

| Cellules dendritiques folliculaires | FDCSP, SERPINE2, PRNP, PDPN, LTBR, BST1, CLU, C1S, C4A |
|---|---|
| Cellules T auxiliaires des cellules B folliculaires | SH2D1A, IL6, MAF, CD40LG, IL21, ICOS, IL4, CD84, CXCR5, BCL6 |
| Granulocytes | CCR3, FFAR2, CXCL2, CMA1, SIGLEC8, TPSAB1, RNASE3, IL5, PRG3, CXCR1, CXCR2, ELANE, CXCL8, CXCL1, PRTN3, RNASE2, FCGR3B, GATA1, EPX, IL13, MPO, IL5RA, CTSG, CXCL5, PRG2, CD177, CPA3, CCL11, PGLYRP1, MS4A2, IL4, PRSS33 |

dans lequel les au moins certains de la pluralité de groupes de gènes comprennent le groupe de gènes des granulocytes, le groupe de gènes de l'angiogenèse, le groupe de gènes des cellules B, le groupe de gènes des signatures M1, et le groupe de gènes de l'endothélium ;

la génération de multiples signatures de TME du BLBC à partir des multiples ensembles de données d'expression d'ARN, chacune des multiples signatures de TME du BLBC comprenant des scores d'expression de groupes de gènes pour des groupes de gènes respectifs dans la pluralité de groupes de gènes, la génération comprenant, pour chaque signature particulière des multiples signatures de TME du BLBC :

la détermination de la signature de TME du BLBC par la détermination des scores d'expression de groupes de gènes en utilisant les taux d'expression d'ARN dans l'ensemble particulier de données d'expression d'ARN pour lequel la signature de TME du BLBC particulière est générée ; et
le regroupement des multiples signatures du BLBC afin d'obtenir la pluralité de clusters de signature de TME du BLBC.

11. Procédé selon l'une quelconque des revendications 8 à 10, comprenant en outre :

la mise à jour de la pluralité de clusters de signature de TME du BLBC en utilisant la signature de TME du BLBC du sujet, dans lequel la signature de TME du BLBC du sujet est une signature d'un nombre seuil de signatures de TME du BLBC pour un nombre seuil de sujets, dans lequel lorsque le nombre seuil de signatures de TME du BLBC est généré, les clusters de signature de TME du BLBC sont mis à jour,
dans lequel le nombre seuil de signatures de TME du BLBC est au moins 50, au moins 75, au moins 100, au moins 200, au moins 500, au moins 1000, ou au moins 5000 signatures de TME du BLBC.

12. Procédé selon la revendication 11, dans lequel la mise à jour est réalisée en utilisant un algorithme de clustering sélectionné dans le groupe consistant en un algorithme de clustering de densité, un algorithme de clustering spectral, un algorithme de clustering des k-moyennes, un algorithme de clustering hiérarchique, et un algorithme de clustering agglomératif.

13. Procédé selon l'une quelconque des revendications 8 à 12, comprenant en outre :
la détermination d'un type de TME du BLBC d'un deuxième sujet, dans lequel le type de TME du BLBC du deuxième sujet est identifié en utilisant les clusters de signature de TME du BLBC mis à jour, dans lequel l'identification comprend :

la détermination d'une signature de TME du BLBC du deuxième sujet à partir des données d'expression d'ARN obtenues par le séquençage d'un échantillon biologique obtenu chez le deuxième sujet ;
l'association de la signature de TME du BLBC du deuxième sujet avec un cluster particulier de la pluralité de clusters de signature de TME du BLBC mis à jour ; et
l'identification du type de TME du BLBC pour le deuxième sujet en tant que type de TME du BLBC correspondant au cluster particulier de la pluralité de clusters de signature de TME du BLBC mis à jour auquel la signature de TME du BLBC du deuxième sujet est associée.

14. Système, comprenant :

au moins un processeur de matériel informatique, et

au moins un support lisible par ordinateur non transitoire stockant des instructions exécutables par un processeur qui, lorsqu'elles sont exécutées par le au moins un processeur de matériel informatique, permettent à le au moins un processeur de matériel informatique de réaliser le procédé selon l'une quelconque des revendications 1 à 13.

15. Au moins un support lisible par ordinateur non transitoire stockant des instructions exécutables par un processeur qui, lorsqu'elles sont exécutées par le au moins un processeur de matériel informatique, permettent à le au moins un processeur de matériel informatique de réaliser le procédé selon l'une quelconque des revendications 1 à 13.

FIG. 1

FIG. 2

# FIG. 3

104

400

Obtaining sequencing data

402

Normalizing sequencing data to TPM units

404

Log transformation of TPM units

# FIG. 4

FIG. 5

FIG. 6

**FIG. 7**

FIG. 8

FIG. 8 cont.

FIG. 9

FIG. 10A

FIG. 10B

FIG. 10C

FIG. 11

FIG. 12

FIG. 13

FIG. 14

PAM50 subtypes in TME clusters

Grades in TME clusters

**FIG. 14 cont.**

B_cells    NK_cells    Endothelium    Neutrophils
CD4_T_cells    Macrophages    Fibroblasts    Other
CD8_T_cells    Monocytes

## FIG. 15

OS Hormone treated, Metabric, N =759
p-value = 0.002

Angiogenic (E)
Fibrotic (F)
Immune Desert (D)
Immune-Enriched, Fibrotic (IE/F)
Immune-Enriched, Non-Fibrotic (IE)

Months

## FIG. 16

FIG. 17

FIG. 18

FIG. 18 cont.

FIG. 19

FIG. 19 cont.

FIG. 20

FIG. 21

TME
BG_Grade
PDCD1
CD274
CTLA4
LAG3
PDCD1LG2
BTLA
TIGIT
VEGFA

FIG. 22

**FIG. 23**

## REFERENCES CITED IN THE DESCRIPTION

### Patent documents cited in the description

- US 2021208161 A1
- US 20200273543 A

- WO 2021183917 A

### Non-patent literature cited in the description

- **WANG YANYAN et al.** Identification of Tumor Microenvironment-Related Prognostic Biomarkers in Luminal Breast Cancer. *FRONTIERS IN GENETICS*, 10 November 2020, vol. 11
- **JAMES R. BRADFORD et al.** Consensus Analysis of Whole Transcriptome Profiles from Two Breast Cancer Patient Cohorts Reveals Long Non-Coding RNAs Associated with Intrinsic Subtype and the Tumour Microenvironment. *PLOS ONE*, 29 September 2016, vol. 11 (9)
- **SADEGHALVAD MONA et al.** Immune microenvironment in different molecular subtypes of ductal breast carcinoma. *BREAST CANCER RESEARCH AND TREATMENT*, 03 October 2020, vol. 185 (2)
- **PEROU et al.** *Nature*, 2000, vol. 406 (6796), 747-752
- **ZHANG**. *Arch Pathol Lab Med.*, 22 September 2022
- **VAUGHT et al.** *Cancer Epidemiol Biomarkers Prev.*, February 2012, vol. 21 (2), 253-5
- **VAUGHT** ; **HENDERSON**. Biological sample collection, processing, storage and information management. *IARC Sci Publ.*, 2011, vol. 2011 (163), 23-42
- **NICOLAS L BRAY** ; **HAROLD PIMENTEL** ; **PÁLL MELSTED** ; **LIOR PACHTER**. Near-optimal probabilistic RNA-seq quantification. *Nature Biotechnology*, 2016, vol. 34, 525-527

- *Bioinformatics*, 12 February 2004, vol. 20 (3), 307-15
- **LAURENT GAUTIER 1** ; **LESLIE COPE** ; **BENJAMIN M BOLSTAD** ; **RAFAEL A IRIZARRY**. *affy-- analysis of Affymetrix GeneChip data at the probe level*
- **RITCHIE ME** ; **PHIPSON B** ; **WU D** ; **HU Y** ; **LAW CW** ; **SHI W** ; **SMYTH GK**. limma powers differential expression analyses for RNA-sequencing and microarray studies. *Nucleic Acids Res.*, 20 April 2015, vol. 43 (7), e47. 20
- **WAGNER et al.** *Theory Biosci.*, 2012, vol. 131, 281-285
- **WU J**. gcrma: Background Adjustment Using Sequence Information. R package version 2.66.0.. *Gentry RIwcfJMJ*, 2021
- **BARBIE et al.** *Nature*, 05 November 2009, vol. 462 (7269), 108-112
- **LOUEDEC et al.** *Vaccines (Basel)*, December 2020, vol. 8 (4), 632
- **GERRITSE et al.** *Cancer Treat Rev.*, June 2021, vol. 97, 102171
- **RASSY et al.** *Ther Adv Med Oncol.*, 2020, vol. 12
- **SCHUBERT, M.** ; **KLINGER, B.** ; **KLÜNEMANN, M. et al.** Perturbation-response genes reveal signaling footprints in cancer gene expression. *Nat Commun*, 2018, vol. 9, 20, www.nature.com/articles/s41467-017-02391-6